# EUROPEAN PATENT SPECIFICATION

(11) **EP 2 824 183 B1**
(45) Date of publication and mention of the grant of the patent: **29.07.2020**
(21) Application number: 14176527.1
(22) Date of filing: 31.03.2006
(51) Int. Cl.: C07K 16/00

(54) **Methods for producing bispecific antibodies**
Verfahren zur Herstellung bispezifischer Antikörper
Procédé pour la production d'anticorps bispécifiques

(30) Priority: 08.04.2005 JP 2005112514
(43) Date of publication of application: 14.01.2015
(62) Divisional of application: 06730769.4
(73) Proprietor: CHUGAI SEIYAKU KABUSHIKI KAISHA, Tokyo, 115-8543 (JP)
(72) Inventor: Kojima, Tetsuo, Shizuoka 4128513 (JP); Hattori, Kunihiro, Shizuoka 4128513 (JP); Soeda, Tetsuhiro, Shizuoka 4128513 (JP); Miyazaki, Taro, Shizuoka 4128513 (JP); Saito, Hiroyuki, Shizuoka 4128513 (JP)
(74) Representative: Vossius & Partner Patentanwälte Rechtsanwälte mbB

(56) References cited:
- WO-A1-96/07754
- WO-A1-2004/065611
- WO-A1-2004/111233
- WO-A2-98/50431
- WO-A2-2004/009618
- WO-A2-2004/097041
- US-A1- 2002 062 010
- MERCHANT A MARGARET ET AL: "An efficient route to human bispecific IgG", NATURE BIOTECHNOLOGY, NATURE PUBLISHING GROUP, NEW YORK, NY, US, vol. 16, no. 7, 1 July 1998 (1998-07-01), pages 677-681, XP002141015, ISSN: 1087-0156, DOI: 10.1038/NBT0798-677
- CARTER PAUL: "Bispecific human IgG by design", JOURNAL OF IMMUNOLOGICAL METHODS, ELSEVIER SCIENCE PUBLISHERS B.V.,AMSTERDAM, NL, vol. 248, no. 1-2, 1 January 2001 (2001-01-01), pages 7-15, XP002974199, ISSN: 0022-1759, DOI: 10.1016/S0022-1759(00)00339-2
- FIGINI M ET AL: "In Vitro Assembly of Repertoires of Antibody Chains on the Surface of Phage by Renaturation", JOURNAL OF MOLECULAR BIOLOGY, ACADEMIC PRESS, UNITED KINGDOM, vol. 239, no. 1, 26 May 1994 (1994-05-26), pages 68-78, XP024009507, ISSN: 0022-2836, DOI: 10.1006/JMBI.1994.1351 [retrieved on 1994-05-26]
- VAUGHAN T J ET AL: "HUMAN ANTIBODIES WITH SUB-NANOMOLAR AFFINITIES ISOLATED FROM A LARGE NON-IMMUNIZED PHAGE DISPLAY LIBRARY", NATURE BIOTECHNOLOGY, NATURE PUBLISHING GROUP, NEW YORK, NY, US, vol. 14, 1 March 1996 (1996-03-01), pages 309-314, XP000196144, ISSN: 1087-0156, DOI: 10.1038/NBT0396-309
- JIRHOLT P ET AL: "Exploiting sequence space: shuffling in vivo formed complementarity determining regions into a master framework", GENE, ELSEVIER, AMSTERDAM, NL, vol. 215, no. 2, 1 July 1998 (1998-07-01), pages 471-476, XP004149272, ISSN: 0378-1119, DOI: 10.1016/S0378-1119(98)00317-5

### SEQUENCE LISTING

<110> CHUGAI SEIYAKU KABUSHIKI KAISHA
<120> Antibody substituting for function of blood coagulation factor VIII
<130> C1-A0504P
<150> JP 2005-112514
   <151> 2005-04-08
<160> 144
<170> Patent In version 3.3
<210> 1
   <211> 119
   <212> PRT
   <213> Mus musculus
<400> 1
   Met Gln Val Gln Leu Gln Gln Ser Gly Ala Glu Leu Ala Lys Pro Gly
   1 5 10 15
   Ala Ser Val Lys Leu Ser Cys Lys Ala Ser Gly Tyr Thr Phe Thr Ser
   20 25 30
   Ser Trp Met His Trp Ile Lys Gln Arg Pro Gly Gln Gly Leu Glu Trp
   35 40 45
   Leu Gly Tyr Ile Asn Pro Ser Ser Gly Tyr Thr Lys Tyr Asn Arg Lys
   50 55 60
   Phe Arg Asp Lys Ala Thr Leu Thr Ala Asp Lys Ser Ser Ser Thr Ala
   65 70 75 80
   Tyr Met Gln Leu Thr Ser Leu Thr Tyr Glu Asp Ser Ala Val Tyr Tyr
   85 90 95
   Cys Ala Arg Gly Gly Asn Gly Tyr Tyr Phe Asp Tyr Trp Gly Gln Gly
   100 105 110
   Thr Thr Leu Thr Val Ser Ser
   115
<210> 2
   <211> 15
   <212> DNA
   <213> Mus musculus
<220>
   <221> CDS
   <222> (1).. (15)
<400> 2
   agc tcc tgg atg cac 15
   Ser Ser Trp Met His
   1 5
<210> 3
   <211> 5
   <212> PRT
   <213> Mus musculus
<400> 3
   Ser Ser Trp Met His
1 5
<210> 4
   <211> 51
   <212> DNA
   <213> Mus musculus
<220>
   <221> CDS
   <222> (1).. (51)
<400> 4
   tac att aat cct agc agt ggt tat act aag tac aat cgg aag ttc agg 48
   Tyr Ile Asn Pro Ser Ser Gly Tyr Thr Lys Tyr Asn Arg Lys Phe Arg
   1 5 10 15
   gac 51
   Asp
<210> 5
   <211> 17
   <212> PRT
   <213> Mus musculus
<400> 5
   Tyr Ile Asn Pro Ser Ser Gly Tyr Thr Lys Tyr Asn Arg Lys Phe Arg
   1 5 10 15
   Asp
<210> 6
   <211> 27
   <212> DNA
   <213> Mus musculus
<220>
   <221> CDS
   <222> (1).. (27)
<400> 6
   ggg ggt aac ggt tac tac ttt gac tac                          27
   Gly Gly Asn Gly Tyr Tyr Phe Asp Tyr
   1               5
<210> 7
   <211> 9
   <212> PRT
   <213> Mus musculus
<400> 7
   Gly Gly Asn Gly Tyr Tyr Phe Asp Tyr
   1               5
<210> 8
   <211> 107
   <212> PRT
   <213> Mus musculus
<400> 8
   Asp Ile Val Met Thr Gln Ser His Lys Phe Met Ser Thr Ser Val Gly
   1               5                   10                  15
   Asp Arg Val Ser Ile Thr Cys Lys Ala Ser Gln Asp Val Gly Thr Ala
               20                  25                  30
   Val Ala Trp Tyr Gln Gln Lys Pro Gly Gln Ser Pro Lys Leu Leu Ile
           35                  40                  45
   Tyr Trp Ala Ser Thr Arg His Thr Gly Val Pro Asp Arg Phe Thr Gly
       50                  55                  60
   Ser Arg Tyr Gly Thr Asp Phe Thr Leu Thr Ile Ser Asn Val Gln Ser
   65                  70                  75                  80
   Glu Asp Leu Ala Asp Tyr Leu Cys Gln Gln Tyr Ser Asn Tyr Ile Thr
                   85                  90                  95
   Phe Gly Gly Gly Thr Lys Leu Glu Leu Lys Arg
               100                 105
<210> 9
   <211> 33
   <212> DNA
   <213> Mus musculus
<220>
   <221> CDS
   <222> (1).. (33)
<400> 9
   aag gcc agt cag gat gtg ggg act gct gta gcc 33
   Lys Ala Ser Gln Asp Val Gly Thr Ala Val Ala
   1 5 10
<210> 10
   <211> 11
   <212> PRT
   <213> Mus musculus
<400> 10
   Lys Ala Ser Gln Asp Val Gly Thr Ala Val Ala
   1 5 10
<210> 11
   <211> 21
   <212> DNA
   <213> Mus musculus
<220>
   <221> CDS
   <222> (1).. (21)
<400> 11
   tgg gca tcc acc cgg cac act 21
   Trp Ala Ser Thr Arg His Thr
   1 5
<210> 12
   <211> 7
   <212> PRT
   <213> Mus musculus
<400> 12
   Trp Ala Ser Thr Arg His Thr
   1 5
<210> 13
   <211> 24
   <212> DNA
   <213> Mus musculus
<220>
   <221> CDS
   <222> (1).. (24)
<400> 13
   cag caa tat agc aac tat atc acg 24
   Gln Gln Tyr Ser Asn Tyr Ile Thr
   1 5
<210> 14
   <211> 8
   <212> PRT
   <213> Mus musculus
<400> 14
   Gln Gln Tyr Ser Asn Tyr Ile Thr
   1 5
<210> 15
   <211> 119
   <212> PRT
   <213> Mus musculus
<400> 15
   Met Gln Val Gln Leu Gln Gln Ser Gly Ala Glu Leu Ala Lys Pro Gly
   1 5 10 15
   Ala Ser Val Lys Leu Ser Cys Lys Ala Ser Gly Tyr Thr Phe Thr Thr
   20 25 30
   Tyr Trp Met His Trp Val Lys Gln Arg Pro Gly Gln Gly Leu Glu Trp
   35 40 45
   Ile Gly Tyr Ile Asn Pro Ser Ser Gly Tyr Thr Lys Tyr Asn Gln Lys
   50 55 60
   Phe Lys Val Lys Ala Thr Leu Thr Ala Asp Lys Ser Ser Ser Thr Ala
   65 70 75 80
   Tyr Met Gln Leu Ser Ser Leu Thr Asp Glu Asp Ser Ala Val Tyr Tyr
   85 90 95
   Cys Ala Asn Gly Asn Leu Gly Tyr Phe Phe Asp Tyr Trp Gly Gln Gly
   100 105 110
   Thr Thr Leu Thr Val Ser Ser
   115
<210> 16
   <211> 5
   <212> PRT
   <213> Mus musculus
<400> 16
   Thr Tyr Trp Met His
   1 5
<210> 17
   <211> 17
   <212> PRT
   <213> Mus musculus
<400> 17
   Tyr Ile Asn Pro Ser Ser Gly Tyr Thr Lys Tyr Asn Gln Lys Phe Lys
   1 5 10 15
   Val
<210> 18
   <211> 9
   <212> PRT
   <213> Mus musculus
<400> 18
   Gly Asn Leu Gly Tyr Phe Phe Asp Tyr
   1 5
<210> 19
   <211> 8
   <212> PRT
   <213> Mus musculus
<400> 19
   Gln Gln Tyr Ser Ser Tyr Leu Thr
   1 5
<210> 20
   <211> 119
   <212> PRT
   <213> Mus musculus
<400> 20
   Met Glu Val Gln Leu Gln Gln Ser Gly Ala Glu Leu Val Lys Pro Gly
   1 5 10 15
   Ala Ser Val Lys Leu Ser Cys Thr Ala Ser Gly Phe Asn Ile Lys Asp
   20 25 30
   Tyr Tyr Met His Trp Ile Lys Gln Arg Pro Gly Gln Gly Leu Glu Trp
   35 40 45
   Leu Gly Tyr Ile Asn Pro Ser Ser Gly Tyr Thr Lys Tyr Asn Arg Lys
   50 55 60
   Phe Arg Asp Lys Ala Thr Leu Thr Ala Asp Lys Ser Ser Ser Thr Ala
   65 70 75 80
   Tyr Met Gln Leu Thr Ser Leu Thr Tyr Glu Asp Ser Ala Val Tyr Tyr
   85 90 95
   Cys Ala Arg Gly Gly Asn Gly Tyr Tyr Leu Asp Tyr Trp Gly Gln Gly
   100 105 110
   Thr Thr Leu Thr Val Ser Ser
   115
<210> 21
   <211> 5
   <212> PRT
   <213> Mus musculus
<400> 21
   Asp Tyr Tyr Met His
   1               5
<210> 22
   <211> 9
   <212> PRT
   <213> Mus musculus
<400> 22
   Gly Gly Asn Gly Tyr Tyr Leu Asp Tyr
   1               5
<210> 23
   <211> 11
   <212> PRT
   <213> Mus musculus
<400> 23
   Lys Ala Ser Gln Asp Val Ser Thr Ala Val Ala
   1               5                   10
<210> 24
   <211> 120
   <212> PRT
   <213> Mus musculus
<400> 24
   Met Gln Val Gln Leu Gln Gln Ser Gly Pro Glu Leu Val Lys Pro Gly
   1               5                   10                  15
   Ala Ser Val Lys Ile Ser Cys Lys Ala Ser Gly Tyr Thr Phe Thr Asp
               20                  25                  30
   Asn Asn Met Asp Trp Val Lys Gln Ser His Gly Lys Gly Leu Glu Trp
           35                  40                  45
   Ile Gly Asp Ile Asn Thr Lys Ser Gly Gly Ser Ile Tyr Asn Gln Lys
       50                  55                  60
   Phe Lys Gly Lys Ala Thr Leu Thr Ile Asp Lys Ser Ser Ser Thr Ala
   65                  70                  75                  80
   Tyr Met Glu Leu Arg Ser Leu Thr Ser Glu Asp Thr Ala Val Tyr Tyr
                   85                  90                  95
   Cys Ala Arg Arg Arg Ser Tyr Gly Tyr Tyr Phe Asp Tyr Trp Gly Gln
               100                 105                 110
   Gly Thr Thr Leu Thr Val Ser Ser
           115                 120
<210> 25
   <211> 15
   <212> DNA
   <213> Mus musculus
<220>
   <221> CDS
   <222> (1).. (15)
<400> 25
   gac aac aac atg gac 15
   Asp Asn Asn Met Asp
   1               5
<210> 26
   <211> 5
   <212> PRT
   <213> Mus musculus
<400> 26
   Asp Asn Asn Met Asp
   1 5
<210> 27
   <211> 51
   <212> DNA
   <213> Mus musculus
<220>
   <221> CDS
   <222> (1).. (51)
<400> 27
   gat att aat act aaa agt ggt ggt tct atc tac aac cag aag ttc aag 48
   Asp Ile Asn Thr Lys Ser Gly Gly Ser Ile Tyr Asn Gln Lys Phe Lys
   1 5 10 15
   ggc 51
   Gly
<210> 28
   <211> 17
   <212> PRT
   <213> Mus musculus
<400> 28
   Asp Ile Asn Thr Lys Ser Gly Gly Ser Ile Tyr Asn Gln Lys Phe Lys
   1 5 10 15
   Gly
<210> 29
   <211> 30
   <212> DNA
   <213> Mus musculus
<220>
   <221> CDS
   <222> (1).. (30)
<400> 29
   agg agg agc tac ggc tac tac ttt gac tac 30
   Arg Arg Ser Tyr Gly Tyr Tyr Phe Asp Tyr
   1 5 10
<210> 30
   <211> 10
   <212> PRT
   <213> Mus musculus
<400> 30
   Arg Arg Ser Tyr Gly Tyr Tyr Phe Asp Tyr
   1 5 10
<210> 31
   <211> 108
   <212> PRT
   <213> Mus musculus
<400> 31
   Asp Ile Val Leu Thr Gln Ser Gln Lys Phe Met Ser Thr Ser Val Gly
   1 5 10 15
   Asp Arg Val Ser Ile Thr Cys Lys Ala Ser Gln Asn Val Gly Thr Ala
   20 25 30
   Val Ala Trp Tyr Gln Gln Lys Pro Gly Gln Ser Pro Lys Ala Leu Ile
   35 40 45
   Tyr Ser Ala Ser Tyr Arg Tyr Ser Gly Val Pro Asp Arg Phe Thr Gly
   50 55 60
   Ser Gly Ser Gly Thr Asp Phe Thr Leu Thr Ile Ser Asn Val Gln Ser
   65 70 75 80
   Glu Asp Leu Ala Glu Tyr Phe Cys Gln Gln Tyr Asn Ser Tyr Pro Leu
   85 90 95
   Thr Phe Gly Ala Gly Thr Lys Leu Glu Ile Lys Arg
   100 105
<210> 32
   <211> 33
   <212> DNA
   <213> Mus musculus
<220>
   <221> CDS
   <222> (1).. (33)
<400> 32
   aag gcc agt cag aat gtg ggt act gct gta gcc                33
   Lys Ala Ser Gln Asn Val Gly Thr Ala Val Ala
   1               5                   10
<210> 33
   <211> 11
   <212> PRT
   <213> Mus musculus
<400> 33
   Lys Ala Ser Gln Asn Val Gly Thr Ala Val Ala
   1               5                   10
<210> 34
   <211> 21
   <212> DNA
   <213> Mus musculus
<220>
   <221> CDS
   <222> (1).. (21)
<400> 34
   tcg gca tcc tac cgg tac agt                21
   Ser Ala Ser Tyr Arg Tyr Ser
   1               5
<210> 35
   <211> 7
   <212> PRT
   <213> Mus musculus
<400> 35
   Ser Ala Ser Tyr Arg Tyr Ser
   1               5
<210> 36
   <211> 27
   <212> DNA
   <213> Mus musculus
<220>
   <221> CDS
   <222> (1).. (27)
<400> 36
   cag caa tat aac agc tat cct ctc acg 27
   Gln Gln Tyr Asn Ser Tyr Pro Leu Thr
   1 5
<210> 37
   <211> 9
   <212> PRT
   <213> Mus musculus
<400> 37
   Gln Gln Tyr Asn Ser Tyr Pro Leu Thr
   1 5
<210> 38
   <211> 22
   <212> DNA
   <213> Artificial
<220>
   <223> An artificially synthesized primer sequence
<400> 38
   cagctatgaa atacctattg cc 22
<210> 39
   <211> 23
   <212> DNA
   <213> Artificial
<220>
   <223> An artificially synthesized primer sequence
<400> 39
   cttttcataa tcaaaatcac cgg 23
<210> 40
   <211> 19
   <212> DNA
   <213> Artificial
<220>
   <223> An artificially synthesized primer sequence
<400> 40
   attgcctacg gcagccgct 19
<210> 41
   <211> 20
   <212> DNA
   <213> Artificial
<220>
   <223> An artificially synthesized primer sequence
<400> 41
   aaatcaccgg aaccagagcc 20
<210> 42
   <211> 24
   <212> DNA
   <213> Artificial
<220>
   <223> An artificially synthesized primer sequence
<400> 42
   ttactcgcgg cccagccggc catg 24
<210> 43
   <211> 28
   <212> DNA
   <213> Artificial
<220>
   <223> An artificially synthesized primer sequence
<400> 43
   ggaattcggc ccccgaggcc cactcacg 28
<210> 44
   <211> 1215
   <212> DNA
   <213> Homo sapiens
<400> 44
   ggcctcgggg gccagctttc tggggcaggc caggcctgac cttggctttg gggcagggag 60
   ggggctaagg tgaggcaggt ggcgccagcc aggtgcacac ccaatgccca tgagcccaga 120
   cactggacgc tgaacctcgc ggacagttaa gaacccaggg gcctctgcgc cctgggccca 180
   gctctgtccc acaccgcggt cacatggcac cacctctctt gcagcttcca ccaagggccc 240
   atccgtcttc cccctggcgc cctgctccag gagcacctcc gagagcacag ccgccctggg 300
   ctgcctggtc aaggactact tccccgaacc ggtgacggtg tcgtggaact caggcgccct 360
   gaccagcggc gtgcacacct tcccggctgt cctacagtcc tcaggactct actccctcag 420
   cagcgtggtg accgtgccct ccagcagctt gggcacgaag acctacacct gcaacgtaga 480
   tcacaagccc agcaacacca aggtggacaa gagagttgag tccaaatatg gtcccccatg 540
   cccaccatgc ccagcacctg agttcctggg gggaccatca gtcttcctgt tccccccaaa 600
   acccaaggac actctcatga tctcccggac ccctgaggtc acgtgcgtgg tggtggacgt 660
   gagccaggaa gaccccgagg tccagttcaa ctggtacgtg gatggcgtgg aggtgcataa 720
   tgccaagaca aagccgcggg aggagcagtt caacagcacg taccgtgtgg tcagcgtcct 780
   caccgtcctg caccaggact ggctgaacgg caaggagtac aagtgcaagg tctccaacaa 840
   aggcctcccg tcctccatcg agaaaaccat ctccaaagcc aaagggcagc cccgagagcc 900
   acaggtgtgc accctgcccc catcccagga ggagatgacc aagaaccagg tcagcctgtg 960
   gtgcctggtc aaaggcttct accccagcga catcgccgtg gagtgggaga gcaatgggca 1020
   gccggagaac aactacaaga ccacgcctcc cgtgctggac tccgacggct ccttcttcct 1080
   ctacagcagg ctaaccgtgg acaagagcag gtggcaggag gggaatgtct tctcatgctc 1140
   cgtgatgcat gaggctctgc acaaccacta cacacagaag agcctctccc tgtctctggg 1200
   taaatgagcg gccgc 1215
<210> 45
   <211> 684
   <212> DNA
   <213> Homo sapiens
<400> 45
   ggcctcgggg gccgaattcc taaactctga gggggtcgga tgacgtggcc attctttgcc 60
   taaagcattg agtttactgc aaggtcagaa aagcatgcaa agccctcaga atggctgcaa 120
   agagctccaa caaaacaatt tagaacttta ttaaggaata gggggaagct aggaagaaac 180
   tcaaaacatc aagattttaa atacgcttct tggtctcctt gctataatta tctgggataa 240
   gcatgctgtt ttctgtctgt ccctaacatg ccctgtgatt atccgcaaac aacacaccca 300
   agggcagaac tttgttactt aaacaccatc ctgtttgctt ctttcctcag gaactgtggc 360
   tgcaccatct gtcttcatct tcccgccatc tgatgagcag ttgaaatctg gaactgcctc 420
   tgttgtgtgc ctgctgaata acttctatcc cagagaggcc aaagtacagt ggaaggtgga 480
   taacgccctc caatcgggta actcccagga gagtgtcaca gagcaggaca gcaaggacag 540
   cacctacagc ctcagcagca ccctgacgct gagcaaagca gactacgaga aacacaaagt 600
   ctacgcctgc gaagtcaccc atcagggcct gagctcgccc gtcacaaaga gcttcaacag 660
   gggagagtgt tagagggcgg ccgc 684
<210> 46
   <211> 1215
   <212> DNA
   <213> Homo sapiens
<400> 46
   ggcctcgggg gcctcccagg ctctgggcag gcacaggcta ggtgccccta acccaggccc      60
   tgcacacaaa ggggcaggtg ctgggctcag acctgccaag agccatatcc gggaggaccc     120
   tgcccctgac ctaagcccac cccaaaggcc aaactctcca ctccctcagc tcggacacct     180
   tctctcctcc cagattccag taactcccaa tcttctctct gcagcttcca ccaagggccc     240
   atccgtcttc cccctggcgc cctgctccag gagcacctcc gagagcacag ccgccctggg     300
   ctgcctggtc aaggactact tccccgaacc ggtgacggtg tcgtggaact caggcgccct     360
   gaccagcggc gtgcacacct tcccggctgt cctacagtcc tcaggactct actccctcag     420
   cagcgtggtg accgtgccct ccagcagctt gggcacgaag acctacacct gcaacgtaga     480
   tcacaagccc agcaacacca aggtggacaa gagagttgag tccaaatatg gtcccccatg     540
   cccaccatgc ccagcacctg agttcctggg gggaccatca gtcttcctgt tccccccaaa     600
   acccaaggac actctcatga tctcccggac ccctgaggtc acgtgcgtgg tggtggacgt     660
   gagccaggaa gaccccgagg tccagttcaa ctggtacgtg gatggcgtgg aggtgcataa     720
   tgccaagaca aagccgcggg aggagcagtt caacagcacg taccgtgtgg tcagcgtcct     780
   caccgtcctg caccaggact ggctgaacgg caaggagtac aagtgcaagg tctccaacaa     840
   aggcctcccg tcctccatcg agaaaaccat ctccaaagcc aaagggcagc cccgagagcc     900
   acaggtgtac accctgcccc catcccagtg cgagatgacc aagaaccagg tcagcctgtc     960
   ctgcgcggtc aaaggcttct atcccagcga catcgccgtg gagtgggaga gcaatgggca    1020
   gccggagaac aactacaaga ccacgcctcc cgtgctggac tccgacggct ccttcttcct    1080
   cgtgagcagg ctaaccgtgg acaagagcag gtggcaggag gggaatgtct tctcatgctc    1140
   cgtgatgcat gaggctctgc acaaccacta cacacagaag agcctctccc tgtctctggg    1200
   taaatgagcg gccgc                                                     1215
<210> 47
   <211> 21
   <212> DNA
   <213> Artificial
<220>
   <223> An artificially synthesized primer sequence
<400> 47
   cgcaaatggg cggtaggcgt g 21
<210> 48
   <211> 18
   <212> DNA
   <213> Artificial
<220>
   <223> An artificially synthesized primer sequence
<400> 48
   tagaaggcac agtcgagg 18
<210> 49
   <211> 24
   <212> DNA
   <213> Artificial
<220>
   <223> An artificially synthesized primer sequence
<400> 49
   ctctgaatac tttcaacaag ttac 24
<210> 50
   <211> 69
   <212> DNA
   <213> Artificial
<220>
   <223> An artificially synthesized primer sequence
<400> 50
   gccatggcgg actacaaaga tattgtgatg acccagtctc acaaattcat gtccacatca 60
   gtaggagac 69
<210> 51
   <211> 69
   <212> DNA
   <213> Artificial
<220>
   <223> An artificially synthesized primer sequence
<400> 51
   ggctacagca gtccccacat cctgactggc cttgcaggtg atgctgaccc tgtctcctac 60
   tgatgtgga 69
<210> 52
   <211> 63
   <212> DNA
   <213> Artificial
<220>
   <223> An artificially synthesized primer sequence
<400> 52
   gtggggactg ctgtagcctg gtatcaacag aaaccagggc aatctcctaa actactgatt 60
   tac 63
<210> 53
   <211> 57
   <212> DNA
   <213> Artificial
<220>
   <223> An artificially synthesized primer sequence
<400> 53
   gaagcgatca gggactccag tgtgccgggt ggatgcccag taaatcagta gtttagg 57
<210> 54
   <211> 57
   <212> DNA
   <213> Artificial
<220>
   <223> An artificially synthesized primer sequence
<400> 54
   ggagtccctg atcgcttcac aggcagtaga tatgggacag atttcactct caccatt 57
<210> 55
   <211> 57
   <212> DNA
   <213> Artificial
<220>
   <223> An artificially synthesized primer sequence
<400> 55
   acagagataa tctgccaggt cttcagactg cacattgcta atggtgagag tgaaatc 57
<210> 56
   <211> 57
   <212> DNA
   <213> Artificial
<220>
   <223> An artificially synthesized primer sequence
<400> 56
   ctggcagatt atctctgtca gcaatatagc aactatatca cgttcggtgg tgggacc 57
<210> 57
   <211> 64
   <212> DNA
   <213> Artificial
<220>
   <223> An artificially synthesized primer sequence
<400> 57
   ggaattcggc ccccgaggcc gacttaccac gtttcagctc cagcttggtc ccaccaccga 60
   acgt 64
<210> 58
   <211> 64
   <212> DNA
   <213> Artificial
<220>
   <223> An artificially synthesized primer sequence
<400> 58
   ggaattcggc ccccgaggcc gacttacctc gtttcagctc cagcttggtc ccaccaccga 60
   acgt 64
<210> 59
   <211> 69
   <212> DNA
   <213> Artificial
<220>
   <223> An artificially synthesized primer sequence
<400> 59
   ggctacagca gtccccacat tctgactggc cttgcaggtg atgctgaccc tgtctcctac 60
   tgatgtgga 69
<210> 60
   <211> 57
   <212> DNA
   <213> Artificial
<220>
   <223> An artificially synthesized primer sequence
<400> 60
   gaagcgatca gggactccac tgtaccggta ggatgccgag taaatcagta gtttagg 57
<210> 61
   <211> 60
   <212> DNA
   <213> Artificial
<220>
   <223> An artificially synthesized primer sequence
<400> 61
   ctggcagatt atctctgtca gcaatataac agctatccac tcacgttcgg tggtgggacc 60
<210> 62
   <211> 69
   <212> DNA
   <213> Artificial
<220>
   <223> An artificially synthesized primer sequence
<400> 62
   ggctacagca gtactcacat cctgactggc cttgcaggtg atgctgaccc tgtctcctac 60
   tgatgtgga 69
<210> 63
   <211> 57
   <212> DNA
   <213> Artificial
<220>
   <223> An artificially synthesized primer sequence
<400> 63
   ctggcagatt atctctgtca gcaatatagc agctatttaa cgttcggtgg tgggacc 57
<210> 64
   <211> 37
   <212> DNA
   <213> Artificial
<220>
   <223> An artificially synthesized primer sequence
<400> 64
   ttactcgcgg cccagccggc catggcggac tacaaag 37
<210> 65
   <211> 17
   <212> DNA
   <213> Artificial
<220>
   <223> An artificially synthesized primer sequence
<400> 65
   ggaattcggc ccccgag 17
<210> 66
   <211> 319
   <212> DNA
   <213> Homo sapiens
<220>
   <221> CDS
   <222> (1).. (318)
<400> 66
   gat att gtg atg acc cag tct cac aaa ttc atg tcc aca tca gta gga 48
   Asp Ile Val Met Thr Gln Ser His Lys Phe Met Ser Thr Ser Val Gly
   1 5 10 15
   gac agg gtc agc atc acc tgc aag gcc agt cag gat gtg ggg act gct 96
   Asp Arg Val Ser Ile Thr Cys Lys Ala Ser Gln Asp Val Gly Thr Ala
   20 25 30
   gta gcc tgg tat caa cag aaa cca ggg caa tct cct aaa cta ctg att 144
   Val Ala Trp Tyr Gln Gln Lys Pro Gly Gln Ser Pro Lys Leu Leu Ile
   35 40 45
   tac tgg gca tcc acc cgg cac act gga gtc cct gat cgc ttc aca ggc 192
   Tyr Trp Ala Ser Thr Arg His Thr Gly Val Pro Asp Arg Phe Thr Gly
   50 55 60
   agt aga tat ggg aca gat ttc act ctc acc att agc aat gtg cag tct 240
   Ser Arg Tyr Gly Thr Asp Phe Thr Leu Thr Ile Ser Asn Val Gln Ser
   65 70 75 80
   gaa gac ctg gca gat tat ctc tgt cag caa tat agc aac tat atc acg 288
   Glu Asp Leu Ala Asp Tyr Leu Cys Gln Gln Tyr Ser Asn Tyr Ile Thr
   85 90 95
   ttc ggt ggt ggg acc aag ctg gag ctg aaa c 319
   Phe Gly Gly Gly Thr Lys Leu Glu Leu Lys
   100 105
<210> 67
   <211> 107
   <212> PRT
   <213> Homo sapiens
<400> 67
   Asp Ile Val Met Thr Gln Ser His Lys Phe Met Ser Thr Ser Val Gly
   1 5 10 15
   Asp Arg Val Ser Ile Thr Cys Lys Ala Ser Gln Asp Val Gly Thr Ala
   20 25 30
   Val Ala Trp Tyr Gln Gln Lys Pro Gly Gln Ser Pro Lys Leu Leu Ile
   35 40 45
   Tyr Trp Ala Ser Thr Arg His Thr Gly Val Pro Asp Arg Phe Thr Gly
   50 55 60
   Ser Arg Tyr Gly Thr Asp Phe Thr Leu Thr Ile Ser Asn Val Gln Ser
   65 70 75 80
   Glu Asp Leu Ala Asp Tyr Leu Cys Gln Gln Tyr Ser Asn Tyr Ile Thr
   85 90 95
   Phe Gly Gly Gly Thr Lys Leu Glu Leu Lys Arg
   100 105
<210> 68
   <211> 319
   <212> DNA
   <213> Homo sapiens
<220>
   <221> CDS
   <222> (1).. (318)
<400> 68
   gat att gtg atg acc cag tct cac aaa ttc atg tcc aca tca gta gga 48
   Asp Ile Val Met Thr Gln Ser His Lys Phe Met Ser Thr Ser Val Gly
   1 5 10 15
   gac agg gtc agc atc acc tgc aag gcc agt cag aat gtg ggg act gct 96
   Asp Arg Val Ser Ile Thr Cys Lys Ala Ser Gln Asn Val Gly Thr Ala
   20 25 30
   gta gcc tgg tat caa cag aaa cca ggg caa tct cct aaa cta ctg att 144
   Val Ala Trp Tyr Gln Gln Lys Pro Gly Gln Ser Pro Lys Leu Leu Ile
   35 40 45
   tac tcg gca tcc tac cgg tac agt gga gtc cct gat cgc ttc aca ggc 192
   Tyr Ser Ala Ser Tyr Arg Tyr Ser Gly Val Pro Asp Arg Phe Thr Gly
   50 55 60
   agt aga tat ggg aca gat ttc act ctc acc att agc aat gtg cag tct 240
   Ser Arg Tyr Gly Thr Asp Phe Thr Leu Thr Ile Ser Asn Val Gln Ser
   65 70 75 80
   gaa gac ctg gca gat tat ctc tgt cag caa tat agc aac tat atc acg 288
   Glu Asp Leu Ala Asp Tyr Leu Cys Gln Gln Tyr Ser Asn Tyr Ile Thr
   85 90 95
   ttc ggt ggt ggg acc aag ctg gag ctg aaa c 319
   Phe Gly Gly Gly Thr Lys Leu Glu Leu Lys
   100 105
<210> 69
   <211> 107
   <212> PRT
   <213> Homo sapiens
<400> 69
   Asp Ile Val Met Thr Gln Ser His Lys Phe Met Ser Thr Ser Val Gly
   1 5 10 15
   Asp Arg Val Ser Ile Thr Cys Lys Ala Ser Gln Asn Val Gly Thr Ala
   20 25 30
   Val Ala Trp Tyr Gln Gln Lys Pro Gly Gln Ser Pro Lys Leu Leu Ile
   35 40 45
   Tyr Ser Ala Ser Tyr Arg Tyr Ser Gly Val Pro Asp Arg Phe Thr Gly
   50 55 60
   Ser Arg Tyr Gly Thr Asp Phe Thr Leu Thr Ile Ser Asn Val Gln Ser
   65 70 75 80
   Glu Asp Leu Ala Asp Tyr Leu Cys Gln Gln Tyr Ser Asn Tyr Ile Thr
   85 90 95
   Phe Gly Gly Gly Thr Lys Leu Glu Leu Lys Arg
   100 105
<210> 70
   <211> 319
   <212> DNA
   <213> Homo sapiens
<220>
   <221> CDS
   <222> (1).. (318)
<400> 70
   gat att gtg atg acc cag tct cac aaa ttc atg tcc aca tca gta gga      48
   Asp Ile Val Met Thr Gln Ser His Lys Phe Met Ser Thr Ser Val Gly
   1               5                   10                  15
   gac agg gtc agc atc acc tgc aag gcc agt cag aat gtg ggg act gct      96
   Asp Arg Val Ser Ile Thr Cys Lys Ala Ser Gln Asn Val Gly Thr Ala
               20                  25                  30
   gta gcc tgg tat caa cag aaa cca ggg caa tct cct aaa cta ctg att      144
   Val Ala Trp Tyr Gln Gln Lys Pro Gly Gln Ser Pro Lys Leu Leu Ile
           35                  40                  45
   tac tgg gca tcc acc cgg cac act gga gtc cct gat cgc ttc aca ggc      192
   Tyr Trp Ala Ser Thr Arg His Thr Gly Val Pro Asp Arg Phe Thr Gly
       50                  55                  60
   agt aga tat ggg aca gat ttc act ctc acc att agc aat gtg cag tct      240
   Ser Arg Tyr Gly Thr Asp Phe Thr Leu Thr Ile Ser Asn Val Gln Ser
   65                  70                  75                  80
   gaa gac ctg gca gat tat ctc tgt cag caa tat agc aac tat atc acg      288
   Glu Asp Leu Ala Asp Tyr Leu Cys Gln Gln Tyr Ser Asn Tyr Ile Thr
                   85                  90                  95
   ttc ggt ggt ggg acc aag ctg gag ctg aaa c                            319
   Phe Gly Gly Gly Thr Lys Leu Glu Leu Lys
               100                 105
<210> 71
   <211> 107
   <212> PRT
   <213> Homo sapiens
<400> 71
   Asp Ile Val Met Thr Gln Ser His Lys Phe Met Ser Thr Ser Val Gly
   1 5 10 15
   Asp Arg Val Ser Ile Thr Cys Lys Ala Ser Gln Asn Val Gly Thr Ala
   20 25 30
   Val Ala Trp Tyr Gln Gln Lys Pro Gly Gln Ser Pro Lys Leu Leu Ile
   35 40 45
   Tyr Trp Ala Ser Thr Arg His Thr Gly Val Pro Asp Arg Phe Thr Gly
   50 55 60
   Ser Arg Tyr Gly Thr Asp Phe Thr Leu Thr Ile Ser Asn Val Gln Ser
   65 70 75 80
   Glu Asp Leu Ala Asp Tyr Leu Cys Gln Gln Tyr Ser Asn Tyr Ile Thr
   85 90 95
   Phe Gly Gly Gly Thr Lys Leu Glu Leu Lys Arg
   100 105
<210> 72
   <211> 319
   <212> DNA
   <213> Homo sapiens
<220>
   <221> CDS
   <222> (1).. (318)
<400> 72
   gat att gtg atg acc cag tct cac aaa ttc atg tcc aca tca gta gga 48
   Asp Ile Val Met Thr Gln Ser His Lys Phe Met Ser Thr Ser Val Gly
   1 5 10 15
   gac agg gtc agc atc acc tgc aag gcc agt cag gat gtg ggg act gct 96
   Asp Arg Val Ser Ile Thr Cys Lys Ala Ser Gln Asp Val Gly Thr Ala
   20 25 30
   gta gcc tgg tat caa cag aaa cca ggg caa tct cct aaa cta ctg att 144
   Val Ala Trp Tyr Gln Gln Lys Pro Gly Gln Ser Pro Lys Leu Leu Ile
   35 40 45
   tac tcg gca tcc tac cgg tac agt gga gtc cct gat cgc ttc aca ggc 192
   Tyr Ser Ala Ser Tyr Arg Tyr Ser Gly Val Pro Asp Arg Phe Thr Gly
   50 55 60
   agt aga tat ggg aca gat ttc act ctc acc att agc aat gtg cag tct 240
   Ser Arg Tyr Gly Thr Asp Phe Thr Leu Thr Ile Ser Asn Val Gln Ser
   65 70 75 80
   gaa gac ctg gca gat tat ctc tgt cag caa tat agc aac tat atc acg 288
   Glu Asp Leu Ala Asp Tyr Leu Cys Gln Gln Tyr Ser Asn Tyr Ile Thr
   85 90 95
   ttc ggt ggt ggg acc aag ctg gag ctg aaa c 319
   Phe Gly Gly Gly Thr Lys Leu Glu Leu Lys
   100 105
<210> 73
   <211> 107
   <212> PRT
   <213> Homo sapiens
<400> 73
   Asp Ile Val Met Thr Gln Ser His Lys Phe Met Ser Thr Ser Val Gly
   1 5 10 15
   Asp Arg Val Ser Ile Thr Cys Lys Ala Ser Gln Asp Val Gly Thr Ala
   20 25 30
   Val Ala Trp Tyr Gln Gln Lys Pro Gly Gln Ser Pro Lys Leu Leu Ile
   35 40 45
   Tyr Ser Ala Ser Tyr Arg Tyr Ser Gly Val Pro Asp Arg Phe Thr Gly
   50 55 60
   Ser Arg Tyr Gly Thr Asp Phe Thr Leu Thr Ile Ser Asn Val Gln Ser
   65 70 75 80
   Glu Asp Leu Ala Asp Tyr Leu Cys Gln Gln Tyr Ser Asn Tyr Ile Thr
   85 90 95
   Phe Gly Gly Gly Thr Lys Leu Glu Leu Lys Arg
   100 105
<210> 74
   <211> 319
   <212> DNA
   <213> Homo sapiens
<220>
   <221> CDS
   <222> (1).. (318)
<400> 74
   gat att gtg atg acc cag tct cac aaa ttc atg tcc aca tca gta gga 48
   Asp Ile Val Met Thr Gln Ser His Lys Phe Met Ser Thr Ser Val Gly
   1 5 10 15
   gac agg gtc agc atc acc tgc aag gcc agt cag gat gtg ggg act gct 96
   Asp Arg Val Ser Ile Thr Cys Lys Ala Ser Gln Asp Val Gly Thr Ala
   20 25 30
   gta gcc tgg tat caa cag aaa cca ggg caa tct cct aaa cta ctg att 144
   Val Ala Trp Tyr Gln Gln Lys Pro Gly Gln Ser Pro Lys Leu Leu Ile
   35 40 45
   tac tgg gca tcc acc cgg cac act gga gtc cct gat cgc ttc aca ggc 192
   Tyr Trp Ala Ser Thr Arg His Thr Gly Val Pro Asp Arg Phe Thr Gly
   50 55 60
   agt aga tat ggg aca gat ttc act ctc acc att agc aat gtg cag tct 240
   Ser Arg Tyr Gly Thr Asp Phe Thr Leu Thr Ile Ser Asn Val Gln Ser
   65 70 75 80
   gaa gac ctg gca gat tat ctc tgt cag caa tat agc agc tat tta acg 288
   Glu Asp Leu Ala Asp Tyr Leu Cys Gln Gln Tyr Ser Ser Tyr Leu Thr
   85 90 95
   ttc ggt ggt ggg acc aag ctg gag ctg aaa c 319
   Phe Gly Gly Gly Thr Lys Leu Glu Leu Lys
   100 105
<210> 75
   <211> 107
   <212> PRT
   <213> Homo sapiens
<400> 75
   Asp Ile Val Met Thr Gln Ser His Lys Phe Met Ser Thr Ser Val Gly
   1 5 10 15
   Asp Arg Val Ser Ile Thr Cys Lys Ala Ser Gln Asp Val Gly Thr Ala
   20 25 30
   Val Ala Trp Tyr Gln Gln Lys Pro Gly Gln Ser Pro Lys Leu Leu Ile
   35 40 45
   Tyr Trp Ala Ser Thr Arg His Thr Gly Val Pro Asp Arg Phe Thr Gly
   50 55 60
   Ser Arg Tyr Gly Thr Asp Phe Thr Leu Thr Ile Ser Asn Val Gln Ser
   65 70 75 80
   Glu Asp Leu Ala Asp Tyr Leu Cys Gln Gln Tyr Ser Ser Tyr Leu Thr
   85 90 95
   Phe Gly Gly Gly Thr Lys Leu Glu Leu Lys Arg
   100 105
<210> 76
   <211> 319
   <212> DNA
   <213> Homo sapiens
<220>
   <221> CDS
   <222> (1).. (318)
<400> 76
   gat att gtg atg acc cag tct cac aaa ttc atg tcc aca tca gta gga      48
   Asp Ile Val Met Thr Gln Ser His Lys Phe Met Ser Thr Ser Val Gly
   1               5                   10                  15
   gac agg gtc agc atc acc tgc aag gcc agt cag aat gtg ggg act gct      96
   Asp Arg Val Ser Ile Thr Cys Lys Ala Ser Gln Asn Val Gly Thr Ala
               20                  25                  30
   gta gcc tgg tat caa cag aaa cca ggg caa tct cct aaa cta ctg att      144
   Val Ala Trp Tyr Gln Gln Lys Pro Gly Gln Ser Pro Lys Leu Leu Ile
           35                  40                  45
   tac tgg gca tcc acc cgg cac act gga gtc cct gat cgc ttc aca ggc      192
   Tyr Trp Ala Ser Thr Arg His Thr Gly Val Pro Asp Arg Phe Thr Gly
       50                  55                  60
   agt aga tat ggg aca gat ttc act ctc acc att agc aat gtg cag tct      240
   Ser Arg Tyr Gly Thr Asp Phe Thr Leu Thr Ile Ser Asn Val Gln Ser
   65                  70                  75                  80
   gaa gac ctg gca gat tat ctc tgt cag caa tat agc agc tat tta acg      288
   Glu Asp Leu Ala Asp Tyr Leu Cys Gln Gln Tyr Ser Ser Tyr Leu Thr
                   85                  90                  95
   ttc ggt ggt ggg acc aag ctg gag ctg aaa c                            319
   Phe Gly Gly Gly Thr Lys Leu Glu Leu Lys
               100                 105
<210> 77
   <211> 107
   <212> PRT
   <213> Homo sapiens
<400> 77
   Asp Ile Val Met Thr Gln Ser His Lys Phe Met Ser Thr Ser Val Gly
   1               5                   10                  15
   Asp Arg Val Ser Ile Thr Cys Lys Ala Ser Gln Asn Val Gly Thr Ala
   20 25 30
   Val Ala Trp Tyr Gln Gln Lys Pro Gly Gln Ser Pro Lys Leu Leu Ile
   35 40 45
   Tyr Trp Ala Ser Thr Arg His Thr Gly Val Pro Asp Arg Phe Thr Gly
   50 55 60
   Ser Arg Tyr Gly Thr Asp Phe Thr Leu Thr Ile Ser Asn Val Gln Ser
   65 70 75 80
   Glu Asp Leu Ala Asp Tyr Leu Cys Gln Gln Tyr Ser Ser Tyr Leu Thr
   85 90 95
   Phe Gly Gly Gly Thr Lys Leu Glu Leu Lys Arg
   100 105
<210> 78
   <211> 319
   <212> DNA
   <213> Homo sapiens
<220>
   <221> CDS
   <222> (1).. (318)
<400> 78
   gat att gtg atg acc cag tct cac aaa ttc atg tcc aca tca gta gga 48
   Asp Ile Val Met Thr Gln Ser His Lys Phe Met Ser Thr Ser Val Gly
   1 5 10 15
   gac agg gtc agc atc acc tgc aag gcc agt cag gat gtg ggg act gct 96
   Asp Arg Val Ser Ile Thr Cys Lys Ala Ser Gln Asp Val Gly Thr Ala
   20 25 30
   gta gcc tgg tat caa cag aaa cca ggg caa tct cct aaa cta ctg att 144
   Val Ala Trp Tyr Gln Gln Lys Pro Gly Gln Ser Pro Lys Leu Leu Ile
   35 40 45
   tac tcg gca tcc tac cgg tac agt gga gtc cct gat cgc ttc aca ggc 192
   Tyr Ser Ala Ser Tyr Arg Tyr Ser Gly Val Pro Asp Arg Phe Thr Gly
   50 55 60
   agt aga tat ggg aca gat ttc act ctc acc att agc aat gtg cag tct 240
   Ser Arg Tyr Gly Thr Asp Phe Thr Leu Thr Ile Ser Asn Val Gln Ser
   65 70 75 80
   gaa gac ctg gca gat tat ctc tgt cag caa tat agc agc tat tta acg 288
   Glu Asp Leu Ala Asp Tyr Leu Cys Gln Gln Tyr Ser Ser Tyr Leu Thr
   85 90 95
   ttc ggt ggt ggg acc aag ctg gag ctg aaa c 319
   Phe Gly Gly Gly Thr Lys Leu Glu Leu Lys
   100 105
<210> 79
   <211> 107
   <212> PRT
   <213> Homo sapiens
<400> 79
   Asp Ile Val Met Thr Gln Ser His Lys Phe Met Ser Thr Ser Val Gly
   1 5 10 15
   Asp Arg Val Ser Ile Thr Cys Lys Ala Ser Gln Asp Val Gly Thr Ala
   20 25 30
   Val Ala Trp Tyr Gln Gln Lys Pro Gly Gln Ser Pro Lys Leu Leu Ile
   35 40 45
   Tyr Ser Ala Ser Tyr Arg Tyr Ser Gly Val Pro Asp Arg Phe Thr Gly
   50 55 60
   Ser Arg Tyr Gly Thr Asp Phe Thr Leu Thr Ile Ser Asn Val Gln Ser
   65 70 75 80
   Glu Asp Leu Ala Asp Tyr Leu Cys Gln Gln Tyr Ser Ser Tyr Leu Thr
   85 90 95
   Phe Gly Gly Gly Thr Lys Leu Glu Leu Lys Arg
   100 105
<210> 80
   <211> 319
   <212> DNA
   <213> Homo sapiens
<220>
   <221> CDS
   <222> (1).. (318)
<400> 80
   gat att gtg atg acc cag tct cac aaa ttc atg tcc aca tca ata gga       48
   Asp Ile Val Met Thr Gln Ser His Lys Phe Met Ser Thr Ser Ile Gly
   1               5                   10                  15
   gac agg gtc agc atc acc tgc aag gcc agt cag aat gtg ggg act gct       96
   Asp Arg Val Ser Ile Thr Cys Lys Ala Ser Gln Asn Val Gly Thr Ala
               20                  25                  30
   gta gcc tgg tat caa cag aaa cca ggg caa tct cct aaa cta ctg att      144
   Val Ala Trp Tyr Gln Gln Lys Pro Gly Gln Ser Pro Lys Leu Leu Ile
           35                  40                  45
   tac tcg gca tcc tac cgg tac agt gga gtc cct gat cgc ttc aca ggc      192
   Tyr Ser Ala Ser Tyr Arg Tyr Ser Gly Val Pro Asp Arg Phe Thr Gly
       50                  55                  60
   agt aga tat ggg aca gat ttc act ctc acc att agc aat gtg cag tct      240
   Ser Arg Tyr Gly Thr Asp Phe Thr Leu Thr Ile Ser Asn Val Gln Ser
   65                  70                  75                  80
   gaa gac ctg gca gat tat ctc tgt cag caa tat agc agc tat tta acg      288
   Glu Asp Leu Ala Asp Tyr Leu Cys Gln Gln Tyr Ser Ser Tyr Leu Thr
                   85                  90                  95
   ttc ggt ggt ggg acc aag ctg gag ctg aaa c                            319
   Phe Gly Gly Gly Thr Lys Leu Glu Leu Lys
               100                 105
<210> 81
   <211> 107
   <212> PRT
   <213> Homo sapiens
<400> 81
   Asp Ile Val Met Thr Gln Ser His Lys Phe Met Ser Thr Ser Ile Gly
   1 5 10 15
   Asp Arg Val Ser Ile Thr Cys Lys Ala Ser Gln Asn Val Gly Thr Ala
   20 25 30
   Val Ala Trp Tyr Gln Gln Lys Pro Gly Gln Ser Pro Lys Leu Leu Ile
   35 40 45
   Tyr Ser Ala Ser Tyr Arg Tyr Ser Gly Val Pro Asp Arg Phe Thr Gly
   50 55 60
   Ser Arg Tyr Gly Thr Asp Phe Thr Leu Thr Ile Ser Asn Val Gln Ser
   65 70 75 80
   Glu Asp Leu Ala Asp Tyr Leu Cys Gln Gln Tyr Ser Ser Tyr Leu Thr
   85 90 95
   Phe Gly Gly Gly Thr Lys Leu Glu Leu Lys Arg
   100 105
<210> 82
   <211> 319
   <212> DNA
   <213> Homo sapiens
<220>
   <221> CDS
   <222> (1).. (318)
<400> 82
   gat att gtg atg acc cag tct cac aaa ttc atg tcc aca tca gta gga        48
   Asp Ile Val Met Thr Gln Ser His Lys Phe Met Ser Thr Ser Val Gly
   1               5                   10                  15
   gac agg gtc agc atc acc tgc aag gcc agt cag gat gtg agt act gct        96
   Asp Arg Val Ser Ile Thr Cys Lys Ala Ser Gln Asp Val Ser Thr Ala
               20                  25                  30
   gta gcc tgg tat caa cag aaa cca ggg caa tct cct aaa cta ctg att       144
   Val Ala Trp Tyr Gln Gln Lys Pro Gly Gln Ser Pro Lys Leu Leu Ile
           35                  40                  45
   tac tgg gca tcc acc cgg cac act gga gtc cct gat cgc ttc aca ggc       192
   Tyr Trp Ala Ser Thr Arg His Thr Gly Val Pro Asp Arg Phe Thr Gly
       50                  55                  60
   agt aga tat ggg aca gat ttc act ctc acc att agc aat gtg cag tct       240
   Ser Arg Tyr Gly Thr Asp Phe Thr Leu Thr Ile Ser Asn Val Gln Ser
   65                  70                  75                  80
   gaa gac ctg gca gat tat ctc tgt cag caa tat agc aac tat atc acg       288
   Glu Asp Leu Ala Asp Tyr Leu Cys Gln Gln Tyr Ser Asn Tyr Ile Thr
                   85                  90                  95
   ttc ggt ggt ggg acc aag ctg gag ctg aaa c                             319
   Phe Gly Gly Gly Thr Lys Leu Glu Leu Lys
               100                 105
<210> 83
   <211> 107
   <212> PRT
   <213> Homo sapiens
<400> 83
   Asp Ile Val Met Thr Gln Ser His Lys Phe Met Ser Thr Ser Val Gly
   1               5                   10                  15
   Asp Arg Val Ser Ile Thr Cys Lys Ala Ser Gln Asp Val Ser Thr Ala
               20                  25                  30
   Val Ala Trp Tyr Gln Gln Lys Pro Gly Gln Ser Pro Lys Leu Leu Ile
   35 40 45
   Tyr Trp Ala Ser Thr Arg His Thr Gly Val Pro Asp Arg Phe Thr Gly
   50 55 60
   Ser Arg Tyr Gly Thr Asp Phe Thr Leu Thr Ile Ser Asn Val Gln Ser
   65 70 75 80
   Glu Asp Leu Ala Asp Tyr Leu Cys Gln Gln Tyr Ser Asn Tyr Ile Thr
   85 90 95
   Phe Gly Gly Gly Thr Lys Leu Glu Leu Lys Arg
   100 105
<210> 84
   <211> 319
   <212> DNA
   <213> Homo sapiens
<220>
   <221> CDS
   <222> (1).. (318)
<400> 84
   gat att gtg atg acc cag tct cac aaa ttc atg tcc aca tca gta gga 48
   Asp Ile Val Met Thr Gln Ser His Lys Phe Met Ser Thr Ser Val Gly
   1 5 10 15
   gac agg gtc agc atc acc tgc aag gcc agt cag gat gtg agt act gct 96
   Asp Arg Val Ser Ile Thr Cys Lys Ala Ser Gln Asp Val Ser Thr Ala
   20 25 30
   gta gcc tgg tat caa cag aaa cca ggg caa tct cct aaa cta ctg att 144
   Val Ala Trp Tyr Gln Gln Lys Pro Gly Gln Ser Pro Lys Leu Leu Ile
   35 40 45
   tac tcg gca tcc tac cgg tac agt gga gtc cct gat cgc ttc aca ggc 192
   Tyr Ser Ala Ser Tyr Arg Tyr Ser Gly Val Pro Asp Arg Phe Thr Gly
   50 55 60
   agt aga tat ggg aca gat ttc act ctc acc att agc aat gtg cag tct 240
   Ser Arg Tyr Gly Thr Asp Phe Thr Leu Thr Ile Ser Asn Val Gln Ser
   65 70 75 80
   gaa gac ctg gca gat tat ctc tgt cag caa tat agc aac tat atc acg 288
   Glu Asp Leu Ala Asp Tyr Leu Cys Gln Gln Tyr Ser Asn Tyr Ile Thr
   85 90 95
   ttc ggt ggt ggg acc aag ctg gag ctg aaa c 319
   Phe Gly Gly Gly Thr Lys Leu Glu Leu Lys
   100 105
<210> 85
   <211> 107
   <212> PRT
   <213> Homo sapiens
<400> 85
   Asp Ile Val Met Thr Gln Ser His Lys Phe Met Ser Thr Ser Val Gly
   1 5 10 15
   Asp Arg Val Ser Ile Thr Cys Lys Ala Ser Gln Asp Val Ser Thr Ala
   20 25 30
   Val Ala Trp Tyr Gln Gln Lys Pro Gly Gln Ser Pro Lys Leu Leu Ile
   35 40 45
   Tyr Ser Ala Ser Tyr Arg Tyr Ser Gly Val Pro Asp Arg Phe Thr Gly
   50 55 60
   Ser Arg Tyr Gly Thr Asp Phe Thr Leu Thr Ile Ser Asn Val Gln Ser
   65 70 75 80
   Glu Asp Leu Ala Asp Tyr Leu Cys Gln Gln Tyr Ser Asn Tyr Ile Thr
   85 90 95
   Phe Gly Gly Gly Thr Lys Leu Glu Leu Lys Arg
   100 105
<210> 86
   <211> 322
   <212> DNA
   <213> Homo sapiens
<220>
   <221> CDS
   <222> (1).. (321)
<400> 86
   gat att gtg atg acc cag tct cac aaa ttc atg tcc aca tca gta gga       48
   Asp Ile Val Met Thr Gln Ser His Lys Phe Met Ser Thr Ser Val Gly
   1               5                   10                  15
   gac agg gtc agc atc acc tgc aag gcc agt cag gat gtg ggg act gct       96
   Asp Arg Val Ser Ile Thr Cys Lys Ala Ser Gln Asp Val Gly Thr Ala
               20                  25                  30
   gta gcc tgg tat caa cag aaa cca ggg caa tct cct aaa cta ctg att      144
   Val Ala Trp Tyr Gln Gln Lys Pro Gly Gln Ser Pro Lys Leu Leu Ile
           35                  40                  45
   tac tgg gca tcc acc cgg cac act gga gtc cct gat cgc ttc aca ggc      192
   Tyr Trp Ala Ser Thr Arg His Thr Gly Val Pro Asp Arg Phe Thr Gly
       50                  55                  60
   agt aga tat ggg aca gat ttc act ctc acc att agc aat gtg cag tct      240
   Ser Arg Tyr Gly Thr Asp Phe Thr Leu Thr Ile Ser Asn Val Gln Ser
   65                  70                  75                  80
   gaa gac ctg gca gat tat ctc tgt cag caa tat agc aac tat atc acg      288
   Glu Asp Leu Ala Asp Tyr Leu Cys Gln Gln Tyr Ser Asn Tyr Ile Thr
                   85                  90                  95
   ttc ggt ggt ggg acc aag ctg gag ctg aaa cga g                        322
   Phe Gly Gly Gly Thr Lys Leu Glu Leu Lys Arg
               100                 105
<210> 87
   <211> 108
   <212> PRT
   <213> Homo sapiens
<400> 87
   Asp Ile Val Met Thr Gln Ser His Lys Phe Met Ser Thr Ser Val Gly
   1 5 10 15
   Asp Arg Val Ser Ile Thr Cys Lys Ala Ser Gln Asp Val Gly Thr Ala
   20 25 30
   Val Ala Trp Tyr Gln Gln Lys Pro Gly Gln Ser Pro Lys Leu Leu Ile
   35 40 45
   Tyr Trp Ala Ser Thr Arg His Thr Gly Val Pro Asp Arg Phe Thr Gly
   50 55 60
   Ser Arg Tyr Gly Thr Asp Phe Thr Leu Thr Ile Ser Asn Val Gln Ser
   65 70 75 80
   Glu Asp Leu Ala Asp Tyr Leu Cys Gln Gln Tyr Ser Asn Tyr Ile Thr
   85 90 95
   Phe Gly Gly Gly Thr Lys Leu Glu Leu Lys Arg Gly
   100 105
<210> 88
   <211> 322
   <212> DNA
   <213> Homo sapiens
<220>
   <221> CDS
   <222> (1).. (321)
<400> 88
   gat att gtg atg acc cag tct cac aaa ttc atg tcc aca tca gta gga 48
   Asp Ile Val Met Thr Gln Ser His Lys Phe Met Ser Thr Ser Val Gly
   1 5 10 15
   gac agg gtc agc atc acc tgc aag gcc agt cag aat gtg ggg act gct 96
   Asp Arg Val Ser Ile Thr Cys Lys Ala Ser Gln Asn Val Gly Thr Ala
   20 25 30
   gta gcc tgg tat caa cag aaa cca ggg caa tct cct aaa cta ctg att 144
   Val Ala Trp Tyr Gln Gln Lys Pro Gly Gln Ser Pro Lys Leu Leu Ile
   35 40 45
   tac tcg gca tcc tac cgg tac agt gga gtc cct gat cgc ttc aca ggc 192
   Tyr Ser Ala Ser Tyr Arg Tyr Ser Gly Val Pro Asp Arg Phe Thr Gly
   50 55 60
   agt aga tat ggg aca gat ttc act ctc acc att agc aat gtg cag tct 240
   Ser Arg Tyr Gly Thr Asp Phe Thr Leu Thr Ile Ser Asn Val Gln Ser
   65 70 75 80
   gaa gac ctg gca gat tat ctc tgt cag caa tat agc aac tat atc acg 288
   Glu Asp Leu Ala Asp Tyr Leu Cys Gln Gln Tyr Ser Asn Tyr Ile Thr
   85 90 95
   ttc ggt ggt ggg acc aag ctg gag ctg aaa cga g 322
   Phe Gly Gly Gly Thr Lys Leu Glu Leu Lys Arg
   100 105
<210> 89
   <211> 108
   <212> PRT
   <213> Homo sapiens
<400> 89
   Asp Ile Val Met Thr Gln Ser His Lys Phe Met Ser Thr Ser Val Gly
   1 5 10 15
   Asp Arg Val Ser Ile Thr Cys Lys Ala Ser Gln Asn Val Gly Thr Ala
   20 25 30
   Val Ala Trp Tyr Gln Gln Lys Pro Gly Gln Ser Pro Lys Leu Leu Ile
   35 40 45
   Tyr Ser Ala Ser Tyr Arg Tyr Ser Gly Val Pro Asp Arg Phe Thr Gly
   50 55 60
   Ser Arg Tyr Gly Thr Asp Phe Thr Leu Thr Ile Ser Asn Val Gln Ser
   65 70 75 80
   Glu Asp Leu Ala Asp Tyr Leu Cys Gln Gln Tyr Ser Asn Tyr Ile Thr
   85 90 95
   Phe Gly Gly Gly Thr Lys Leu Glu Leu Lys Arg Gly
   100 105
<210> 90
   <211> 322
   <212> DNA
   <213> Homo sapiens
<220>
   <221> CDS
   <222> (1).. (321)
<400> 90
   gat att gtg atg acc cag tct cac aaa ttc atg tcc aca tca gta gga 48
   Asp Ile Val Met Thr Gln Ser His Lys Phe Met Ser Thr Ser Val Gly
   1 5 10 15
   gac agg gtc agc atc acc tgc aag gcc agt cag aat gtg ggg act gct 96
   Asp Arg Val Ser Ile Thr Cys Lys Ala Ser Gln Asn Val Gly Thr Ala
   20 25 30
   gta gcc tgg tat caa cag aaa cca ggg caa tct cct aaa cta ctg att 144
   Val Ala Trp Tyr Gln Gln Lys Pro Gly Gln Ser Pro Lys Leu Leu Ile
   35 40 45
   tac tgg gca tcc acc cgg cac act gga gtc cct gat cgc ttc aca ggc 192
   Tyr Trp Ala Ser Thr Arg His Thr Gly Val Pro Asp Arg Phe Thr Gly
   50 55 60
   agt aga tat ggg aca gat ttc act ctc acc att agc aat gtg cag tct 240
   Ser Arg Tyr Gly Thr Asp Phe Thr Leu Thr Ile Ser Asn Val Gln Ser
   65 70 75 80
   gaa gac ctg gca gat tat ctc tgt cag caa tat agc aac tat atc acg 288
   Glu Asp Leu Ala Asp Tyr Leu Cys Gln Gln Tyr Ser Asn Tyr Ile Thr
   85 90 95
   ttc ggt ggt ggg acc aag ctg gag ctg aaa cga g 322
   Phe Gly Gly Gly Thr Lys Leu Glu Leu Lys Arg
   100 105
<210> 91
   <211> 108
   <212> PRT
   <213> Homo sapiens
<400> 91
   Asp Ile Val Met Thr Gln Ser His Lys Phe Met Ser Thr Ser Val Gly
   1 5 10 15
   Asp Arg Val Ser Ile Thr Cys Lys Ala Ser Gln Asn Val Gly Thr Ala
   20 25 30
   Val Ala Trp Tyr Gln Gln Lys Pro Gly Gln Ser Pro Lys Leu Leu Ile
   35 40 45
   Tyr Trp Ala Ser Thr Arg His Thr Gly Val Pro Asp Arg Phe Thr Gly
   50 55 60
   Ser Arg Tyr Gly Thr Asp Phe Thr Leu Thr Ile Ser Asn Val Gln Ser
   65 70 75 80
   Glu Asp Leu Ala Asp Tyr Leu Cys Gln Gln Tyr Ser Asn Tyr Ile Thr
   85 90 95
   Phe Gly Gly Gly Thr Lys Leu Glu Leu Lys Arg Gly
   100 105
<210> 92
   <211> 322
   <212> DNA
   <213> Homo sapiens
<220>
   <221> CDS
   <222> (1).. (321)
<400> 92
   gat att gtg atg acc cag tct cac aaa ttc atg tcc aca tca gta gga      48
   Asp Ile Val Met Thr Gln Ser His Lys Phe Met Ser Thr Ser Val Gly
   1               5                   10                  15
   gac agg gtc agc atc acc tgc aag gcc agt cag gat gtg ggg act gct      96
   Asp Arg Val Ser Ile Thr Cys Lys Ala Ser Gln Asp Val Gly Thr Ala
               20                  25                  30
   gta gcc tgg tat caa cag aaa cca ggg caa tct cct aaa cta ctg att      144
   Val Ala Trp Tyr Gln Gln Lys Pro Gly Gln Ser Pro Lys Leu Leu Ile
           35                  40                  45
   tac tcg gca tcc tac cgg tac agt gga gtc cct gat cgc ttc aca ggc      192
   Tyr Ser Ala Ser Tyr Arg Tyr Ser Gly Val Pro Asp Arg Phe Thr Gly
       50                  55                  60
   agt aga tat ggg aca gat ttc act ctc acc att agc aat gtg cag tct      240
   Ser Arg Tyr Gly Thr Asp Phe Thr Leu Thr Ile Ser Asn Val Gln Ser
   65                  70                  75                  80
   gaa gac ctg gca gat tat ctc tgt cag caa tat agc aac tat atc acg      288
   Glu Asp Leu Ala Asp Tyr Leu Cys Gln Gln Tyr Ser Asn Tyr Ile Thr
                   85                  90                  95
   ttc ggt ggt ggg acc aag ctg gag ctg aaa cga g                        322
   Phe Gly Gly Gly Thr Lys Leu Glu Leu Lys Arg
               100                 105
<210> 93
   <211> 108
   <212> PRT
   <213> Homo sapiens
<400> 93
   Asp Ile Val Met Thr Gln Ser His Lys Phe Met Ser Thr Ser Val Gly
   1               5                   10                  15
   Asp Arg Val Ser Ile Thr Cys Lys Ala Ser Gln Asp Val Gly Thr Ala
   20 25 30
   Val Ala Trp Tyr Gln Gln Lys Pro Gly Gln Ser Pro Lys Leu Leu Ile
   35 40 45
   Tyr Ser Ala Ser Tyr Arg Tyr Ser Gly Val Pro Asp Arg Phe Thr Gly
   50 55 60
   Ser Arg Tyr Gly Thr Asp Phe Thr Leu Thr Ile Ser Asn Val Gln Ser
   65 70 75 80
   Glu Asp Leu Ala Asp Tyr Leu Cys Gln Gln Tyr Ser Asn Tyr Ile Thr
   85 90 95
   Phe Gly Gly Gly Thr Lys Leu Glu Leu Lys Arg Gly
   100 105
<210> 94
   <211> 322
   <212> DNA
   <213> Homo sapiens
<220>
   <221> CDS
   <222> (1).. (321)
<400> 94
   gat att gtg atg acc cag tct cac aaa ttc atg tcc aca tca gta gga 48
   Asp Ile Val Met Thr Gln Ser His Lys Phe Met Ser Thr Ser Val Gly
   1 5 10 15
   gac agg gtc agc atc acc tgc aag gcc agt cag gat gtg ggg act gct 96
   Asp Arg Val Ser Ile Thr Cys Lys Ala Ser Gln Asp Val Gly Thr Ala
   20 25 30
   gta gcc tgg tat caa cag aaa cca ggg caa tct cct aaa cta ctg att 144
   Val Ala Trp Tyr Gln Gln Lys Pro Gly Gln Ser Pro Lys Leu Leu Ile
   35 40 45
   tac tgg gca tcc acc cgg cac act gga gtc cct gat cgc ttc aca ggc 192
   Tyr Trp Ala Ser Thr Arg His Thr Gly Val Pro Asp Arg Phe Thr Gly
   50 55 60
   agt aga tat ggg aca gat ttc act ctc acc att agc aat gtg cag tct 240
   Ser Arg Tyr Gly Thr Asp Phe Thr Leu Thr Ile Ser Asn Val Gln Ser
   65 70 75 80
   gaa gac ctg gca gat tat ctc tgt cag caa tat agc agc tat tta acg 288
   Glu Asp Leu Ala Asp Tyr Leu Cys Gln Gln Tyr Ser Ser Tyr Leu Thr
   85 90 95
   ttc ggt ggt ggg acc aag ctg gag ctg aaa cga g 322
   Phe Gly Gly Gly Thr Lys Leu Glu Leu Lys Arg
   100 105
<210> 95
   <211> 108
   <212> PRT
   <213> Homo sapiens
<400> 95
   Asp Ile Val Met Thr Gln Ser His Lys Phe Met Ser Thr Ser Val Gly
   1 5 10 15
   Asp Arg Val Ser Ile Thr Cys Lys Ala Ser Gln Asp Val Gly Thr Ala
   20 25 30
   Val Ala Trp Tyr Gln Gln Lys Pro Gly Gln Ser Pro Lys Leu Leu Ile
   35 40 45
   Tyr Trp Ala Ser Thr Arg His Thr Gly Val Pro Asp Arg Phe Thr Gly
   50 55 60
   Ser Arg Tyr Gly Thr Asp Phe Thr Leu Thr Ile Ser Asn Val Gln Ser
   65 70 75 80
   Glu Asp Leu Ala Asp Tyr Leu Cys Gln Gln Tyr Ser Ser Tyr Leu Thr
   85 90 95
   Phe Gly Gly Gly Thr Lys Leu Glu Leu Lys Arg Gly
   100 105
<210> 96
   <211> 322
   <212> DNA
   <213> Homo sapiens
<220>
   <221> CDS
   <222> (1).. (321)
<400> 96
   gat att gtg atg acc cag tct cac aaa ttc atg tcc aca tca gta gga       48
   Asp Ile Val Met Thr Gln Ser His Lys Phe Met Ser Thr Ser Val Gly
   1               5                   10                  15
   gac agg gtc agc atc acc tgc aag gcc agt cag aat gtg ggg act gct       96
   Asp Arg Val Ser Ile Thr Cys Lys Ala Ser Gln Asn Val Gly Thr Ala
               20                  25                  30
   gta gcc tgg tat caa cag aaa cca ggg caa tct cct aaa cta ctg att      144
   Val Ala Trp Tyr Gln Gln Lys Pro Gly Gln Ser Pro Lys Leu Leu Ile
           35                  40                  45
   tac tgg gca tcc acc cgg cac act gga gtc cct gat cgc ttc aca ggc      192
   Tyr Trp Ala Ser Thr Arg His Thr Gly Val Pro Asp Arg Phe Thr Gly
       50                  55                  60
   agt aga tat ggg aca gat ttc act ctc acc att agc aat gtg cag tct      240
   Ser Arg Tyr Gly Thr Asp Phe Thr Leu Thr Ile Ser Asn Val Gln Ser
   65                  70                  75                  80
   gaa gac ctg gca gat tat ctc tgt cag caa tat agc agc tat tta acg      288
   Glu Asp Leu Ala Asp Tyr Leu Cys Gln Gln Tyr Ser Ser Tyr Leu Thr
                   85                  90                  95
   ttc ggt ggt ggg acc aag ctg gag ctg aaa cgt g                        322
   Phe Gly Gly Gly Thr Lys Leu Glu Leu Lys Arg
   100 105
<210> 97
   <211> 108
   <212> PRT
   <213> Homo sapiens
<400> 97
   Asp Ile Val Met Thr Gln Ser His Lys Phe Met Ser Thr Ser Val Gly
   1 5 10 15
   Asp Arg Val Ser Ile Thr Cys Lys Ala Ser Gln Asn Val Gly Thr Ala
   20 25 30
   Val Ala Trp Tyr Gln Gln Lys Pro Gly Gln Ser Pro Lys Leu Leu Ile
   35 40 45
   Tyr Trp Ala Ser Thr Arg His Thr Gly Val Pro Asp Arg Phe Thr Gly
   50 55 60
   Ser Arg Tyr Gly Thr Asp Phe Thr Leu Thr Ile Ser Asn Val Gln Ser
   65 70 75 80
   Glu Asp Leu Ala Asp Tyr Leu Cys Gln Gln Tyr Ser Ser Tyr Leu Thr
   85 90 95
   Phe Gly Gly Gly Thr Lys Leu Glu Leu Lys Arg Gly
   100 105
<210> 98
   <211> 322
   <212> DNA
   <213> Homo sapiens
<220>
   <221> CDS
   <222> (1)..(321)
<400> 98
   gat att gtg atg acc cag tct cac aaa ttc atg tcc aca tca gta gga 48
   Asp Ile Val Met Thr Gln Ser His Lys Phe Met Ser Thr Ser Val Gly
   1 5 10 15
   gac agg gtc agc atc acc tgc aag gcc agt cag gat gtg ggg act gct 96
   Asp Arg Val Ser Ile Thr Cys Lys Ala Ser Gln Asp Val Gly Thr Ala
   20 25 30
   gta gcc tgg tat caa cag aaa cca ggg caa tct cct aaa cta ctg att 144
   Val Ala Trp Tyr Gln Gln Lys Pro Gly Gln Ser Pro Lys Leu Leu Ile
   35 40 45
   tac tcg gca tcc tac cgg tac agt gga gtc cct gat cgc ttc aca ggc 192
   Tyr Ser Ala Ser Tyr Arg Tyr Ser Gly Val Pro Asp Arg Phe Thr Gly
   50 55 60
   agt aga tat ggg aca gat ttc act ctc acc att agc aat gtg cag tct 240
   Ser Arg Tyr Gly Thr Asp Phe Thr Leu Thr Ile Ser Asn Val Gln Ser
   65 70 75 80
   gaa gac ctg gca gat tat ctc tgt cag caa tat agc agc tat tta acg 288
   Glu Asp Leu Ala Asp Tyr Leu Cys Gln Gln Tyr Ser Ser Tyr Leu Thr
   85 90 95
   ttc ggt ggt ggg acc aag ctg gag ctg aaa cgt g 322
   Phe Gly Gly Gly Thr Lys Leu Glu Leu Lys Arg
   100 105
<210> 99
   <211> 108
   <212> PRT
   <213> Homo sapiens
<400> 99
   Asp Ile Val Met Thr Gln Ser His Lys Phe Met Ser Thr Ser Val Gly
   1 5 10 15
   Asp Arg Val Ser Ile Thr Cys Lys Ala Ser Gln Asp Val Gly Thr Ala
   20 25 30
   Val Ala Trp Tyr Gln Gln Lys Pro Gly Gln Ser Pro Lys Leu Leu Ile
   35 40 45
   Tyr Ser Ala Ser Tyr Arg Tyr Ser Gly Val Pro Asp Arg Phe Thr Gly
   50 55 60
   Ser Arg Tyr Gly Thr Asp Phe Thr Leu Thr Ile Ser Asn Val Gln Ser
   65 70 75 80
   Glu Asp Leu Ala Asp Tyr Leu Cys Gln Gln Tyr Ser Ser Tyr Leu Thr
   85 90 95
   Phe Gly Gly Gly Thr Lys Leu Glu Leu Lys Arg Gly
   100 105
<210> 100
   <211> 322
   <212> DNA
   <213> Homo sapiens
<220>
   <221> CDS
   <222> (1).. (321)
<400> 100
   gat att gtg atg acc cag tct cac aaa ttc atg tcc aca tca ata gga 48
   Asp Ile Val Met Thr Gln Ser His Lys Phe Met Ser Thr Ser Ile Gly
   1 5 10 15
   gac agg gtc agc atc acc tgc aag gcc agt cag aat gtg ggg act gct 96
   Asp Arg Val Ser Ile Thr Cys Lys Ala Ser Gln Asn Val Gly Thr Ala
   20 25 30
   gta gcc tgg tat caa cag aaa cca ggg caa tct cct aaa cta ctg att 144
   Val Ala Trp Tyr Gln Gln Lys Pro Gly Gln Ser Pro Lys Leu Leu Ile
   35 40 45
   tac tcg gca tcc tac cgg tac agt gga gtc cct gat cgc ttc aca ggc 192
   Tyr Ser Ala Ser Tyr Arg Tyr Ser Gly Val Pro Asp Arg Phe Thr Gly
   50 55 60
   agt aga tat ggg aca gat ttc act ctc acc att agc aat gtg cag tct 240
   Ser Arg Tyr Gly Thr Asp Phe Thr Leu Thr Ile Ser Asn Val Gln Ser
   65 70 75 80
   gaa gac ctg gca gat tat ctc tgt cag caa tat agc agc tat tta acg 288
   Glu Asp Leu Ala Asp Tyr Leu Cys Gln Gln Tyr Ser Ser Tyr Leu Thr
   85 90 95
   ttc ggt ggt ggg acc aag ctg gag ctg aaa cgt g 322
   Phe Gly Gly Gly Thr Lys Leu Glu Leu Lys Arg
   100 105
<210> 101
   <211> 108
   <212> PRT
   <213> Homo sapiens
<400> 101
   Asp Ile Val Met Thr Gln Ser His Lys Phe Met Ser Thr Ser Ile Gly
   1 5 10 15
   Asp Arg Val Ser Ile Thr Cys Lys Ala Ser Gln Asn Val Gly Thr Ala
   20 25 30
   Val Ala Trp Tyr Gln Gln Lys Pro Gly Gln Ser Pro Lys Leu Leu Ile
   35 40 45
   Tyr Ser Ala Ser Tyr Arg Tyr Ser Gly Val Pro Asp Arg Phe Thr Gly
   50 55 60
   Ser Arg Tyr Gly Thr Asp Phe Thr Leu Thr Ile Ser Asn Val Gln Ser
   65 70 75 80
   Glu Asp Leu Ala Asp Tyr Leu Cys Gln Gln Tyr Ser Ser Tyr Leu Thr
   85 90 95
   Phe Gly Gly Gly Thr Lys Leu Glu Leu Lys Arg Gly
   100 105
<210> 102
   <211> 322
   <212> DNA
   <213> Homo sapiens
<220>
   <221> CDS
   <222> (1).. (321)
<400> 102
   gat att gtg atg acc cag tct cac aaa ttc atg tcc aca tca gta gga      48
   Asp Ile Val Met Thr Gln Ser His Lys Phe Met Ser Thr Ser Val Gly
   1               5                   10                  15
   gac agg gtc agc atc acc tgc aag gcc agt cag gat gtg agt act gct      96
   Asp Arg Val Ser Ile Thr Cys Lys Ala Ser Gln Asp Val Ser Thr Ala
               20                  25                  30
   gta gcc tgg tat caa cag aaa cca ggg caa tct cct aaa cta ctg att      144
   Val Ala Trp Tyr Gln Gln Lys Pro Gly Gln Ser Pro Lys Leu Leu Ile
           35                  40                  45
   tac tgg gca tcc acc cgg cac act gga gtc cct gat cgc ttc aca ggc      192
   Tyr Trp Ala Ser Thr Arg His Thr Gly Val Pro Asp Arg Phe Thr Gly
       50                  55                  60
   agt aga tat ggg aca gat ttc act ctc acc att agc aat gtg cag tct      240
   Ser Arg Tyr Gly Thr Asp Phe Thr Leu Thr Ile Ser Asn Val Gln Ser
   65                  70                  75                  80
   gaa gac ctg gca gat tat ctc tgt cag caa tat agc aac tat atc acg      288
   Glu Asp Leu Ala Asp Tyr Leu Cys Gln Gln Tyr Ser Asn Tyr Ile Thr
                   85                  90                  95
   ttc ggt ggt ggg acc aag ctg gag ctg aaa cgt g                        322
   Phe Gly Gly Gly Thr Lys Leu Glu Leu Lys Arg
               100                 105
<210> 103
   <211> 108
   <212> PRT
   <213> Homo sapiens
<400> 103
   Asp Ile Val Met Thr Gln Ser His Lys Phe Met Ser Thr Ser Val Gly
   1 5 10 15
   Asp Arg Val Ser Ile Thr Cys Lys Ala Ser Gln Asp Val Ser Thr Ala
   20 25 30
   Val Ala Trp Tyr Gln Gln Lys Pro Gly Gln Ser Pro Lys Leu Leu Ile
   35 40 45
   Tyr Trp Ala Ser Thr Arg His Thr Gly Val Pro Asp Arg Phe Thr Gly
   50 55 60
   Ser Arg Tyr Gly Thr Asp Phe Thr Leu Thr Ile Ser Asn Val Gln Ser
   65 70 75 80
   Glu Asp Leu Ala Asp Tyr Leu Cys Gln Gln Tyr Ser Asn Tyr Ile Thr
   85 90 95
   Phe Gly Gly Gly Thr Lys Leu Glu Leu Lys Arg Gly
   100 105
<210> 104
   <211> 322
   <212> DNA
   <213> Homo sapiens
<220>
   <221> CDS
   <222> (1).. (321)
<400> 104
   gat att gtg atg acc cag tct cac aaa ttc atg tcc aca tca gta gga 48
   Asp Ile Val Met Thr Gln Ser His Lys Phe Met Ser Thr Ser Val Gly
   1 5 10 15
   gac agg gtc agc atc acc tgc aag gcc agt cag gat gtg agt act gct 96
   Asp Arg Val Ser Ile Thr Cys Lys Ala Ser Gln Asp Val Ser Thr Ala
   20 25 30
   gta gcc tgg tat caa cag aaa cca ggg caa tct cct aaa cta ctg att 144
   Val Ala Trp Tyr Gln Gln Lys Pro Gly Gln Ser Pro Lys Leu Leu Ile
   35 40 45
   tac tcg gca tcc tac cgg tac agt gga gtc cct gat cgc ttc aca ggc 192
   Tyr Ser Ala Ser Tyr Arg Tyr Ser Gly Val Pro Asp Arg Phe Thr Gly
   50 55 60
   agt aga tat ggg aca gat ttc act ctc acc att agc aat gtg cag tct 240
   Ser Arg Tyr Gly Thr Asp Phe Thr Leu Thr Ile Ser Asn Val Gln Ser
   65 70 75 80
   gaa gac ctg gca gat tat ctc tgt cag caa tat agc aac tat atc acg 288
   Glu Asp Leu Ala Asp Tyr Leu Cys Gln Gln Tyr Ser Asn Tyr Ile Thr
   85 90 95
   ttc ggt ggt ggg acc aag ctg gag ctg aaa cga g 322
   Phe Gly Gly Gly Thr Lys Leu Glu Leu Lys Arg
   100 105
<210> 105
   <211> 108
   <212> PRT
   <213> Homo sapiens
<400> 105
   Asp Ile Val Met Thr Gln Ser His Lys Phe Met Ser Thr Ser Val Gly
   1 5 10 15
   Asp Arg Val Ser Ile Thr Cys Lys Ala Ser Gln Asp Val Ser Thr Ala
   20 25 30
   Val Ala Trp Tyr Gln Gln Lys Pro Gly Gln Ser Pro Lys Leu Leu Ile
   35 40 45
   Tyr Ser Ala Ser Tyr Arg Tyr Ser Gly Val Pro Asp Arg Phe Thr Gly
   50 55 60
   Ser Arg Tyr Gly Thr Asp Phe Thr Leu Thr Ile Ser Asn Val Gln Ser
   65 70 75 80
   Glu Asp Leu Ala Asp Tyr Leu Cys Gln Gln Tyr Ser Asn Tyr Ile Thr
   85 90 95
   Phe Gly Gly Gly Thr Lys Leu Glu Leu Lys Arg Gly
   100 105
<210> 106
   <211> 1215
   <212> DNA
   <213> Homo sapiens
<400> 106
   ggcctcgggg gccagctttc tggggcaggc caggcctgac cttggctttg gggcagggag 60
   ggggctaagg tgaggcaggt ggcgccagcc aggtgcacac ccaatgccca tgagcccaga 120
   cactggacgc tgaacctcgc ggacagttaa gaacccaggg gcctctgcgc cctgggccca 180
   gctctgtccc acaccgcggt cacatggcac cacctctctt gcagcttcca ccaagggccc 240
   atccgtcttc cccctggcgc cctgctccag gagcacctcc gagagcacag ccgccctggg 300
   ctgcctggtc aaggactact tccccgaacc ggtgacggtg tcgtggaact caggcgccct 360
   gaccagcggc gtgcacacct tcccggctgt cctacagtcc tcaggactct actccctcag 420
   cagcgtggtg accgtgccct ccagcagctt gggcacgaag acctacacct gcaacgtaga 480
   tcacaagccc agcaacacca aggtggacaa gagagttgag tccaaatatg gtcccccatg 540
   cccaccatgc ccagcacctg agttcctggg gggaccatca gtcttcctgt tccccccaaa 600
   acccaaggac actctcatga tctcccggac ccctgaggtc acgtgcgtgg tggtggacgt 660
   gagccaggaa gaccccgagg tccagttcaa ctggtacgtg gatggcgtgg aggtgcataa 720
   tgccaagaca aagccgcggg aggagcagtt caacagcacg taccgtgtgg tcagcgtcct 780
   caccgtcctg caccaggact ggctgaacgg caaggagtac aagtgcaagg tctccaacaa 840
   aggcctcccg tcctccatcg agaaaaccat ctccaaagcc aaagggcagc cccgagagcc 900
   acaggtgtgc accctgcccc catcccagga ggagatgacc aagaaccagg tcagcctgtg 960
   gtgcctggtc aaaggcttct accccagcga catcgccgtg gagtgggaga gcaatgggca 1020
   gccggagaac aactacaaga ccacgcctcc cgtgctggac tccgacggct ccttcttcct 1080
   ctacagcagg ctaaccgtgg acaagagcag gtggcaggag gggaatgtct tctcatgctc 1140
   cgtgatgcat gaggctctgc acaaccacta cacacagaag agcctctccc tgtctctggg 1200
   taaatgagcg gccgc 1215
<210> 107
   <211> 684
   <212> DNA
   <213> Homo sapiens
<400> 107
   ggcctcgggg gccgaattcc taaactctga gggggtcgga tgacgtggcc attctttgcc 60
   taaagcattg agtttactgc aaggtcagaa aagcatgcaa agccctcaga atggctgcaa 120
   agagctccaa caaaacaatt tagaacttta ttaaggaata gggggaagct aggaagaaac 180
   tcaaaacatc aagattttaa atacgcttct tggtctcctt gctataatta tctgggataa 240
   gcatgctgtt ttctgtctgt ccctaacatg ccctgtgatt atccgcaaac aacacaccca 300
   agggcagaac tttgttactt aaacaccatc ctgtttgctt ctttcctcag gaactgtggc 360
   tgcaccatct gtcttcatct tcccgccatc tgatgagcag ttgaaatctg gaactgcctc 420
   tgttgtgtgc ctgctgaata acttctatcc cagagaggcc aaagtacagt ggaaggtgga 480
   taacgccctc caatcgggta actcccagga gagtgtcaca gagcaggaca gcaaggacag 540
   cacctacagc ctcagcagca ccctgacgct gagcaaagca gactacgaga aacacaaagt 600
   ctacgcctgc gaagtcaccc atcagggcct gagctcgccc gtcacaaaga gcttcaacag 660
   gggagagtgt tagagggcgg ccgc 684
<210> 108
   <211> 1215
   <212> DNA
   <213> Homo sapiens
<400> 108
   ggcctcgggg gcctcccagg ctctgggcag gcacaggcta ggtgccccta acccaggccc 60
   tgcacacaaa ggggcaggtg ctgggctcag acctgccaag agccatatcc gggaggaccc 120
   tgcccctgac ctaagcccac cccaaaggcc aaactctcca ctccctcagc tcggacacct 180
   tctctcctcc cagattccag taactcccaa tcttctctct gcagcttcca ccaagggccc 240
   atccgtcttc cccctggcgc cctgctccag gagcacctcc gagagcacag ccgccctggg 300
   ctgcctggtc aaggactact tccccgaacc ggtgacggtg tcgtggaact caggcgccct 360
   gaccagcggc gtgcacacct tcccggctgt cctacagtcc tcaggactct actccctcag 420
   cagcgtggtg accgtgccct ccagcagctt gggcacgaag acctacacct gcaacgtaga 480
   tcacaagccc agcaacacca aggtggacaa gagagttgag tccaaatatg gtcccccatg 540
   cccaccatgc ccagcacctg agttcctggg gggaccatca gtcttcctgt tccccccaaa 600
   acccaaggac actctcatga tctcccggac ccctgaggtc acgtgcgtgg tggtggacgt 660
   gagccaggaa gaccccgagg tccagttcaa ctggtacgtg gatggcgtgg aggtgcataa 720
   tgccaagaca aagccgcggg aggagcagtt caacagcacg taccgtgtgg tcagcgtcct 780
   caccgtcctg caccaggact ggctgaacgg caaggagtac aagtgcaagg tctccaacaa 840
   aggcctcccg tcctccatcg agaaaaccat ctccaaagcc aaagggcagc cccgagagcc 900
   acaggtgtac accctgcccc catcccagtg cgagatgacc aagaaccagg tcagcctgtc 960
   ctgcgcggtc aaaggcttct atcccagcga catcgccgtg gagtgggaga gcaatgggca 1020
   gccggagaac aactacaaga ccacgcctcc cgtgctggac tccgacggct ccttcttcct 1080
   cgtgagcagg ctaaccgtgg acaagagcag gtggcaggag gggaatgtct tctcatgctc 1140
   cgtgatgcat gaggctctgc acaaccacta cacacagaag agcctctccc tgtctctggg 1200
   taaatgagcg gccgc 1215
<210> 109
   <211> 15
   <212> DNA
   <213> Mus musculus
<400> 109
   acctactgga tgcac 15
<210> 110
   <211> 51
   <212> DNA
   <213> Mus musculus
<400> 110
   tacattaatc ctagcagtgg ttatactaag tacaatcaga agttcaaggt c 51
<210> 111
   <211> 27
   <212> DNA
   <213> Mus musculus
<400> 111
   ggtaacctcg gctacttctt tgactac 27
<210> 112
   <211> 15
   <212> DNA
   <213> Mus musculus
<400> 112
   gactactata tgcac 15
<210> 113
   <211> 51
   <212> DNA
   <213> Mus musculus
<400> 113
   tacattaatc ctagcagtgg ttatactaag tacaatcgga agttcaggga c 51
<210> 114
   <211> 27
   <212> DNA
   <213> Mus musculus
<400> 114
   gggggtaacg gttactacct tgactac 27
<210> 115
   <211> 106
   <212> PRT
   <213> Mus musculus
<400> 115
   Asp Ile Val Met Thr Gln Ser His Lys Phe Met Ser Thr Ser Val Gly
   1 5 10 15
   Asp Arg Val Ser Ile Thr Cys Lys Ala Ser Gln Asp Val Gly Thr Ala
   20 25 30
   Val Ala Trp Tyr Gln Gln Lys Pro Gly Leu Ser Pro Lys Leu Leu Ile
   35 40 45
   Tyr Trp Ala Ser Thr Arg His Thr Gly Val Pro Asp Arg Phe Thr Gly
   50 55 60
   Ser Gly Ser Gly Thr Asp Phe Thr Leu Thr Ile Ser Asn Val Gln Ser
   65 70 75 80
   Glu Asp Leu Ala Asp Tyr Phe Cys Gln Gln Tyr Ser Ser Tyr Leu Thr
   85 90 95
   Phe Gly Ala Gly Thr Lys Leu Glu Ile Lys
   100 105
<210> 116
   <211> 106
   <212> PRT
   <213> Mus musculus
<400> 116
   Asp Ile Gln Met Thr Gln Ser His Lys Phe Met Ser Thr Ser Val Gly
   1               5                   10                  15
   Asp Arg Val Ser Ile Thr Cys Lys Ala Ser Gln Asp Val Ser Thr Ala
               20                  25                  30
   Val Ala Trp Tyr Gln Gln Lys Pro Gly Gln Ser Pro Lys Leu Leu Ile
           35                  40                  45
   Tyr Trp Ala Ser Thr Arg His Thr Gly Val Pro Asp Arg Phe Thr Gly
       50                  55                  60
   Ser Gly Ser Gly Thr Asp Phe Thr Leu Thr Ile Ser Asn Val Gln Ser
   65                  70                  75                  80
   Glu Asp Leu Ala Asp Tyr Leu Cys Gln Gln Tyr Ser Asn Tyr Ile Thr
                   85                  90                  95
   Phe Gly Ala Gly Thr Lys Leu Glu Leu Lys
               100                 105
<210> 117
   <211> 33
   <212> DNA
   <213> Mus musculus
<400> 117
   aaggccagtc aggatgtggg tactgctgta gcc 33
<210> 118
   <211> 21
   <212> DNA
   <213> Mus musculus
<400> 118
   tgggcatcca cccggcacac t 21
<210> 119
   <211> 24
   <212> DNA
   <213> Mus musculus
<400> 119
   cagcaatata gcagctatct cacg 24
<210> 120
   <211> 33
   <212> DNA
   <213> Mus musculus
<400> 120
   aaggccagtc aggatgtgag tactgctgta gcc 33
<210> 121
   <211> 21
   <212> DNA
   <213> Mus musculus
<400> 121
   tgggcatcca cccggcacac t 21
<210> 122
   <211> 24
   <212> DNA
   <213> Mus musculus
<400> 122
   cagcaatata gcaactatat cacg 24
<210> 123
   <211> 57
   <212> DNA
   <213> Homo sapiens
<220>
   <221> CDS
   <222> (1).. (57)
<400> 123
   atg gac tgg acc tgg aga atc ctc ttt ttg gtg gca gca gcc aaa ggt 48
   Met Asp Trp Thr Trp Arg Ile Leu Phe Leu Val Ala Ala Ala Lys Gly
   1 5 10 15
   gcc cac tcc 57
   Ala His Ser
<210> 124
   <211> 19
   <212> PRT
   <213> Homo sapiens
<400> 124
   Met Asp Trp Thr Trp Arg Ile Leu Phe Leu Val Ala Ala Ala Lys Gly
   1 5 10 15
   Ala His Ser
<210> 125
   <211> 57
   <212> DNA
   <213> Homo sapiens
<220>
   <221> CDS
   <222> (1).. (57)
<400> 125
   atg gac tgg acc tgg agc atc ctt ttc ttg gtg gca gca gca aca ggt      48
   Met Asp Trp Thr Trp Ser Ile Leu Phe Leu Val Ala Ala Ala Thr Gly
   1               5                   10                  15
   gcc cac tcc                                                          57
   Ala His Ser
<210> 126
   <211> 19
   <212> PRT
   <213> Homo sapiens
<400> 126
   Met Asp Trp Thr Trp Ser Ile Leu Phe Leu Val Ala Ala Ala Thr Gly
   1               5                   10                  15
   Ala His Ser
<210> 127
   <211> 66
   <212> DNA
   <213> Homo sapiens
<220>
   <221> CDS
   <222> (1).. (66)
<400> 127
   atg gac atg agg gtc ccc gct cag ctc ctg ggg ctc ctg cta ctc tgg      48
   Met Asp Met Arg Val Pro Ala Gln Leu Leu Gly Leu Leu Leu Leu Trp
   1               5                   10                  15
   ctc cga ggt gcc aga tgt                                              66
   Leu Arg Gly Ala Arg Cys
               20
<210> 128
   <211> 22
   <212> PRT
   <213> Homo sapiens
<400> 128
   Met Asp Met Arg Val Pro Ala Gln Leu Leu Gly Leu Leu Leu Leu Trp
   1               5                   10                  15
   Leu Arg Gly Ala Arg Cys
               20
<210> 129
   <211> 354
   <212> DNA
   <213> Homo sapiens
<220>
   <221> CDS
   <222> (1)..(354)
<400> 129
   cag gtc cag ctt gtg cag tct ggg gct gag gtg aag aag cct ggg tcc      48
   Gln Val Gln Leu Val Gln Ser Gly Ala Glu Val Lys Lys Pro Gly Ser
   1               5                   10                  15
   tca gtg aag gtt tcc tgc aag gcc tct gga ggc acc ttc agt gac tac      96
   Ser Val Lys Val Ser Cys Lys Ala Ser Gly Gly Thr Phe Ser Asp Tyr
               20                  25                  30
   tat atg cac tgg gtg cgc cag gcc ccc gga caa ggg ctt gag tgg atg      144
   Tyr Met His Trp Val Arg Gln Ala Pro Gly Gln Gly Leu Glu Trp Met
           35                  40                  45
   gga tac att aat cct agc agt ggt tat act aag tac aat cgg aag ttc      192
   Gly Tyr Ile Asn Pro Ser Ser Gly Tyr Thr Lys Tyr Asn Arg Lys Phe
       50                  55                  60
   agg gac aga gtc acc att acc gcg gac aaa tcc acg agc aca gcc tac      240
   Arg Asp Arg Val Thr Ile Thr Ala Asp Lys Ser Thr Ser Thr Ala Tyr
   65                  70                  75                  80
   atg gag ctg agc agc ctg aga tct gaa gac acg gct gtg tat tac tgt      288
   Met Glu Leu Ser Ser Leu Arg Ser Glu Asp Thr Ala Val Tyr Tyr Cys
                   85                  90                  95
   gcg aga ggg ggt aac ggt tac tac ctt gac tac tgg ggc cag ggc acc      336
   Ala Arg Gly Gly Asn Gly Tyr Tyr Leu Asp Tyr Trp Gly Gln Gly Thr
               100                 105                 110
   acg gtc acc gtc tcc tca                                              354
   Thr Val Thr Val Ser Ser
           115
<210> 130
   <211> 118
   <212> PRT
   <213> Homo sapiens
<400> 130
   Gln Val Gln Leu Val Gln Ser Gly Ala Glu Val Lys Lys Pro Gly Ser
   1               5                   10                  15
   Ser Val Lys Val Ser Cys Lys Ala Ser Gly Gly Thr Phe Ser Asp Tyr
               20                  25                  30
   Tyr Met His Trp Val Arg Gln Ala Pro Gly Gln Gly Leu Glu Trp Met
           35                  40                  45
   Gly Tyr Ile Asn Pro Ser Ser Gly Tyr Thr Lys Tyr Asn Arg Lys Phe
       50                  55                  60
   Arg Asp Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Gly Gly Asn Gly Tyr Tyr Leu Asp Tyr Trp Gly Gln Gly Thr
               100                 105                 110
   Thr Val Thr Val Ser Ser
           115
<210> 131
   <211> 357
   <212> DNA
   <213> Homo sapiens
<220>
   <221> CDS
   <222> (1)..(357)
<400> 131
   cag gtg cag ctg gtg cag tct gga cct gac gtg aag aag ccg ggg gcc 48
   Gln Val Gln Leu Val Gln Ser Gly Pro Asp Val Lys Lys Pro Gly Ala
   1 5 10 15
   tca gtg aag gtc tcc tgc aag gcc tct ggc tac atg ttt tcc gac aac 96
   Ser Val Lys Val Ser Cys Lys Ala Ser Gly Tyr Met Phe Ser Asp Asn
   20 25 30
   aac atg gac tgg gcg cga cag gcc cct gga caa ggg ctt gag tgg atg 144
   Asn Met Asp Trp Ala Arg Gln Ala Pro Gly Gln Gly Leu Glu Trp Met
   35 40 45
   gga gat att aat act aaa agt ggt ggt tct atc tac aac cag aag ttc 192
   Gly Asp Ile Asn Thr Lys Ser Gly Gly Ser Ile Tyr Asn Gln Lys Phe
   50 55 60
   aag ggc aga gtc atc atg acc ata gac aaa tcc acg ggc aca gcc tac 240
   Lys Gly Arg Val Ile Met Thr Ile Asp Lys Ser Thr Gly Thr Ala Tyr
   65 70 75 80
   atg gaa ttg agg agc ctg aga tca gac gac acg gcc ata tat tac tgt 288
   Met Glu Leu Arg Ser Leu Arg Ser Asp Asp Thr Ala Ile Tyr Tyr Cys
   85 90 95
   gcg agg agg agg agc tac ggc tac tac ttt gac tac tgg ggc cag gga 336
   Ala Arg Arg Arg Ser Tyr Gly Tyr Tyr Phe Asp Tyr Trp Gly Gln Gly
   100 105 110
   acc ctg gtc acc gtc tcc tca 357
   Thr Leu Val Thr Val Ser Ser
   115
<210> 132
   <211> 119
   <212> PRT
   <213> Homo sapiens
<400> 132
   Gln Val Gln Leu Val Gln Ser Gly Pro Asp Val Lys Lys Pro Gly Ala
   1 5 10 15
   Ser Val Lys Val Ser Cys Lys Ala Ser Gly Tyr Met Phe Ser Asp Asn
   20 25 30
   Asn Met Asp Trp Ala Arg Gln Ala Pro Gly Gln Gly Leu Glu Trp Met
   35 40 45
   Gly Asp Ile Asn Thr Lys Ser Gly Gly Ser Ile Tyr Asn Gln Lys Phe
   50 55 60
   Lys Gly Arg Val Ile Met Thr Ile Asp Lys Ser Thr Gly Thr Ala Tyr
   65 70 75 80
   Met Glu Leu Arg Ser Leu Arg Ser Asp Asp Thr Ala Ile Tyr Tyr Cys
   85 90 95
   Ala Arg Arg Arg Ser Tyr Gly Tyr Tyr Phe Asp Tyr Trp Gly Gln Gly
   100 105 110
   Thr Leu Val Thr Val Ser Ser
   115
<210> 133
   <211> 318
   <212> DNA
   <213> Homo sapiens
<220>
   <221> CDS
   <222> (1).. (318)
<400> 133
   gac atc gtg atg acc cag tct cca tcc tcc ctg tct gca tct gta gga 48
   Asp Ile Val Met Thr Gln Ser Pro Ser Ser Leu Ser Ala Ser Val Gly
   1 5 10 15
   gac aga gtc acc atc act tgc aag gcc agt cag aat gtg ggg act gct 96
   Asp Arg Val Thr Ile Thr Cys Lys Ala Ser Gln Asn Val Gly Thr Ala
   20 25 30
   gta gcc tgg tat cag cag aaa cca ggg aaa gcc cct aag ctc ctg atc 144
   Val Ala Trp Tyr Gln Gln Lys Pro Gly Lys Ala Pro Lys Leu Leu Ile
   35 40 45
   tat tcg gca tcc tac cgg tac agt ggg gtc cca tca agg ttc agt ggc 192
   Tyr Ser Ala Ser Tyr Arg Tyr Ser Gly Val Pro Ser Arg Phe Ser Gly
   50 55 60
   agt cga tat ggg aca gat ttc act ctc acc atc tca agc ttg caa cct 240
   Ser Arg Tyr Gly Thr Asp Phe Thr Leu Thr Ile Ser Ser Leu Gln Pro
   65 70 75 80
   gaa gat tta gca act tac tac tgt cag caa tat agc aac tat atc acg 288
   Glu Asp Leu Ala Thr Tyr Tyr Cys Gln Gln Tyr Ser Asn Tyr Ile Thr
   85 90 95
   ttc ggc gga ggg acc aag gtg gag atc aaa 318
   Phe Gly Gly Gly Thr Lys Val Glu Ile Lys
   100 105
<210> 134
   <211> 106
   <212> PRT
   <213> Homo sapiens
<400> 134
   Asp Ile Val Met Thr Gln Ser Pro Ser Ser Leu Ser Ala Ser Val Gly
   1 5 10 15
   Asp Arg Val Thr Ile Thr Cys Lys Ala Ser Gln Asn Val Gly Thr Ala
   20 25 30
   Val Ala Trp Tyr Gln Gln Lys Pro Gly Lys Ala Pro Lys Leu Leu Ile
   35 40 45
   Tyr Ser Ala Ser Tyr Arg Tyr Ser Gly Val Pro Ser Arg Phe Ser Gly
   50 55 60
   Ser Arg Tyr Gly Thr Asp Phe Thr Leu Thr Ile Ser Ser Leu Gln Pro
   65                  70                  75                  80
   Glu Asp Leu Ala Thr Tyr Tyr Cys Gln Gln Tyr Ser Asn Tyr Ile Thr
                   85                  90                  95
   Phe Gly Gly Gly Thr Lys Val Glu Ile Lys
               100                 105
<210> 135
   <211> 354
   <212> DNA
   <213> Homo sapiens
<220>
   <221> CDS
   <222> (1)..(354)
<400> 135
   gag gtc cag ctt gtg cag tct ggg gct gag gtg gtg aag cct ggg tcc      48
   Glu Val Gln Leu Val Gln Ser Gly Ala Glu Val Val Lys Pro Gly Ser
   1               5                   10                  15
   tca gtg aag gtt tcc tgc acg gcc tct gga tac acc ttc agt gac tac      96
   Ser Val Lys Val Ser Cys Thr Ala Ser Gly Tyr Thr Phe Ser Asp Tyr
               20                  25                  30
   tat atg cac tgg gtg cgc cag gcc ccc gga gaa ggg ctt gag tgg atg      144
   Tyr Met His Trp Val Arg Gln Ala Pro Gly Glu Gly Leu Glu Trp Met
           35                  40                  45
   gga tac att aat cct agc agt ggt tat act aag tac aat cgg aag ttc      192
   Gly Tyr Ile Asn Pro Ser Ser Gly Tyr Thr Lys Tyr Asn Arg Lys Phe
       50                  55                  60
   agg gac aga gtc acc att acc gcg gac aaa tcc acg agc aca gcc tac      240
   Arg Asp Arg Val Thr Ile Thr Ala Asp Lys Ser Thr Ser Thr Ala Tyr
   65                  70                  75                  80
   atg gag ctg agc agc ctg aga tct gaa gac acg gct gtg tat tac tgt      288
   Met Glu Leu Ser Ser Leu Arg Ser Glu Asp Thr Ala Val Tyr Tyr Cys
   85 90 95
   gcg aga ggg ggt ctc ggt tac tac ctt gac tac tgg ggc gag ggc acc 336
   Ala Arg Gly Gly Leu Gly Tyr Tyr Leu Asp Tyr Trp Gly Glu Gly Thr
   100 105 110
   acg gtc acc gtc tcc tca 354
   Thr Val Thr Val Ser Ser
   115
<210> 136
   <211> 118
   <212> PRT
   <213> Homo sapiens
<400> 136
   Glu Val Gln Leu Val Gln Ser Gly Ala Glu Val Val Lys Pro Gly Ser
   1 5 10 15
   Ser Val Lys Val Ser Cys Thr Ala Ser Gly Tyr Thr Phe Ser Asp Tyr
   20 25 30
   Tyr Met His Trp Val Arg Gln Ala Pro Gly Glu Gly Leu Glu Trp Met
   35 40 45
   Gly Tyr Ile Asn Pro Ser Ser Gly Tyr Thr Lys Tyr Asn Arg Lys Phe
   50 55 60
   Arg Asp Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Gly Gly Leu Gly Tyr Tyr Leu Asp Tyr Trp Gly Glu Gly Thr
   100 105 110
   Thr Val Thr Val Ser Ser
   115
<210> 137
   <211> 357
   <212> DNA
   <213> Homo sapiens
<220>
   <221> CDS
   <222> (1)..(357)
<400> 137
   gag gtg cag ctg gtg cag tct gga gct cag gtg aag aag ccg ggg gcc      48
   Glu Val Gln Leu Val Gln Ser Gly Ala Gln Val Lys Lys Pro Gly Ala
   1               5                   10                  15
   tca gtg aag gtc tcc tgc aag gcc tct ggc tac acg ttt tcc gac aac      96
   Ser Val Lys Val Ser Cys Lys Ala Ser Gly Tyr Thr Phe Ser Asp Asn
               20                  25                  30
   aac atg gac tgg gtg cga cag gcc cct gga aaa ggg ctt gag tgg atg      144
   Asn Met Asp Trp Val Arg Gln Ala Pro Gly Lys Gly Leu Glu Trp Met
           35                  40                  45
   gga gat att aat act aaa agt ggt ggt tct atc tac aac cag aag ttc      192
   Gly Asp Ile Asn Thr Lys Ser Gly Gly Ser Ile Tyr Asn Gln Lys Phe
       50                  55                  60
   aag ggc aga gtc atc atg acc ata gac aaa tcc acg ggc aca gcc tac      240
   Lys Gly Arg Val Ile Met Thr Ile Asp Lys Ser Thr Gly Thr Ala Tyr
   65                  70                  75                  80
   atg gaa ttg agg agc ctg aga tca gac gac acg gcc ata tat tac tgt      288
   Met Glu Leu Arg Ser Leu Arg Ser Asp Asp Thr Ala Ile Tyr Tyr Cys
                   85                  90                  95
   gcg agg agg agg agc tac ggc tac tac ttt gac tac tgg ggc cgg gga      336
   Ala Arg Arg Arg Ser Tyr Gly Tyr Tyr Phe Asp Tyr Trp Gly Arg Gly
               100                 105                 110
   acc ctg gtc acc gtc tcc tca                                          357
   Thr Leu Val Thr Val Ser Ser
           115
<210> 138
   <211> 119
   <212> PRT
   <213> Homo sapiens
<400> 138
   Glu Val Gln Leu Val Gln Ser Gly Ala Gln Val Lys Lys Pro Gly Ala
   1               5                   10                  15
   Ser Val Lys Val Ser Cys Lys Ala Ser Gly Tyr Thr Phe Ser Asp Asn
               20                  25                  30
   Asn Met Asp Trp Val Arg Gln Ala Pro Gly Lys Gly Leu Glu Trp Met
           35                  40                  45
   Gly Asp Ile Asn Thr Lys Ser Gly Gly Ser Ile Tyr Asn Gln Lys Phe
       50                  55                  60
   Lys Gly Arg Val Ile Met Thr Ile Asp Lys Ser Thr Gly Thr Ala Tyr
   65                  70                  75                  80
   Met Glu Leu Arg Ser Leu Arg Ser Asp Asp Thr Ala Ile Tyr Tyr Cys
                   85                  90                  95
   Ala Arg Arg Arg Ser Tyr Gly Tyr Tyr Phe Asp Tyr Trp Gly Arg Gly
               100                 105                 110
   Thr Leu Val Thr Val Ser Ser
           115
<210> 139
   <211> 318
   <212> DNA
   <213> Homo sapiens
<220>
   <221> CDS
   <222> (1).. (318)
<400> 139
   gac atc gtg atg acc cag tct cca tcc tcc ctg tct gca tct gta gga 48
   Asp Ile Val Met Thr Gln Ser Pro Ser Ser Leu Ser Ala Ser Val Gly
   1 5 10 15
   gac aga gtc acc atc act tgc aag gcc agt cag aat gtg ggg act gct 96
   Asp Arg Val Thr Ile Thr Cys Lys Ala Ser Gln Asn Val Gly Thr Ala
   20 25 30
   gta gcc tgg tat cag cag aaa cca ggg aaa gcc cct aag ctc ctg atc 144
   Val Ala Trp Tyr Gln Gln Lys Pro Gly Lys Ala Pro Lys Leu Leu Ile
   35 40 45
   tat tcg gca tcc tac cgg tac agt ggg gtc cca tca agg ttc agt ggc 192
   Tyr Ser Ala Ser Tyr Arg Tyr Ser Gly Val Pro Ser Arg Phe Ser Gly
   50 55 60
   agt cga tat ggg aca gat ttc act ctc acc atc tca agc ttg caa cct 240
   Ser Arg Tyr Gly Thr Asp Phe Thr Leu Thr Ile Ser Ser Leu Gln Pro
   65 70 75 80
   gaa gat tta gca act tac tac tgt cag caa tat agc aac tat atc acg 288
   Glu Asp Leu Ala Thr Tyr Tyr Cys Gln Gln Tyr Ser Asn Tyr Ile Thr
   85 90 95
   ttc ggc caa ggg acc aag gtg gag atc aaa 318
   Phe Gly Gln Gly Thr Lys Val Glu Ile Lys
   100 105
<210> 140
   <211> 106
   <212> PRT
   <213> Homo sapiens
<400> 140
   Asp Ile Val Met Thr Gln Ser Pro Ser Ser Leu Ser Ala Ser Val Gly
   1 5 10 15
   Asp Arg Val Thr Ile Thr Cys Lys Ala Ser Gln Asn Val Gly Thr Ala
   20 25 30
   Val Ala Trp Tyr Gln Gln Lys Pro Gly Lys Ala Pro Lys Leu Leu Ile
   35 40 45
   Tyr Ser Ala Ser Tyr Arg Tyr Ser Gly Val Pro Ser Arg Phe Ser Gly
   50 55 60
   Ser Arg Tyr Gly Thr Asp Phe Thr Leu Thr Ile Ser Ser Leu Gln Pro
   65 70 75 80
   Glu Asp Leu Ala Thr Tyr Tyr Cys Gln Gln Tyr Ser Asn Tyr Ile Thr
   85 90 95
   Phe Gly Gln Gly Thr Lys Val Glu Ile Lys
   100 105
<210> 141
   <211> 354
   <212> DNA
   <213> Homo sapiens
<220>
   <221> CDS
   <222> (1)..(354)
<400> 141
   gag gtc cag ctt gtg cag tct ggg gct gag gtg cag aag cct ggg gcc 48
   Glu Val Gln Leu Val Gln Ser Gly Ala Glu Val Gln Lys Pro Gly Ala
   1 5 10 15
   tca gtg aag gtt tcc tgc aag gcc tct gga tac acc ttc act gac tac 96
   Ser Val Lys Val Ser Cys Lys Ala Ser Gly Tyr Thr Phe Thr Asp Tyr
   20 25 30
   tat atg cac tgg gtg cgc cag gcc ccc gga gaa ggg ctt gag tgg atg 144
   Tyr Met His Trp Val Arg Gln Ala Pro Gly Glu Gly Leu Glu Trp Met
   35 40 45
   gga tac att aat cct agc agt ggt tat act aag tac aat cgg aag ttc 192
   Gly Tyr Ile Asn Pro Ser Ser Gly Tyr Thr Lys Tyr Asn Arg Lys Phe
   50 55 60
   agg gac aga gtc acc att acc gcg gac aaa tcc acg agc aca gcc tac 240
   Arg Asp Arg Val Thr Ile Thr Ala Asp Lys Ser Thr Ser Thr Ala Tyr
   65 70 75 80
   atg gag ctg agc agc ctg aga tct gaa gac acg gct gtg tat tac tgt 288
   Met Glu Leu Ser Ser Leu Arg Ser Glu Asp Thr Ala Val Tyr Tyr Cys
   85 90 95
   gcg aga ggg ggt caa ggt tac tac ctt gac tac tgg ggc gag ggc acc 336
   Ala Arg Gly Gly Gln Gly Tyr Tyr Leu Asp Tyr Trp Gly Glu Gly Thr
   100 105 110
   acg gtc acc gtc tcc tca 354
   Thr Val Thr Val Ser Ser
   115
<210> 142
   <211> 118
   <212> PRT
   <213> Homo sapiens
<400> 142
   Glu Val Gln Leu Val Gln Ser Gly Ala Glu Val Gln Lys Pro Gly Ala
   1 5 10 15
   Ser Val Lys Val Ser Cys Lys Ala Ser Gly Tyr Thr Phe Thr Asp Tyr
   20 25 30
   Tyr Met His Trp Val Arg Gln Ala Pro Gly Glu Gly Leu Glu Trp Met
   35 40 45
   Gly Tyr Ile Asn Pro Ser Ser Gly Tyr Thr Lys Tyr Asn Arg Lys Phe
   50 55 60
   Arg Asp Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Gly Gly Gln Gly Tyr Tyr Leu Asp Tyr Trp Gly Glu Gly Thr
   100 105 110
   Thr Val Thr Val Ser Ser
   115
<210> 143
   <211> 318
   <212> DNA
   <213> Homo sapiens
<220>
   <221> CDS
   <222> (1)..(318)
<400> 143
   gac atc gtg atg acc cag tct cca tcc tcc ctg tct gca tct gta gga 48
   Asp Ile Val Met Thr Gln Ser Pro Ser Ser Leu Ser Ala Ser Val Gly
   1 5 10 15
   gac aga gtc acc atc act tgc aag gcc agt cag aat gtg ggg act gct 96
   Asp Arg Val Thr Ile Thr Cys Lys Ala Ser Gln Asn Val Gly Thr Ala
   20 25 30
   gta gcc tgg tat cag cag aaa cca ggg aaa gcc cct aag ctc ctg atc 144
   Val Ala Trp Tyr Gln Gln Lys Pro Gly Lys Ala Pro Lys Leu Leu Ile
   35 40 45
   tat tcg gca tcc tac cgg gcc agt ggg gtc cca tca agg ttc agt ggc 192
   Tyr Ser Ala Ser Tyr Arg Ala Ser Gly Val Pro Ser Arg Phe Ser Gly
   50 55 60
   agt cga tat ggg aca gat ttc act ctc acc atc tca agc ttg caa cct 240
   Ser Arg Tyr Gly Thr Asp Phe Thr Leu Thr Ile Ser Ser Leu Gln Pro
   65 70 75 80
   gaa gat tta gca act tac tac tgt cag caa tat agc aac tat atc acg 288
   Glu Asp Leu Ala Thr Tyr Tyr Cys Gln Gln Tyr Ser Asn Tyr Ile Thr
   85 90 95
   ttc ggc caa ggg acc aag gtg gag atc aaa 318
   Phe Gly Gln Gly Thr Lys Val Glu Ile Lys
   100 105
<210> 144
   <211> 106
   <212> PRT
   <213> Homo sapiens
<400> 144
   Asp Ile Val Met Thr Gln Ser Pro Ser Ser Leu Ser Ala Ser Val Gly
   1 5 10 15
   Asp Arg Val Thr Ile Thr Cys Lys Ala Ser Gln Asn Val Gly Thr Ala
   20 25 30
   Val Ala Trp Tyr Gln Gln Lys Pro Gly Lys Ala Pro Lys Leu Leu Ile
   35 40 45
   Tyr Ser Ala Ser Tyr Arg Ala Ser Gly Val Pro Ser Arg Phe Ser Gly
   50 55 60
   Ser Arg Tyr Gly Thr Asp Phe Thr Leu Thr Ile Ser Ser Leu Gln Pro
   65 70 75 80
   Glu Asp Leu Ala Thr Tyr Tyr Cys Gln Gln Tyr Ser Asn Tyr Ile Thr
   85 90 95
   Phe Gly Gln Gly Thr Lys Val Glu Ile Lys
   100 105

## Description

### Technical Field

The present invention relates to a method for producing a bispecific antibody comprising a first H chain, a second H chain, and commonly shared L chains, wherein the method comprises the steps of:
(1) preparing a first antibody against a first antigen, and a second antibody against a second antigen;
(2) producing a bispecific antibody against the first antigen and the second antigen, which comprises variable regions of the first antibody and the second antibody;
(3) measuring the antigen binding activity or the biological activity of the bispecific antibody produced in step (2);
(4) producing a commonly shared L chain antibody by linking the H chain of the first antibody and the H chain of the second antibody with the L chain of the first antibody or the second antibody;
(5) measuring the antigen binding activity or biological activity of the commonly shared L chain antibody produced in step (4);
(6) substituting one, two, or three CDRs of the commonly shared L chains produced in step (4) with the L chain CDRs of the first antibody, or the second antibody;
(7) selecting the antibody having a desired activity by comparing the antigen binding activity or the biological activity of the antibody obtained in step (6) with that of the original bispecific antibody produced in step (2) or the antibody produced in step (4); and
(8) obtaining the antibody which has an activity equivalent to or higher than that of the original bispecific antibody produced in step (2), by repeating steps (6) and (7) as necessary for the antibody selected in step (7).

Further disclosed are multispecific antibodies that functionally substitute for coagulation factor VIII, a cofactor that enhances enzymatic reactions, methods for producing such antibodies, and pharmaceutical compositions comprising such an antibody as an active ingredient.

### Background Art

Antibodies are highly stable in blood and have low antigenicity; therefore, they have attracted much attention as pharmaceuticals. Bispecific antibodies, i.e., antibodies that can recognize two types of antigens simultaneously, are among such antibodies. Bispecific antibodies have been proposed for some time. However, to date, the only bispecific antibodies reported in the literature are those in which two types of antigen-binding sites are merely linked together, such as those aimed for retargeting NK cells, macrophages, and T cells (Non-patent Document 3). For example, MDX-210, an antibody currently undergoing clinical investigation, is a bispecific antibody which merely retargets FcγRI-expressing monocytes and such against HER-2/neu-expressing cancer cells. Accordingly, until now, there were no examples of bispecific antibodies utilized as functional substitutes for cofactors that enhance enzyme reactions.

A cofactor is a helper molecule needed by an enzyme to be functional, and a protein or non-protein component that binds to an enzyme and is required for its catalytic activity. Examples of protein cofactors include, but are not limited to, coagulation factor VIII (F. VIII), activated coagulation factor VIII (F. VIIIa), coagulation factor V (F. V), activated coagulation factor V (F. Va), tissue factor (TF), thrombomodulin (TM), protein S (PS), protein Z (PZ), heparin, complement C4b, complement regulatory factor H, membrane cofactor protein (MCP), and complement receptor 1 (CR1).

Among these, F. VIII/F. VIIIa is a cofactor required for sufficient expression of activity of activated coagulation factor IX (F. IXa). Using chromogenic assays, Scheiflinger F, *et al.* discovered that a certain type of anti-F. IX/F. IXa antibody can enhance activation of coagulation factor X (F. X) by F. IXa (Patent Document 1). However, coagulation recovery measurements in F. VIII deficient plasma showed that coagulation recovery was not observed when this antibody alone was added; rather, coagulation recovery was observed only when F. IXa was exogenously added.

F. VIIIa is known to interact not only with F. IXa but also with F. X (Non-patent Documents 1 and 2). In this regard, the antibody of Scheiflinger F. *et al.* did not sufficiently substitute functionally for F. VIII/F. VIIIa, and its activity is also estimated to be insufficient [Patent Document 1] WO 01/19992
[Non-patent Document 1] Mertens K et al., Thromb. Haemost., 1999, Vol. 82, p.209-217
[Non-patent Document 2] Lapan KA et al., Thromb. Haemost., 1998, Vol. 80, p.418-422
[Non-patent Document 3] Segal DM et al., Journal of Immunological Methods, 2001, Vol. 248, p.1-6

Methods for making multispecific antibodies having heteromultimeric heavy chain components and common light chain components such as bispecific antibodies are described in US 2002/062010. These methods involve the screening of libraries of a first and second antibody for commonly shared L chains and L chains that present in both libraries are chosen for producing bispecific antibodies.

### Content of the Disclosure

Multispecific antibodies that functionally substitute for coagulation factor VIII, a cofactor that enhances enzymatic reactions.

Various bispecific antibodies that bind specifically to both F. IX/F. IXa and F. X, and functionally substitute for F. VIIIa, more specifically, have cofactor functions to enhance F. X activation by F. IXa are disclosed herein.

Of these antibodies, one antibody (A44/B26) that reduced the coagulation time by 50 seconds or more as compared to that observed when no antibody was added to a coagulation time measuring system using F. VIII-deficient human serum. could be selected. This antibody was used produce a commonly shared L chain antibody by linking its H chains with the A44 L chains. It was shown that that the commonly shared L chain antibody can be produced with A44L; however, the activity of this antibody was attenuated as compared to the activity of the original bispecific antibody (A44HL-B26HL).

In addition, the CDRs derived from the A44 L chain and the B26 L chain were combined with the framework (Fr) derived from the A44 L chain to produce hybrid L chains, and these L chains were used to produce commonly shared antibodies aiming at the recovery of F. VIII activity. As a result, when the combination of CDR1, 2, and 3 was BBA(G) (CDR1, 2, and 3 were CDR derived from the B26 L chain, CDR derived from the B26 L chain, and CDR derived from the A44 L chain, respectively), F. VIII activity was significantly increased as compared to the activity observed with A44/B26. In addition, the coagulation time was reduced by 70 seconds or more as compared to that observed when no antibody was added. This antibody did not attenuate the functions of F. VIII (0.1, 1 U/mL), and, in fact, acted additively. Furthermore, when CDR1, 2, and 3 were ABA(G) or BBA(G), their coagulation times were reduced by 60 seconds or more as compared to that observed when no antibody was added.

When the H chains of antibodies A50 and A69, which are highly homologous to A44, were combined with B26H and the above-mentioned hybrid L chains, and their activities were evaluated, antibodies that have activities higher than those with A44H were obtained. Furthermore, when hybrid L chains combining the CDRs of A44L, B26L, A50L, and A69L were produced and their activities were examined, highly active antibodies were obtained; however, none exceeded the activity of the A44/B26-derived hybrid L chain (BBA(G)).

When various hybrid L chains (BBA, aAA, AAa, ABa, BBa, aBA, BAA, BAa, and ABA)) were combined with A69H and B26H and their activities were evaluated, highly active antibodies were obtained, and, particularly in the case of the BBA or BBa combination, coagulation time was reduced by 80 seconds or more as compared to that observed no antibody was added.

When humanization of these antibodies was further examined, activity equal to that of the original antibodies was accomplished by combining (1) humanized A69H, (2) humanized B26H, and (3) humanized hybrid L chains.

Thus, as described above, it was possible to produce highly active multispecific antibodies that functionally substitute for coagulation factor VIII.

As already stated above, the present invention provides methods for recovering or increasing the activities of these antibodies, which decreased due to the commonly shared L chains of each antibody. The present disclosure relates to multispecific antibodies that functionally substitute for coagulation factor VIII, a cofactor that enhances enzymatic reactions, methods for producing such antibodies, and methods for recovering or increasing their activities that decreased due to the commonly shared L chains of each antibody.

The present invention provides:
A method for producing a bispecific antibody comprising a first H chain, a second H chain, and commonly shared L chains, wherein the method comprises the steps of:
   (1) preparing a first antibody against a first antigen, and a second antibody against a second antigen;
   (2) producing a bispecific antibody against the first antigen and the second antigen, which comprises variable regions of the first antibody and the second antibody;
   (3) measuring the antigen binding activity or the biological activity of the bispecific antibody produced in step (2);
   (4) producing a commonly shared L chain antibody by linking the H chain of the first antibody and the H chain of the second antibody with the L chain of the first antibody or the second antibody;
   (5) measuring the antigen binding activity or biological activity of the commonly shared L chain antibody produced in step (4);
   (6) substituting one, two, or three CDRs of the commonly shared L chains produced in step (4) with the CDRs of the first antibody, or the second antibody;
   (7) selecting the antibody having a desired activity by comparing the antigen binding activity or the biological activity of the antibody obtained in step (6) with that of the original bispecific antibody produced in step (2) or the antibody produced in step (4); and
   (8) obtaining the antibody which has an activity equivalent to or higher than that of the original bispecific antibody produced in step (2), by repeating steps (6) and (7) as necessary for the antibody selected in step (7).

Steps (6) and (7) can be repeated two or more times.

### Brief Description of the Drawings

Fig. 1 is a diagram showing an insertion region of pcDNA4-g4H.
Fig. 2 is a diagram showing an insertion region of pcDNA4-g4L and pIND-g4L.
Fig. 3 is a diagram showing an insertion region of pIND-g4H.
Fig. 4 shows the results of the F. VIIIa-like activity measurement of anti-F. IXa/anti-F. X bispecific antibodies, which were prepared using anti-F. IXa antibody XB12 and anti-F. X antibodies SB04, SB21, SB42, SB38, SB30, SB07, SB05, SB06, and SB34. The concentrations of the antibody solutions are 10 µg/mL (1 µg/mL final concentration). As a result, F. VIIIa-like activity increased in 9 kinds of bispecific antibodies, listed hereafter in the order of increasing activity: XB12/SB04, XB12/SB21, XB12/SB42, XB12/SB38, XB12/SB30, XB12/SB07, XB12/SB05, XB12/SB06, and XB12/SB34.
Fig. 5 shows the results of the F. VIIIa-like activity measurement of anti-F. IXa antibody XT04 and anti-F. IXa/anti-F. X bispecific antibodies prepared using XT04 and anti-F. X antibodies SB04, SB21, SB42, SB38, SB30, SB07, SB05, SB05, and SB34. The concentrations of the antibody solutions are 10 µg/mL (1 µg/mL final concentration). As a result, XT04/SB04, XT04/SB21, XT04/SB42, XT04/SB38, XT04/SB30, XT04/SB07, XT04/SB05, XT04/SB06, and XT04/SB34 showed an increase in F. VIIIa-like activity.
Fig. 6 shows the results of the F.VIIIa-like activity measurement on XB12/SB04, the antibody that exhibited the highest activity in the assay of Fig. 4, in various concentrations. As a result, XB12/SB04 showed a concentration-dependent increase in F. VIIIa-like activity.
Fig. 7 shows the results of the coagulation time measurement observed in the presence of XB12/SB04, XB12/SB21, XB12/SB42, XB12/SB38, XB12/SB30, XB12/SB07, XB12/SB05, XB12/SB06, or XB12/SB34. After antibody solution and F. VIII deficient plasma were mixed, the antibody concentration is 1.7 µg/mL for XB12/SB06 and 10 µg/mL for the rest. As a result, XB12/SB04, XB12/SB21, XB12/SB42, XB12/SB38, XB12/SB30, XB12/SB07, XB12/SB05, XB12/SB06, and XB12/SB34 showed a coagulation time-reducing effect as compared to that observed in the absence of the antibody.
Fig. 8 shows the results of the coagulation time measurement in the presence of XT04/SB04, XT04/SB21, XT04/SB42, XT04/SB38, XT04/SB30, XT04/SB07, XT04/SB05, XT04/SB06, or XT04/SB34. After antibody solution and F. VIII deficient plasma were mixed, the antibody concentration is 5 µg/mL for XT04/SB06 and 10 µg/mL for the rest. As a result, XT04/SB04, XT04/SB21, XT04/SB42, XT04/SB38, XT04/SB30, XT04/SB07, XT04/SB05, and XT04/SB06 showed a coagulation time-reducing effect as compared to that observed in the absence of the antibody. A reduction in coagulation time was not observed for XT04/SB34.
Fig. 9 shows the results of the coagulation time measurement on XB12/SB04, the antibody that demonstrated the greatest coagulation time-reducing effect in the assays of Figs. 7 and 8, in various concentrations. As a result, XB12/SB04 showed a concentration-dependent reduction in coagulation time. The antibody concentrations in Fig. 9 represent the values after mixing the antibody solutions and F. VIII deficient plasma.
Fig. 10 shows the results of GST-AP Western blotting of SB04 or SB06. Photographs 1, 2, and 3 represent the results of reacting the transferred GST-AP with SB04, SB06, and the sample without an antibody, respectively. As the result, only the binding reaction of SB04 with GST-AP was detected.
Fig. 11 is a diagram of pELBGlacI. ColE1 ori, ColE1 series plasmid replication origin region; flori, f1 phage replication origin region; lacI, lactose repressor protein-coding region; P_{lac}, lactose promoter; pelBss, *E.coli* Pe1B protein signal sequence; scFv, single strand antibody-coding region; gene III: f1 phage GeneIII protein-coding region; Amp^{r}, ampicillin resistant gene; and Sfi I, restriction enzyme Sfi I cleavage site.
Fig. 12 shows F. VIIIa-like activity measurements obtained using the culture supernatants of the bispecific antibodies, which were expressed by combining anti-F. IXa antibodies (A19, A25, A31, A38, A39, A40, A41, A44, A50, A69, and XB12) and anti-F. X antibodies (B2, B5, B9, B10, B11, B12, B13, B14, B15, B16, B18, B19, B20, B21, B23, B25, B26, B27, B31, B34-1, B34-2, B35, B36, B38, B42, SB04, SB15, and SB27). The symbol + represents the case where the F. VIIIa-like activity is 0.1 or more.

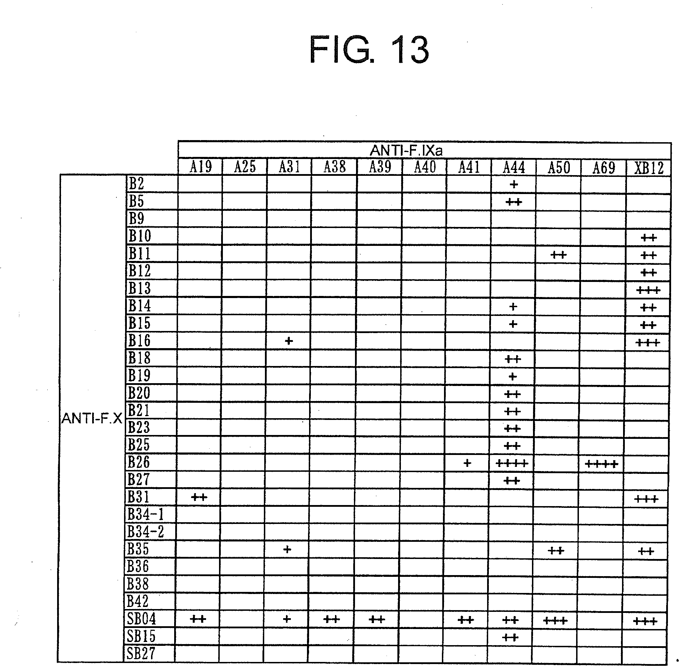

Fig. 13 shows the results of a plasma coagulation assay using the purified bispecific antibodies, which were expressed by combining anti-F. IXa antibodies (A19, A25, A31, A38, A39, A40, A41, A44, A50, A69, and XB12) and anti-F. X antibodies (B2, B5, B9, B10, B11, B12, B13, B14, B15, B16, B18, B19, B20, B21, B23, B25, B26, B27, B31, B34-1, B34-2, B35, B36, B38, B42, SB04, SB15, and SB27). The reductions of the coagulation time, which range from 10 seconds to 20 seconds, from 20 seconds to 40 seconds, from 40 seconds to 50 seconds, or is 50 seconds or more as compared to that observed in the absence of antibody, are represented by the symbol +, ++, +++, and ++++, respectively.
Fig. 14 shows the coagulation time measurements observed using A44/B26, an antibody that demonstrated great coagulation time-reducing effect in the assay of Fig. 13, at various concentrations. The coagulation time observed in the absence of antibody was 113 seconds. Addition of A44/B26 showed a concentration-dependent reduction in coagulation time. The antibody concentrations in Fig. 14 represent the values after mixing the antibody solutions and F. VIII deficient plasma.
Fig. 15 shows the coagulation time measurements observed using A69/B26, an antibody that demonstrated a great coagulation time-reducing effect in the assay of Fig. 13, at various concentrations. The coagulation time observed in the absence of antibody was 109.6 seconds. Addition of A69/B26 showed a concentration-dependent reduction in coagulation time. The antibody concentrations in Fig. 15 represent the values mixing the antibody solutions and F. VIII deficient plasma.
Fig. 16 shows the coagulation time measurements observed in the coexistence of A44/B26 or XB12/SB04 and F. VIII. As a result, the mixture solution of A44/B26 or XB12/SB04 and F. VIII showed a coagulation time-reducing effect as compared to that observed when F. VIII was singly used.
Fig. 17 shows the coagulation time measurements observed in an inhibitory plasma in the presence of A44/B26 or XB12/SB04. As a result, both A44/B26 and XB12/SB04 showed a coagulation time-reducing effect as compared to that observed no antibody was added.
Fig. 18 shows the coagulation time measurements observed using XB12/SB04 and humanized XB12/humanized SB04 at various concentrations. The coagulation time observed when no antibody was added was 111.3 seconds. As a result, humanized XB12/humanized SB04 showed a coagulation time-reducing effect comparable to that of XB12/SB04. The antibody concentrations in Fig. 18 represent the values after mixing the antibody solutions and F. VIII deficient plasma.
Fig. 19 shows the structure of L chain expression vector, pCAGG-κ.
Fig. 20 shows the coagulation time measurements observed using the bispecific antibody produced by combining A44, B26, and AAA. After mixing with the antibody solution and F. VIII deficient plasma, the antibody concentration was 30 µg/mL.

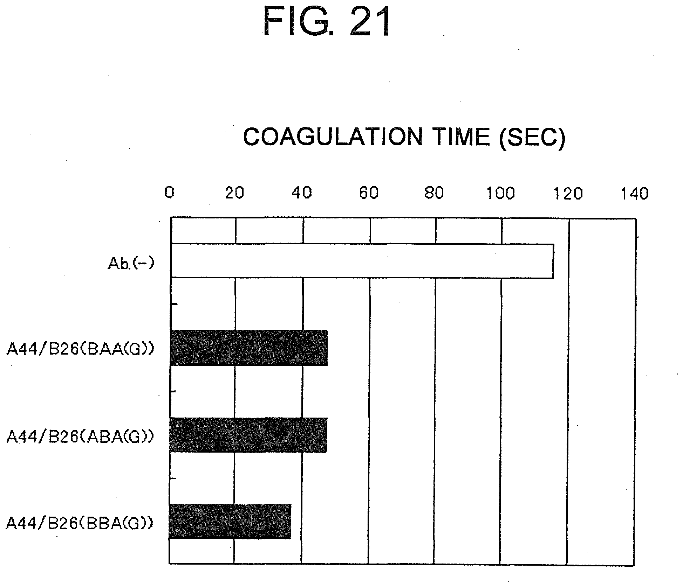

Fig. 21 shows the coagulation time measurements observed using the bispecific antibodies produced by combining A44/B26 and BAA (G), ABA (G) or BBA (G). After mixing the antibody solutions and F. VIII deficient plasma, the antibody concentrations were 30 µg/mL.

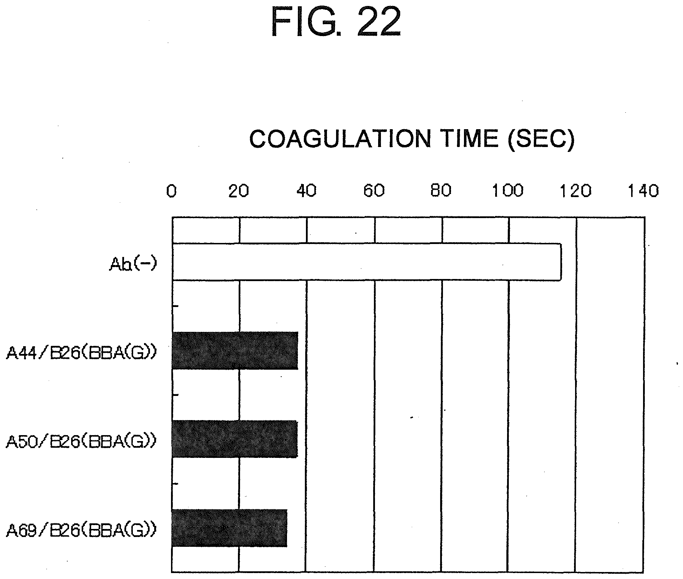

Fig. 22 shows the coagulation time measurements observed using the bispecific antibodies produced by combining B26/AAA and A50 or A69. After mixing the antibody solutions and F. VIII deficient plasma, the antibody concentrations were 30 µg/mL.

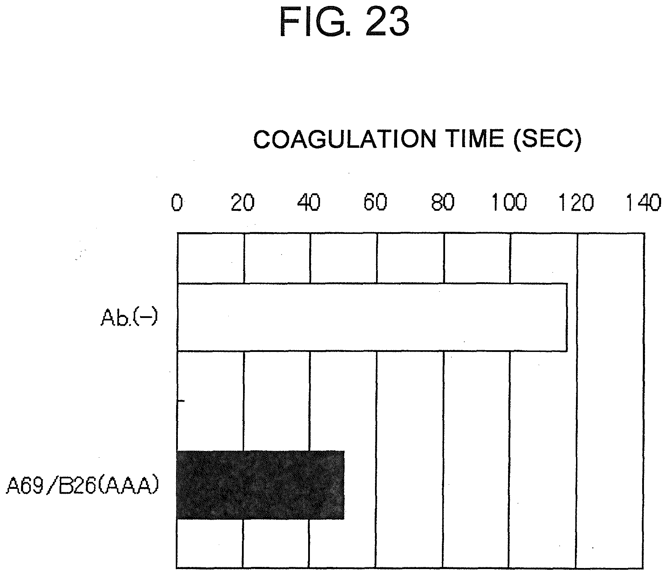

Fig. 23 shows the coagulation time measurements observed using the bispecific antibody produced by combining A69, B26, and AAA. After mixing the antibody solution and F. VIII deficient plasma, the antibody concentration was 30 µg/mL.

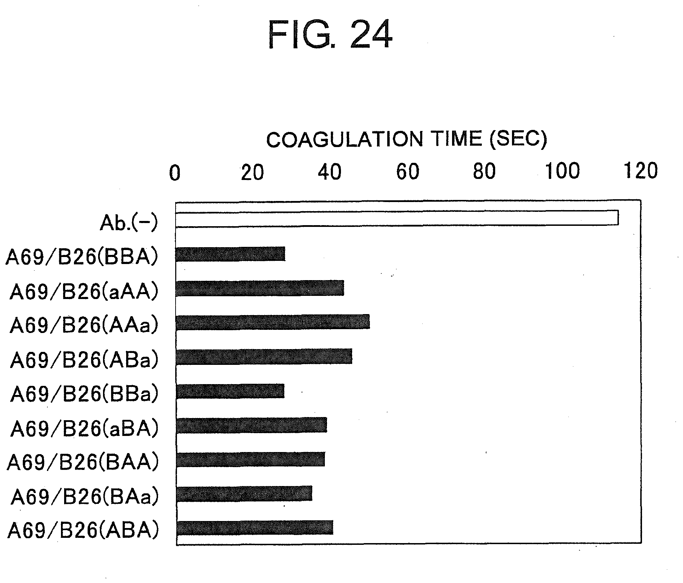

Fig. 24 shows the coagulation time measurements observed using the bispecific antibodies produced by combining A69/B26 and BBA, aAA, AAa, ABa, BBa, aBA, BAA, BAa or ABA. After mixing the antibody solutions and F. VIII deficient plasma, the antibody concentrations were 30 µg/mL.

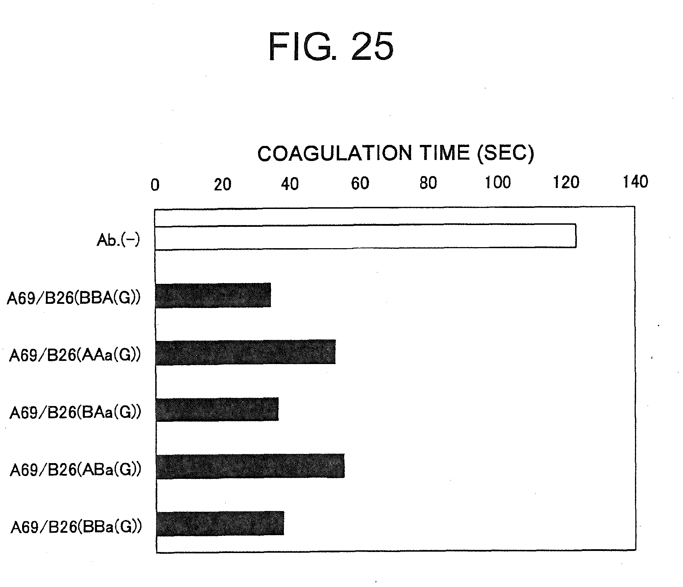

Fig. 25 shows the coagulation time measurements observed using the bispecific antibodies produced by combining A69/B26 and BBA(G), AAa(G), BAa(G), ABa(G) or BBa(G). After mixing the antibody solutions and F. VIII deficient plasma, the antibody concentrations were 30 µg/mL.
Fig. 26 shows the coagulation time measurements observed using the bispecific antibodies produced by combining A69/B26 and aAA(G) or aBA(G). After mixing the antibody solution and F. VIII deficient plasma, the antibody concentrations were 30 µg/mL.
Fig. 27 shows the coagulation time measurements observed using a chimeric bispecific antibody and humanized bispecific antibodies. The "knobs-into-holes" technique was used on the constant regions of each antibody. After mixing the antibody solution and F. VIII deficient plasma, the antibody concentrations were 30 µg/mL.
Fig. 28 shows the coagulation time measurements observed using two types of humanized bispecific antibodies. Wild-type constant regions were used for each antibody. After mixing the antibody solution and F. VIII deficient plasma, the antibody concentrations were 30 µg/mL.

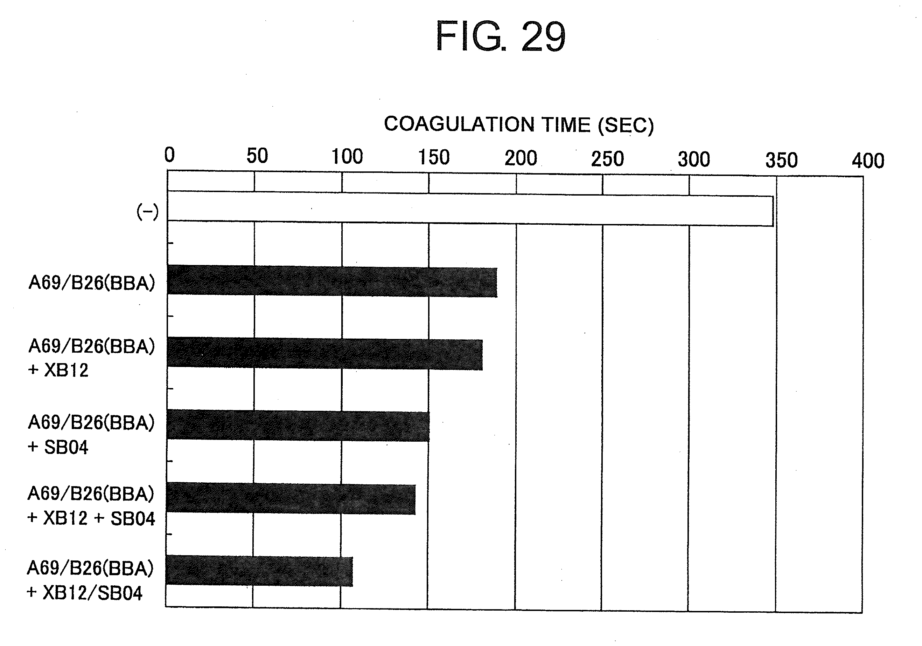

Fig. 29 shows the coagulation time measurements observed when mixing A69/B26/BBA with XB12, SB04, XB12 and SB04, and SB12/SB04, respectively. The concentration of each antibody after mixing was 20 µg/mL.

### Definitions

As described herein, the term "multispecific antibody" refers to an antibody that can specifically bind to at least two different antigens. Examples of preferred multispecific antibodies include, but are not limited to, bispecific antibodies (BsAbs) (also called dual specific antibodies) that can specifically bind to two antigens.

As described herein, the term "different antigen(s)" does not necessarily mean that the antigen molecules themselves are different; it may simply mean that their antigenic determinants are different. Therefore, for example, different antigenic determinants within a single molecule are also included in the different antigens of the present invention, and two antibodies that recognize such different antigenic determinants within a single molecule, respectively, are regarded in the present invention as antibodies that recognize different antigens. Furthermore, in the present invention, the term "commonly shared light (L) chain" refers to a light chain that can link with two or more different heavy chains, and show binding ability to each antigen. Herein, the term "different heavy (H) chain(s)" preferably refers to heavy chains of antibodies against different antigens, but is not limited thereto, and also refers to heavy chains whose amino acid sequences are different from each other.

The multispecific antibodies of the present disclosure (preferably bispecific antibodies) are antibodies having specificity to two or more different antigens, or molecules comprising fragments of such antibodies. These antibodies are not particularly limited, but are preferably monoclonal antibodies.

Bispecific antibodies of the present invention comprise commonly shared light (L) chains.

Bispecific antibodies of the present invention are preferably recombinant antibodies produced using genetic recombination techniques. (See, for example, Borrebaeck CAK and Larrick JW, THERAPEUTIC MONOCLONAL ANTIBODIES, Published in the United Kingdom by MACMILLAN PUBLISHERS LTD, 1990.) Recombinant antibodies can be obtained by cloning DNAs encoding antibodies from hybridomas or antibody-producing cells, such as sensitized lymphocytes, that produce antibodies, inserting them into suitable vectors, and then introducing them into hosts to produce the antibodies.

These antibodies may be antibody fragments or modified antibodies. Antibody fragments include diabodies (Dbs), linear antibodies, and single chain antibodies (hereinafter, also denoted as scFvs). Herein, an "Fv" fragment is defined as the smallest antibody fragment that comprises a complete antigen recognition site and binding site. An "Fv" fragment is a dimer (VH-VL dimer) in which a heavy (H) chain variable region (VH) and a light (L) chain variable region (VL) are strongly linked by non-covalent binding. The three complementarity determining regions (CDRs) of each of the variable regions interact with each other to form an antigen-binding site on the surface of the VH-VL dimer. Six CDRs confer the antigen-binding site to an antibody. However, one variable region (or half of the Fv comprising only three CDRs specific to an antigen) alone can recognize and bind to an antigen, though its affinity is lower than that of the entire binding site.

An Fab fragment (also called F(ab)) further comprises an L chain constant region and an H chain constant region (CHI). An Fab' fragment differs from an Fab fragment in that it additionally comprises several residues derived from the carboxyl terminus of the H chain CH1 region, comprising one or more cysteines from the hinge region of the antibody. Fab'-SH refers to an Fab' in which one or more cysteine residues of its constant region comprise a free thiol group. An F(ab') fragment is produced by cleavage of disulfide bonds between the cysteine residues in the hinge region of F(ab')₂ pepsin digest. Other chemically bound antibody fragments are also known to those skilled in the art.

Diabodies are bivalent antibody fragments constructed by gene fusion (Holliger, P. et al., Proc. Natl. Acad. Sci. USA 90: 6444-6448 (1993); EP 404,097; WO 93/11161). Diabodies are dimers consisting of two polypeptide chains, in which each polypeptide chain comprises an L chain variable region (VL) and an H chain variable region (VH) linked with a linker short enough to prevent association of these two domains within the same chain, for example, a linker of about 5 amino acids. The VL and VH regions encoded on the same polypeptide chain form a dimer since the linker between the VL and VH is too short to form a single chain variable region fragment. Therefore, diabodies comprise two antigen-binding sites.

A single-chain antibody or an scFv antibody fragment comprises the VH and VL regions of an antibody, and these regions exist in a single polypeptide chain. In general, an Fv polypeptide further comprises a polypeptide linker between the VH and VL regions, and this enables an scFv to form a structure necessary for antigen binding (for a review on scFvs, see Pluckthun "The Pharmacology of Monoclonal Antibodies" Vol. 113 (Rosenburg and Moore ed. (Springer Verlag, New York) pp.269-315, 1994). In the context of the present disclosure, linkers are not particularly limited so long as they do not inhibit the expression of the antibody variable regions linked at their ends.

IgG-type bispecific antibodies can be secreted from hybrid hybridomas (quadromas) produced by fusing two kinds of hybridomas that produce IgG antibodies (Milstein C et al. Nature 1983, 305: 537-540). They can also be secreted by taking the L chain and H chain genes constituting the two kinds of IgGs of interest, a total of 4 kinds of genes, and introducing them into cells to coexpress the genes.

In this case, by introducing suitable amino acid substitutions to the CH3 regions of the H chains, IgGs having a heterogeneous combination of H chains can be preferentially secreted (Ridgway JB et al. Protein Engineering 1996, 9: 617-621; Merchant AM et al. Nature Biotechnology 1998, 16: 677-681).

Regarding the L chains, since diversity of L chain variable regions is lower than that of H chain variable regions, commonly shared L chains that can confer binding ability to both H chains may be obtained. The antibodies of the present invention comprise commonly shared L chains. Bispecific IgGs can be efficiently expressed by introducing the genes of the commonly shared L chain and both H chains into cells.

Bispecific antibodies may be produced by chemically crosslinking Fab's. Bispecific F(ab')₂ can be produced, for example, by preparing Fab' from an antibody, using it to produce a maleimidized Fab' with ortho-phenylenedi-maleimide (o-PDM), and then reacting this with Fab' prepared from another antibody to crosslink Fab's derived from different antibodies (Keler T et al. Cancer Research 1997, 57: 4008-4014). The method of chemically linking a Fab'-thionitrobenzoic acid (TNB) derivative and an antibody fragment such as Fab'-thiol (SH) is also known (Brennan M et al. Science 1985, 229: 81-83).

Instead of a chemical crosslink, a leucine zipper derived from Fos and Jun may also be used. Preferential formation of heterodimers by Fos and Jun is utilized, even though they also form homodimers. Fab' to which Fos leucine zipper is added, and another Fab' to which Jun leucine zipper is added are expressed and prepared. Monomeric Fab'-Fos and Fab'-Jun reduced under mild conditions are mixed and reacted to form bispecific F(ab')₂ (Kostelny SA et al. J. of Immunology, 1992, 148: 1547-53). This method can be applied not only to Fab's but also to scFvs, Fvs, and such.

A bispecific antibody may also be produced using a diabody. A bispecific diabody is a heterodimer of two cross-over scFv fragments. More specifically, it is produced by forming a heterodimer using VH(A)-VL(B) and VH(B)-VL(A) prepared by linking VHs and VLs derived from two kinds of antibodies, A and B, using a relatively short linker of about 5 residues (Holliger P et al. Proc Natl. Acad. Sci. USA 1993, 90: 6444-6448).

The desired structure can be promoted by linking the two scFvs with a flexible and relatively long linker comprising about 15 residues (single chain diabody: Kipriyanov SM et al. J. of Molecular Biology. 1999, 293: 41-56), and conducting appropriate amino acid substitutions (knobs-into-holes: Zhu Z et al. Protein Science. 1997, 6: 781-788).

An sc(Fv)₂ that can be produced by linking two types of scFvs with a flexible and relatively long linker, comprising about 15 residues, may also be a bispecific antibody (Mallender WD et al. J. of Biological Chemistry, 1994, 269: 199-206).

Examples of modified antibodies include, but are not limited to, antibodies linked to various molecules such as polyethylene glycol (PEG). In the context of the present disclosure, the substance to which the modified antibodies are linked is not limited. Such modified antibodies can be obtained by chemically modifying obtained antibodies. Such methods are well established in the art.

The antibodies of the present disclosure are preferably derived from human, mouse, rat, or such, but are not limited thereto. They may also be genetically modified antibodies, such as chimeric or humanized antibodies.

Methods for obtaining human antibodies are known in the art. For example, transgenic animals carrying the entire repertoire of human antibody genes can be immunized with desired antigens to obtain desired human antibodies (see International Patent Application WO 93/12227, WO 92/03918, WO 94/02602, WO 94/25585, WO 96/34096, and WO 96/33735).

Genetically modified antibodies can also be produced using known methods. Specifically, for example, chimeric antibodies may comprise H chain and L chain variable regions of an immunized animal antibody, and H chain and L chain constant regions of a human antibody. Chimeric antibodies can be obtained by linking DNAs encoding the variable regions of the antibody derived from the immunized animal, with DNAs encoding the constant regions of a human antibody, inserting this into an expression vector, and then introducing it into host cells to produce the antibodies.

Humanized antibodies are modified antibodies often referred to as "reshaped" human antibodies. A humanized antibody is constructed by transferring the CDRs of an antibody derived from an immunized animal to the complementarity determining regions of a human antibody. Conventional genetic recombination techniques for such purposes are known.

Specifically, a DNA sequence designed so that the CDRs of a mouse antibody and the framework regions (FRs) of a human antibody are linked may be synthesized by PCR from several oligonucleotides prepared to comprise overlapping regions at their ends. The obtained DNA may then be linked with a DNA encoding human antibody constant region, inserted into an expression vector, and introduced into a host to obtain a humanized antibody (see European

Patent Application No. 239400, and International Patent Application WO 96/02576). The human antibody FRs linked through CDRs are selected so that the complementarity determining regions form suitable antigen-binding sites. As necessary, the amino acids of the framework regions in the antibody variable regions may be substituted so that the complementarity determining regions of the reshaped human antibody form appropriate antigen-binding sites (Sato K et al., Cancer Research 1993, 53: 851-856). Substitutions may be introduced into framework regions derived from various human antibodies (see International Patent Application WO 99/51743).

The bispecific antibodies of the present invention may recognize coagulation factor IX (F. IX) and/or activated coagulation factor IX (F. IXa) of coagulation and fibrinolysis-related factors, and coagulation factor X (F. X); have activities that functionally substitute for cofactor F. VIII/F. Villa; and comprise commonly shared L chains. The antibodies of the present invention may ordinarily have a structure comprising anti-F. IXa antibody variable regions and anti-F. X antibody variable regions.

A multispecific antibody of the present disclosure may be an antibody comprising a first domain recognizing coagulation factor IX and/or activated coagulation factor IX and a second domain recognizing coagulation factor X, in which the first and second domains further comprise a third polypeptide comprising the whole or partial sequence of a commonly shared L chain.

An antibody of the present invention may be a bispecific antibody that can functionally substitute for coagulation factor VIII, which comprises a first domain recognizing coagulation factor IX and/or activated coagulation factor IX, and a second domain recognizing coagulation factor X; in which the first domain comprises a first polypeptide comprising the whole or partial H chain of an antibody against coagulation factor IX or activated coagulation factor IX, the second domain comprises a second polypeptide comprising the whole or partial H chain of an antibody against coagulation factor X, and the first and second domains further comprise a third polypeptide comprising a common sequence of the whole or partial L chain.

Activated coagulation factor VIII (F. VIIIa) enhances F. X activation by F. IXa by binding to both F. IXa and F. X. Among the above-described bispecific antibodies that recognize both the enzyme F. IXa and substrate F. X, some of them have the activity to enhance F. X activation. Of such antibodies, some of them may have the activity to functionally substitute for cofactor F. VIII/F. VIIIa.

The F. VIII/F. VIIIa disclosed herein is subject to limited proteolysis by proteases, such as thrombin; however, so long as the cofactor activity of F. VIII/F. VIIIa is present, its form does not matter. Mutant F. VIII/V.VIIIa and F. VIII/F. VIIIa artificially modified by genetic recombination techniques are also comprised in the F. VIII/F. VIIIa of the present invention, so long as they have the cofactor activity of F. VIII/F. VIIIa.

A "third polypeptide" disclosed herein is preferably a polypeptide that comprises a whole or partial sequence of the L chain of an antibody against coagulation factor IX (F. IX), activated coagulation factor IX (F. IXa), or coagulation factor X (F. X).

In addition, a "third polypeptide" disclosed herein preferably comprises an antigen-binding site comprising CDR1, 2, and 3 each independently selected from CDR1, 2, and 3 of each of the L chains of two or more antibodies or antigen-binding site functionally equivalent thereto.

In the context of present disclosure, the H chain CDR1, 2, and 3 of the first polypeptide of an antibody as disclosed herein may constitute specifically, for example, an antigen-binding site comprising amino acid sequences of each sequence of the H chain CDR1, 2, and 3 (SEQ ID NOs: 3, 5, and 7; or 21, 5, and 22) of A44 or A69 described in the following Examples, or an antigen-binding site functionally equivalent thereto.

In the context of present disclosure, the H chain CDR1, 2, and 3 of the second polypeptide may constitute specifically, for example, an antigen-binding site comprising amino acid sequences of each sequence of the H chain CDR1, 2, and 3 (SEQ ID NOs: 26, 28, and 30) of B26 described in the following Examples, or an antigen-binding site functionally equivalent thereto.

The amino acid sequences of the H chain variable regions of A44, A50, A69, and B26 as disclosed herein are described in the following SEQ ID NOs, respectively.
A44: SEQ ID NO: 1
A50: SEQ ID NO: 15
A69: SEQ ID NO: 20
B26: SEQ ID NO: 24

The nucleotide sequences of the H chain CDRs of A44, A50, A69, and B26 are described in the following SEQ ID NOs, in order of CDRs 1, 2, and 3 (each of SEQ ID NOs in parentheses indicates the amino acid sequence encoded by the nucleotide sequence).
A44: SEQ ID NOs: 2 (3), 4 (5), and 6 (7)
A50: SEQ ID NOs: 109 (16), 110 (17), and 111 (18)
A69: SEQ ID NOs: 112 (21), 113 (5), and 114 (22)
B26: SEQ ID NOs: 25 (26), 27 (28), and 29 (30)

The amino acid sequences of the L chain variable regions of A44, A50, A69, and B26 of the present disclosure are described in the following SEQ ID NOs, respectively.
A44: SEQ ID NO: 8
A50: SEQ ID NO: 115
A69: SEQ ID NO: 116
B26: SEQ ID NO: 31

The nucleotide sequences of the L chain CDRs of A44, A50, A69, and B26 are described in the following SEQ ID NOs, in order of CDR 1, 2, and 3 (each of SEQ ID NOs in parentheses indicates the amino acid sequence encoded by the nucleotide sequence).
A44: SEQ ID NOs: 9 (10), 11 (12), and 13 (14)
A50: SEQ ID NOs: 117 (10), 118 (12), and 119 (19)
A69: SEQ ID NOs: 120 (23), 121 (12), and 122 (14)
B26: SEQ ID NOs: 32 (33), 34 (35), and 36 (37)

The amino acid sequences of CDR1 are shown as follows..
A44: SEQ ID NOs: 3 and 10
A50: SEQ ID NOs: 16 and 10
A69: SEQ ID NOs: 21 and 23
B26: SEQ ID NOs: 26 and 33

The amino acid sequences of CDR2 are shown as follows.
A44: SEQ ID NOs: 5 and 12
A50: SEQ ID NOs: 17 and 12
A69: SEQ ID NOs: 5 and 12
B26: SEQ ID NOs: 28 and 35

The amino acid sequences of CDR3 are shown as follows.
A44: SEQ ID NOs: 7 and 14
A50: SEQ ID NOs: 18 and 19
A69: SEQ ID NOs: 22 and 14
B26: SEQ ID NOs: 30 and 37

When producing a full-length antibody using the variable regions disclosed herein, without particular limitations, constant regions well known to those skilled in the art may be used. For example, constant regions described in "Sequences of proteins of immunological interest", (1991), U.S. Department of Health and Human Services. Public Health Service National Institutes of Health, or "An efficient route to human bispecific IgG", (1998). Nature Biotechnology vol. 16, 677-681 can be used.

The bispecific antibodies disclosed herein were evaluated for their activity to substitute for F. VIII/F. VIIIa (a cofactor for F. X activation by F. IXa) using a measurement system comprising F. XIa (F. IX activating enzyme), F. IX, F. X, synthetic substrate of F. Xa (S-2222), and phospholipids. These results were used to select, in principle, the bispecific antibodies indicating F. VIIIa-like activity of 0.1 or more as those having activity to substitute for F. VIII/F. VIIIa. The "F. VIIIa-like activity" mentioned herein is a value obtained by subtracting the change in absorbance of the solvent or culture supernatant without antibody expression for 30 minutes or 60 minutes, from the change in the absorbance of the antibody solution or culture supernatant containing expressed antibodies for 30 minutes or 60 minutes.

The ability of the bispecific antibodies selected above, or related bispecific antibodies, to recover coagulation was measured in a coagulation time measurement system using F. VIII-deficient human plasma. As a result, bispecific antibodies that reduce the coagulation time as compared to that observed when no antibodies were added were obtained. The coagulation time mentioned herein refers to the measured activated partial thromboplastin time (APTT) using F. VIII-deficient human plasma, as described in Example 7. Using these bispecific antibodies, reduction of the coagulation time was preferably 10 seconds or more, more preferably 20 seconds or more, even more preferably 40 seconds or more, or most preferably 50 seconds or more.

More specifically, the multispecific antibodies disclosed herein can functionally substitute for coagulation factor VIII, which recognizes coagulation factor IX and/or activated coagulation factor IX and coagulation factor X.

The substitutive function of F.VIII by these multispecific antibodies can be demonstrated by measuring the reduction of coagulation time as compared to that observed when no antibody is added in a coagulation time-measurement system using F. VIII-deficient human plasma. The coagulation time mentioned herein refers to, for example, activated partial thromboplastin time (APTT) in a coagulation time-measurement system using F. VIII-deficient human plasma, as described in Example 21. Preferably, the coagulation time is reduced by 50 seconds or more, preferably 60 seconds or more, more preferably 70 seconds or more, and even more preferably 80 seconds or more.

The multispecific antibodies disclosed herein preferably comprise H chain CDRs of an anti-coagulation factor IX/IXa antibody and CDRs functionally equivalent thereto, and H chain CDRs of an anti-coagulation factor X antibody or CDRs functionally equivalent thereto.

They preferably comprise an antigen-binding site comprising the amino acid sequences of H chain CDR1, 2, and 3 of SEQ ID NOs: 3, 5, and 7 (H chain CDRs of A44), or the amino acid sequences of H chain CDR1, 2, and 3 of SEQ ID NOs: 21, 5, and 22 (H chain CDRs of A69) of an anti-coagulation factor IX/IXa antibody, or an antigen-binding site functionally equivalent thereto, and an antigen-binding site comprising the amino acid sequences of H chain CDR1, 2, and 3 of SEQ ID NOs: 26, 28, and 30 (H chain CDRs of B26) of an anti-coagulation factor X antibody, or an antigen-binding site functionally equivalent thereto.

In the context of present disclosure: A "functionally equivalent" antigen-binding site has binding properties similar to those of an antigen-binding site comprising the various CDRs described herein. More specifically, if the following amino acid substitutions for stabilization allow recognition of a similar antigenic determinant (epitope), resulting antigen-binding sites incorporating such substitutions are "functionally equivalent".

Amino acid substitutions can be performed on the antibodies (clones) of the present disclosure to avoid deamidation, methionine oxidation, and such, or to structurally stabilize the antibodies, as described below.

Amino acid residues of the antibodies of the present disclosure can be modified as necessary to avoid deamidation, methionine oxidation, and such, or to structurally stabilize the antibodies.

N and M residues may be modified for deamidation, methionine oxidation, and so on. The G residue of the NG sequence in the H chain CDR3 of A44 and A69, and the T residue of the NT sequence in the H chain CDR2 of B26 may also be modified. In addition, M residues may be modified to avoid methionine oxidation. Furthermore, the D residue of the RD sequence at the end of the H chain CDR2 of A44 and A69, and the V residue of the KV sequence of the A50 H chain CDR2 may be modified to increase thermostability, by improving the turn structure, and thus modification to a G, S, or T residue is particularly preferred. Similarly, the Y residue of the A44 L chain CDR3, kabat 95, can be modified to a P residue. Furthermore, to increase thermostability, by improving the hydrophobic core, the V residue of the B26 L chain CDR1, kabat 33, can be modified to an L residue. In addition, to correct disturbance of the VH/VL interfaces, the L residue of the LDY sequence or the F residue of FDY sequence at the end of the H chain CDR3 of A44, A50, and A69 can be modified. Similarly, the I residue of the IT sequence or the L residue of the LT sequence at the end of the L chain CDR3 of A44, A50, and A69 can be modified. The Y residue of the RYS sequence of the B26 L chain CDR2 may also be modified.

Sequences of each of the CDRs of A44, A50, A69, and B26 are shown below; the amino acid residues that may be substituted are underlined.

| | |
|---|---|
| A44 H chain CDR1: | SSWMH (SEQ ID NO: 3) |
| A50 H chain CDR1: | TYWMH (SEQ ID NO: 16) |
| A69 H chain CDR1: | DYYMH (SEQ ID NO: 21) |
| B26 H chain CDR1: | DNNMD (SEQ ID NO: 26) |
| A44, A69 H chain CDR2: | YINPSSGYTKYNRKFRD (SEQ ID NO: 5) |
| A50 H chain CDR2: | YINPSSGYTKYNQKFKV (SEQ ID NO: 17) |
| B26 H chain CDR2: | DINTKSGGSIYNQKFKG (SEQ ID NO: 28) |
| A44 H chain CDR3: | GGNGYYFDY (SEQ ID NO: 7) |
| A50 H chain CDR3: | GNLGYFFDY (SEQ ID NO: 18) |
| A69 H chain CDR3: | GGNGYYLDY (SEQ ID NO: 22) |
| B26 H chain CDR3: | RRSYGYYFDY (SEQ ID NO: 30) |
| A44, A50 L chain CDR1: | KASQDVGTAVA (SEQ ID NO: 10) |
| A69 L chain CDR1: | KASQDVSTAVA (SEQ ID NO: 23) |
| B26 L chain CDR1: | KASQNVGTAVA (SEQ ID NO: 33) |
| A44, A50, A69 L chain CDR2: | WASTRHT (SEQ ID NO: 12) |
| B26 L chain CDR2: | SASYRYS (SEQ ID NO: 35) |
| A44, A69 L chain CDR3: | QQYSNYIT (SEQ ID NO: 14) |
| A50 L chain CDR3: | QQYSSYLT (SEQ ID NO: 19) |
| B26 L chain CDR3: | QQYNSYPLT (SEQ ID NO: 37) |

In the method of the present invention, first, bispecific antibodies whose L chains are not commonly shared in each antibody are produced.

In the present invention, without particular limitation, the bispecific antibodies can be obtained by any method. For example, to obtain functionally substituting bispecific antibodies of a cofactor against enzyme A and substrate B, animals are separately immunized with enzyme A and substrate B so as to obtain anti-enzyme A antibodies and anti-substrate B antibodies. Subsequently, bispecific antibodies comprising the H and L chains from the anti-enzyme A antibody and the H and L chains of the anti-substrate B antibody are produced. Preferably, several types of both anti-enzyme A antibodies and anti-substrate B antibodies are obtained, and preferably, these are used to produce bispecific antibodies derived from as many combinations as possible. After producing the bispecific antibodies, those having an activity to functionally substitute for the cofactor are selected.

Antibodies against enzymes or substrates can be obtained by methods well known to those skilled in the art. For example, they can be prepared by immunizing animals with antigens. Antigens used to immunize the animals include complete antigens that have immunogenicity, and incomplete antigens (including haptens) having no immunogenicity. In the context of the present invention, enzymes or substrates, on which the functionally substituting antibodies of cofactors of the present invention are considered to act, are used as the antigens (immunogen). Examples of the animals that can be immunized include, but are not limited to, mice, rats, hamsters, guinea pigs, rabbits, chickens, or rhesus monkeys. Immunizing these animals with the antigens can be performed by methods well known to those skilled in the art. In the present invention, the antibody L chain and H chain variable regions are preferably collected from the immunized animals or cells of such animals. This process can be carried out using techniques generally known to those skilled in the art. The animals immunized by the antigens express antibodies against those antigens, especially in spleen cells. Therefore, for example, the L chain and H chain variable regions can be collected by preparing mRNAs from spleen cells of immunized animals, and then performing RT-PCR using primers corresponding to the variable regions of the antibodies.

More specifically, the enzymes and substrates may be used individually to immunize the animals. Enzymes and substrates used as immunogens may be whole proteins, or partial peptides of such proteins. An immunogen used to immunize animals may be prepared as a soluble antigen by linking a moiety that serves as an antigen to another molecule, or a fragment thereof, depending on the situation.

Spleen cells may be isolated from the spleen of immunized mice and fused with mouse myeloma cells to produce hybridomas. Hybridomas that bind to antigens are then individually selected, and the L chain and H chain variable regions can be collected by RT-PCR using primers that correspond to the variable regions. Primers corresponding to the CDRs, primers corresponding to the frameworks which are less diverse than CDRs, or primers corresponding to the signal sequence and CH1 or the L chain constant regions (CLs) may be used.

Alternatively, mRNAs may be extracted from spleen cells of immunized animals and the cDNAs of the L chain and H chain variable regions may be collected by RT-PCR using primers corresponding to sites near the variable regions. Lymphocytes may also be immunized *in vitro* and used to construct a library displaying scFvs or Fabs. Antigen-binding antibody clones can be concentrated and cloned by panning to obtain the variable regions. In this case, screening can be performed using a similar library produced from mRNAs derived from peripheral blood monocytes, spleens, tonsils, or such of humans or non-immunized animals.

Using the obtained variable regions, antibody expression vectors are produced. A bispecific antibody can be obtained by introducing an anti-enzyme antibody expression vector and an anti-substrate antibody expression vector into the same cells to express the antibody.

Next, in the above-mentioned method of the present invention, antigen binding activities or biological activities of the produced bispecific antibodies are measured. For example, antibodies having an activity to functionally substitute for a cofactor can be selected by methods such those described below.
(1) Selecting antibodies using a reaction system comprising the enzyme and the substrate, and using as an indicator, the increase of the enzyme activity (substrate degradation) due to addition of the antibody.
(2) Selecting antibodies using a system that measures or mimics biological functions in which the enzyme, substrate, and cofactor are involved, and using as an indicator, the activity of functional recovery brought about by adding the antibody in the absence of the cofactor.

More specifically, "activity" can be measured by measuring the coagulation ability of test antibodies, for example, in a coagulation time measurement system using coagulation factor-deficient human plasma.

The obtained antibodies can be purified to homogeneity. Separation and purification of the antibodies can be performed using conventional separation and purification methods used for ordinary proteins. For example, the antibodies can be separated and purified by appropriately selecting and combining column chromatography such as affinity chromatography, filtration, ultrafiltration, salt precipitation, dialysis, SDS polyacrylamide gel electrophoresis, isoelectric focusing, and such, without limitation (Antibodies : A Laboratory Manual. Ed Harlow and David Lane, Cold Spring Harbor Laboratory, 1988). Columns used for affinity chromatography include, for example, protein A columns and protein G columns.

The cofactor to be substituted is F. VIII/F. VIIIa, and, more specifically, the combination of enzyme and substrate is a coagulation/fibrinolysis-related factor, F. IXa and F. X. A specific antibody may be produced having a structure comprising the variable region of an anti-F. IXa antibody and the variable region of an anti-F. X antibody.

More specifically, for example, a functionally substituting bispecific antibody of F. VIII/F. VIIIa can be produced by the following method.

Mice are immunized by subcutaneously injecting commercially available F. IXa and F. X, individually. Spleen cells are isolated from the spleens of immunized mice showing increased antibody titer and fused with mouse myeloma cells to produce hybridomas. Hybridomas that bind to the antigens (F. IXa and F. X) are separately selected, and the L chain and H chain variable regions are collected by RT-PCR using primers corresponding to the variable regions. The L chain variable regions are inserted into L chain expression vectors comprising the L chain constant region, and the H chain variable regions are inserted into H chain expression vectors comprising the H chain constant region, respectively. mRNAs are extracted from the spleens of these immunized mice, and the cDNAs of the L chain and H chain variable regions are collected by RT-PCR using primers corresponding to the variable regions. A phage library displaying scFvs is then constructed using these variable regions. Next, antigen-binding antibody clones are concentrated and cloned by panning and their variable regions are used to produce antibody expression vectors. An anti-F. IXa antibody (H chain and L chain) expression vector and an anti-F. X antibody (H chain and L chain) expression vector are then introduced into the same cells so as to express the antibodies and obtain bispecific antibodies.

The bispecific antibodies comprising commonly shared L chains, which are produced by the above-mentioned methods of the present invention, are also described herein. One aspect of the present disclosure relates to antibodies which have the activity to functionally substitute for cofactor F. VIII; therefore, these antibodies can be exptected to become effective pharmaceutical agents for diseases caused by decreased activity (function) of this cofactor. Examples of the above-mentioned diseases include, but are not limited to, bleeding, diseases accompanying bleeding, and diseases caused by bleeding. For example, reduction or deficiency in F. VIII/F. VIIIa function causes a bleeding disorder called hemophilia.

Among hemophilias, a bleeding disorder arising from a congenital reduction or deficiency in F. VIII/F. VIIIa function is called hemophilia A. Bleeding in a hemophilia A patient is treated by replacement therapy using an F. VIII preparation. When hard exercise or excursion causes frequent intraarticular bleeding, or when a patient has severe hemophilia, preventive administration of an F. VIII preparation may be conducted (Nilsson IM et al., J. Intern. Med., 1992, Vol. 235, p.25-32; Lδfqvist T et al., J. Intern. Med., 1997, Vol. 241, p.395-400). Since this preventive administration of an F. VIII preparation dramatically decreases bleeding episodes in patients with hemophilia A, such practice has become widespread in recent years. The decrease in bleeding episodes not only reduces the dangers of lethal and nonlethal bleeding and distress accompanying such bleeding, but also prevents hemophilic arthropathy caused by frequent intraarticular bleeding. As a result, the quality of life of hemophilia A patients may be greatly improved.

The half-life of an F. VIII preparation in blood is short, approximately 12 to 16 hours. Therefore, for continuous prevention, the F. VIII preparation must be administered approximately three times a week. This dosage maintains F. VIII activity at approximately 1% or more (The 24th Academic Meeting of the Japanese Society on Thrombosis and Hemostasis, Academic Special Committee, Committee for discussing on standardization of hemophilia, mini symposium, 2001). In replacement therapy at the time of bleeding, unless the bleeding is mild, additional administration of the F. VIII preparation should also be conducted regularly for a certain period to prevent rebleeding and achieve complete hemostasis.

An F. VIII preparation is typically administered intravenously. However, there are technical difficulties associated with intravenous administration. In particular, administration to young patients is still more difficult because the target veins are generally quite narrow.

Often times, home treatment and self injection are performed for the preventive administration of F. VIII preparation, and for emergency administration when bleeding. The need for frequent administration and technical difficulties of administration not only causes patient distress, but also leads patients to opt out of home therapy and self injection. Therefore, there is a strong demand for pharmaceutical agents that can be administered at longer intervals or more easily as compared to the currently available coagulation factor VIII preparations.

In hemophilia A patients, particularly severe hemophilia A patients, antibodies against F. VIII called inhibitors may appear. When such inhibitors are produced, the effect of the F. VIII preparation is disturbed by the inhibitors. As a result, hemostasis management in patients becomes very difficult

When bleeding occurs in such hemophilia A inhibitor patients, ordinarily, neutralization treatment using large amounts of an F. VIII preparation, or bypass treatment using a complex concentrate or an F. VIIa preparation is carried out However, in the neutralization method, administration of a large amount of the F. VIII preparation can instead increase the titer of the inhibitor (anti-F. VIII antibody). Bypass treatment also has drawbacks, namely the short half life (approximately 2 to 8 hours) of the complex concentrate or F. VIIa preparation in blood. Since their action mechanisms are independent of F. VIII/F. VIIIa function, that is, the function to catalyze F. X activation by F. IXa, in some cases, the hemostasis mechanism cannot function well and thus no response is yielded. As a result, sufficient hemostatic effect is much more difficult to obtain in hemophilia A inhibitor patients than in non-inhibitor hemophilia A patients. Therefore, there is a strong need in the art for a pharmaceutical agent that is not influenced by the presence of the inhibitor, and also that functionally substitutes for F. VIII/ F. VIIIa.

Besides hemophilia and acquired hemophilia involving anti-F. VIII auto-antibodies, another known bleeding disorder relating to F. VIII/F. VIIIa is vonWillebrand's disease caused by functional abnormality or deficiency of vonWillebrand factor (vWF). vWF is necessary not only for platelets to adhere normally to the subendothelial tissues at an injured site of a vascular wall, but also for platelets to form complexes with F. VIII to maintain plasma F. VIII at a normal level. Such functions are decreased and cause abnormalities in hemostasis function in vonWillebrand disease patients.

For developing pharmaceuticals that (i) have long administration intervals, (ii) can be administered easily, (iii) are not influenced by the presence of inhibitors, and (iv) functionally substitute for F. VIII/F. VIIIa independently of them, methods that utilize antibodies may be used. Antibody half-life in blood is, in general, relatively long, ranging from a few days to few weeks. Antibodies generally translocate into the blood after subcutaneous administration. That is, generally, antibody pharmaceuticals satisfy the above-mentioned properties (i) and (ii).

(Pharmaceutical) compositions comprising the antibodies as disclosed herein and pharmaceutically acceptable carriers are disclosed herein. For example, antibodies as disclosed herein that recognize both F. IX or F. IXa and F. X, and functionally substitute for F. VIII are expected to become pharmaceuticals (pharmaceutical compositions) or pharmaceutical agents for preventing and/or treating bleeding, diseases accompanying bleeding, diseases caused by bleeding, and the like.

In the context of the present disclosure, bleeding, diseases accompanying bleeding, and/or diseases caused by bleeding preferably refer to diseases that develop and/or progress due to reduction or deficiency in activity of coagulation factor VIII and/or activated coagulation factor VIII. Such diseases include hemophilia A, diseases in which an inhibitor against coagulation factor VIII and/or activated coagulation factor VIII appear, acquired hemophilia, and vonWillebrand's disease, but are not limited thereto.

Pharmaceutical compositions used for therapeutic or preventive purposes, which comprise antibodies described herein as active ingredients, can be formulated by mixing, if necessary, with suitable pharmaceutically acceptable carriers, vehicles, and such that are inactive against the antibodies. For example, sterilized water, physiological saline, stabilizers, excipients, antioxidants (such as ascorbic acid), buffers (such as phosphate, citrate, and other organic acids), antiseptics, surfactants (such as PEG and Tween), chelating agents (such as EDTA), and binders may be used. They may also comprise other low-molecular-weight polypeptides, proteins such as serum albumin, gelatin, and immunoglobulins, amino acids such as glycine, glutamine, asparagine, arginine, and lysine, sugars and carbohydrates such as polysaccharides and monosaccharides, and sugar alcohols such as mannitol and sorbitol. When preparing an aqueous solution for injection, physiological saline and isotonic solutions comprising glucose and other adjuvants such as D-sorbitol, D-mannose, D-mannitol, and sodium chloride may be used, and if necessary, in combination with appropriate solubilizers such as alcohol (for example, ethanol), polyalcohols (such as propylene glycol and PEG), and nonionic surfactants (such as polysorbate 80 and HCO-50).

If necessary, antibodies described herein may be encapsulated in microcapsules (e.g., those made of hydroxymethylcellulose, gelatin, and poly(methylmetacrylate)), or incorporated as components of colloidal drug delivery systems (e.g., liposomes, albumin microspheres, microemulsion, nanoparticles, and nanocapsules) (see, for example, "Remington's Pharmaceutical Science 16th edition", Oslo Ed. (1980)). Methods for preparing the pharmaceutical agents as controlled-release pharmaceutical agents are also well known, and such methods may be applied to the antibodies disclosed herein (Langer et al., J. Biomed. Mater. Res. 15: 267-277 (1981); Langer, Chemtech. 12: 98-105 (1982); U.S. Patent No. 3,773,919; EP Patent Application No. 58,481; Sidman et al., Biopolymers 22: 547-556 (1983); EP Patent Application No. 133,988).

The antibodies or pharmaceutical compositions disclosed herein can be used in combination with coagulation factor VIII, and can be administered with coagulation factor VIII simultaneously or at different times. The antibodies or pharmaceutical compositions disclosed herein and coagulation factor VIII may also be combined into a kit. When the antibodies or pharmaceutical compositions disclosed herein and coagulation factor VIII are used in combination, the dose of each component can be reduced as needed as compared to when the components are administered individually.

Two or more types of the bispecific antibodies or the pharmaceutical compositions of disclosed herein may be used in combination, and these antibodies or compositions can be used together with other bispecific antibodies against F. IX/F. IXa and F. X, anti-F. IX/F. IXa antibodies, anti-F. X antibodies, or combinations thereof. When two or more types of the bispecific antibodies or the pharmaceutical compositions disclosed herein are used in combination, or when these antibodies or compositions are used together with other bispecific antibodies against F. IX/F. IXa and F. X, anti-F. IX/F. IXa antibodies, anti-F. X antibodies, or combinations thereof, they can be administered simultaneously or at different times. The present disclosure may also be performed as a kit that combines two or more types of the bispecific antibodies or the pharmaceutical compositions disclosed herein , or combines these antibodies or compositions with other bispecific antibodies against F. IX/F. IXa and F. X, anti-F. IX/F. IXa antibodies, anti-F. X antibodies, or combinations thereof. Furthermore, when two or more types of the bispecific antibodies or the pharmaceutical compositions disclosed herein are used in combination, or when these antibodies or compositions are used together with another bispecific antibodies against F. IX/F. IXa and F. X, anti-F. IX/F. IXa antibodies, anti-F. X antibodies, or combinations thereof, the dose of each component may be reduced as needed as compared to when the components are administered individually.

The dose of a pharmaceutical composition disclosed herein may be appropriately determined by considering the dosage form, method of administration, patient age and body weight, symptoms of the patient, type of the disease, and degree of progress of the disease, and is ultimately decided by physicians. Generally, the daily dose for an adult is 0.1 mg to 2000 mg at once or in several portions. The dose is more preferably 1 to 1000 mg/day, even more preferably 50 to 500 mg/day, and most preferably 100 to 300 mg/day. These doses may vary, depending on the patient body weight and age, and the method of administration; however, selection of suitable dosage is well within the purview of those skilled in the art. Similarly, the dosing period may be appropriately determined depending on the therapeutic progress.

Gene therapy may be performed by incorporating genes encoding the antibodies described herein into vectors for gene therapy. In addition to direct administration using naked plasmids, suitable methods of administration include administration after packaging into liposomes and such, forming a variety of virus vectors such as retrovirus vectors, adenovirus vectors, vaccinia virus vectors, poxvirus vectors, adeno-associated virus vectors, and HVJ vectors (see Adolph "Viral Genome Methods" CRC Press, Florida (1996)), or coating with carrier beads such as colloidal gold particles (WO 93/17706, and such). However, so long as the antibodies are expressed *in vivo* and their activities are exercised, any method can be used for administration. Preferably, a sufficient dose can be administered by a suitable parenteral route (such as, for example, injecting or infusing intravenously, intraperitoneally, subcutaneously, intradermally, intramuscularly, into adipose tissues or mammary glands; inhalation; gas-driven particle bombardment (using electron gun and such); or mucosal route such as nasal drops). Alternatively, the genes encoding the antibodies disclosed herein may be administered into blood cells, bone marrow cells, and such *ex vivo* using liposome transfection, particle bombardment (U.S. Patent No. 4,945,050), or viral infection, and the cells may be reintroduced into animals. Any gene encoding an antibody disclosed herein may be used in gene therapy, and its examples include genes comprising nucleotide sequences encoding the CDRs of A44, A69, and B26 described above.

The present disclosure provides methods for preventing and/or treating bleeding, diseases accompanying bleeding, and/or diseases caused by bleeding, such methods comprising administering the antibodies or compositions disclosed herein. The antibodies or compositions can be administered, for example, by the above-mentioned methods.

The present disclosure also describes the use of the antibodies disclosed herein for producing (pharmaceutical) compositions disclosed herein.

Furthermore, the present disclosure describes kits to be used for the above-mentioned methods, such kits comprising at least an antibody or composition disclosed herein. In addition, the kits may include, packaged therewith, a syringe, injection needle, pharmaceutically acceptable vehicle, alcohol-soaked cotton, adhesive bandage, instructions describing the method of use, and the like.

### Examples

### [Example 1] Production of non-neutralizing antibody to Factor IXa (F.IXa)

### 1-1. Immunization and production of hybridomas

Eight BALB/c mice (male, aged 6 weeks at the initiation of immunization, Charles River Laboratories Japan, Inc.) and five MRL/1pr mice (male, aged 6 weeks at the initiation of immunization, Charles River Laboratories Japan, Inc.) were immunized against human Factor IXaβ (Enzyme Research Laboratories, Inc.) as described below. As the first immunization, 40 µg/head of Factor IXaβ, emulsified by Freund's complete adjuvant (FCA), H37Ra (Difco Laboratories), was subcutaneously administered. Two weeks later, 40 µg/head of Factor IXaβ, emulsified by Freund's incomplete adjuvant (FIA, Difco Laboratories), was subcutaneously administered. Thereafter, at weekly intervals, boosters were administered three to seven times. After an increase in serum antibody titer against Factor IXaβ was confirmed by an enzyme-linked immunosorbent assay (ELISA) described in the following Example 1-2, 40 µg/head of Factor IXaβ, diluted with calcium and magnesium ion-free phosphate buffered saline (PBS (-)), was intravenously administered as the final immunization. Three days after the final immunization, the spleens were extracted. A first part of each spleen was used in Example 10-2. The remaining spleen cells were fused with mouse myeloma cells, P3X63Ag8U.1 (hereinafter, referred to as P3U1, ATCC CRL-1597), in accordance with the conventional method, using PEG1500 (Roche Diagnostics). The fused cells, suspended in RPMI1640 medium (Invitrogen) containing 10% FBS (Invitrogen) (hereinafter, referred to as 10% FBS/RPMI1640), were plated in 96 well culture plate. On days 1, 2, 3, and 5 after the cell fusion, the medium was substituted with HAT selective medium (10% FBS/RPMI1640/2%HAT 50x concentrate (Dainippon Sumitomo Pharma Co., Ltd.)/5% BM-Condimed H1 (Roche Diagnostics)) to selectively culture the hybridomas. The culture supernatant collected on day 8 or 9 after the cell fusion was used to measure the binding activity to Factor IXa using an ELISA described in Example 1-2 and the hybridomas having Factor IXa binding activity were selected. Subsequently, the neutralizing activity to the enzyme activity of Factor IXa was measured by the method described in Example 1-3, and the hybridomas having no neutralizing activity to Factor IXa were selected. The hybridomas were cloned by performing the limiting dilution twice, in which one cell per well was plated on 96-well culture plate. On the cells which were confirmed as single colonies by the microscopic observation, ELISA and neutralizing activity measurement described in Examples 1-2 and 1-3 were carried out, and clones were selected. The ascites of the cloned antibody were prepared by the method described in Example 1-4 and the antibody was purified from the ascites. It was confirmed that the purified antibody does not extend activated partial thromboplastin time (APTT) by the method described in Example 1-5.

### 1-2. Factor IXa ELISA

After Factor IXaβ, diluted to 1 µg/mL with a coating buffer (100 mM sodium bicarbonate, pH 9.6, 0.02% sodium azide), was dispensed into Nunc-Immuno plate (Nunc-Immuno™ 96 MicroWell™ plates MaxiSorp™ (Nalge Nunc International)) at 100 µL/well, it was incubated overnight at 4°C. After the plate was washed three times with PBS (-) containing Tween® 20, it was blocked at room temperature for two hours with a diluent buffer (50 mM Tris-HCl, pH 8.1, 1% bovine serum albumin, 1 mM MgCl₂, 0.15 MNaCl, 0.05% Tween® 20, 0.02% sodium azide). After the buffer was removed, either mouse anti-serum or the culture supernatant of hybridoma diluted with the diluent buffer was added at 100 µL/well to the plate and incubated at room temperature for one hour. After the plate was washed three times, 100 µL/well of alkaline phosphatase-labeled goat anti-mouse IgG (H+L) (Zymed Laboratories), diluted to 1/2000 with the diluent buffer, was added and incubated at room temperature for one hour. After the plate was washed six times, 100 µL/well of colorimetric substrate Blue-Phos™ Microwell Phosphatase Substrate (Kirkegaard & Perry Laboratories) was added and incubated at room temperature for 20 minutes. After 100 µL/well of Blue-Phos™ Stop Solution (Kirkegaard & Perry Laboratories) was added, the absorbance at 595 nm was measured by Microplate Reader Model 3550 (Bio-Rad Laboratories).

### 1-3. Neutralizing activity measurement of Factor IXa

400 µg/mL of phospholipid solution was prepared by dissolving phospholipid (Sigma-Aldrich) with distilled water for injection and performing sonication. 40 µL of Tris-buffered physiological saline containing 0.1% bovine serum albumin (hereinafter, referred to as TBSB), 10 µL of 30 ng/mL Factor IXaβ (Enzyme Research Laboratories), 5 µL of 400 µg/mL phospholipid solution, 5 µL of TBSB containing 100 mM CaCl₂ and 20 mM MgCl₂, and 10 µL of hybridoma culture supernatant were mixed in a 96-well plate, followed by incubating at room temperature for one hour. 20 µL of 50 µg/mL Factor X (Enzyme Research Laboratories) and 10 µL of 3 U/mL of Factor VIII (American diagnostica) were added to this mixed solution and reacted at room temperature for 30 minutes. The reaction was stopped by adding 10 µL of 0.5M EDTA to the reaction mixture. After 50 µL of S-2222 solution (Chromogenix) was added to the reaction solution and incubated at room temperature for 30 minutes, the absorbance at 405 nm of measurement wavelength, 655 nm of control wavelength was measured by Microplate Reader Model 3550 (Bio-Rad Laboratories, Inc.).

### 1-4. Production of ascites and purification of antibody

Production of ascites of the established hybridoma was carried out according to the conventional method. Specifically, 2 x 10⁶ cells of hybridoma cultured *in vitro* were transplanted into the abdominal cavities of BALB/c mice (male, aged 5 to 7 weeks when the experiment started, Charles River Laboratories Japan) or BALB/c nude mice (male, age of 5 to 6 weeks at the initiation of the experiment, Charles River Laboratories Japan and CLEA Japan, Inc.), to which pristane (2, 6, 10, 14-tetramethylpentadecane; Wako Pure Chemical Industries, Ltd.) had been administered twice intraperitoneally. The ascites were collected from the mice whose abdomens were enlarged, one to four weeks after the transplantation.

Purification of antibody from ascites was carried out using Protein G Sepharose™ 4 Fast Flow (Amersham Biosciences) column. The ascites, diluted two-fold in the binding buffer (20 mM sodium acetate, pH 5.0), were applied to the column, and washed with 10-column volume of the binding buffer. The antibody was eluted in 5-column volume of the elution buffer (0.1 M glycine-HCl, pH 2.5), and neutralized with the neutralizing buffer (1 M Tris-HCl, pH 9.0). The resulting solution was condensed with Centriprep™ 10 (Millipore), and the solvent was substituted with TBS (50 mM Tris-buffered Saline). The antibody concentration was calculated from the absorbance at 280 nm based on A (1%, 1cm) = 13.5. The absorbance was measured by DU-650 (Beckman Coulter).

### 1-5. Measurement of activated partial thromboplastin time (APTT)

APTT was measured by KC10A (Amelung) connected to CR-A (Amelung). A mixture of 50 µL of the antibody solution, diluted with TBSB, 50 µL of standard human plasma (Dade Behring), and 50 µL of APTT reagent (Dade Behring), was heated at 37°C for 3 minutes. The coagulation reaction was initiated by adding 50 µL of 20 mM CaCl₂ (Dade Behring) to the mixture and the coagulation time was measured.

### [Example 2] Production of non-neutralizing antibody against Factor X (F.X)

### 2-1. Immunization and production of hybridoma

Eight BALB/c mice (male, aged 6 weeks at the initiation of immunization, Charles River Laboratories Japan) and five MRL/lpr mice (male, aged 6 weeks at the initiation of immunization, Charles River Laboratories Japan) were immunized against human Factor X (Enzyme Research Laboratories, Inc.) as described below. As the first immunization, 40 µg/head of Factor X, emulsified with FCA, was subcutaneously administered. After two weeks, 20 or 40 µg/head of Factor X, emulsified with FIA, was subcutaneously administered. Thereafter, at weekly intervals, the boosters were administered 3 to 6 times in total. After an increase in serum antibody titer against Factor X was confirmed by ELISA described in Example 2-2, 20 or 40 µg/head of Factor X, diluted in PBS (-), was intravenously administered as the final immunization. Three days after the final immunization, the spleens of the mice were removed. A first part of each spleen was used in Example 10-2. The remaining spleen cells were fused with mouse myeloma cells, P3U1, in accordance with the conventional method, using PEG1500. The fused cells, suspended in 10% FBS/RPMI1640 medium, were plated in a 96-well culture plate. On days 1, 2, 3, and 5 after the cell fusion, the medium was substituted with HAT selective medium to selectively culture the hybridomas. The binding activity to Factor X was measured using the culture supernatant collected on day 8 after the cell fusion by utilizing ELISA described in Example 2-2. The hybridomas having the binding activity to Factor X were selected. Subsequently, the neutralizing activity to the enzyme activity of Factor Xa was measured as described in Example 2-3. The hybridomas having no neutralizing activity to Factor Xa were cloned by performing the limiting dilution twice. The ascites of the cloned antibody were produced by the method described in Example 1-4 to purify the antibody from the ascites. It was confirmed that the purified antibody did not extend APTT by the method described in Example 1-5.

### 2-2. Factor X ELISA

After Factor X, diluted to 1 µg/mL with the coating buffer, was dispensed into Nunc-Immurio plate at 100 µL/well, it was incubated overnight at 4°C. The plate was washed three times with PBS (-) containing Tween® 20, and then blocked at room temperature for two hours by the diluent buffer. After the buffer was removed, either mouse anti-serum or the culture supernatant of hybridoma, diluted with the diluent buffer, was added to the plate, and incubated at room temperature for one hour. After the plate was washed three times, alkaline phosphatase-labeled goat anti-mouse IgG (H+L), diluted to 1/2000 with the diluent buffer, was added and incubated at room temperature for one hour. After the plate was washed 6 times, 100 µL/well of colorimetric substrate Blue-Phos™ Phosphatase Substrate (Kirkegaard & Perry Laboratories) was added and incubated at room temperature for 20 minutes. After 100 µL/well of Blue-Phos™ Stop Solution (Kirkegaard & Perry Laboratories) was added, the absorbance at 595 nm was measured with Microplate Reader Model 3550 (Bio-Rad Laboratories).

### 2-3. Measurement of Factor Xa neutralizing activity

10 µL of the hybridoma culture supernatant, five-fold diluted with TBSB, and 40 µL of TBCP (TBSB containing 2.78 mM CaCl₂, 22.2 µM phospholipid (phosphatidyl choline : phosphatidyl serine = 75:25, Sigma-Aldrich)) containing 250 pg/mL Factor Xa (Enzyme Research Laboratories) were mixed and incubated at room temperature for one hour. 50 µL of TBCP, containing 20 µg/mL of prothrombin (Enzyme Research Laboratories) and 100 ng/mL of activated coagulation factor V (Factor Va; Haematologic Technologies), was added to this mixed solution and reacted at room temperature for 10 minutes. The reaction was stopped by adding 10 µL of 0.5 M EDTA. After 50 µL of 1 mM S-2238 solution (Chromogenix) was added to this reaction solution and incubated at room temperature for 30 minutes, the absorbance at 405 nm was measured with Microplate Reader Model 3550 (Bio-Rad Laboratories).

### [Example 3] Construction of chimeric bispecific antibody expression vectors 3-1. Preparation of DNA fragments encoding antibody variable regions from hybridomas

From hybridomas producing anti-F.IXa antibody or anti-F.X antibody, total RNAs were extracted using QIAGEN® Rneasy® Mini Kit (QIAGEN) in accordance with the method described in the instruction manual. Total RNAs were dissolved in 40 µL of sterilized water. Single strand cDNAs were synthesized by RT-PCR method using SuperScript cDNA synthesis system (Invitrogen) in accordance with the method described in the instruction manual, using 1 to 2 µg of the purified RNAs as a template.

### 3-2. PCR Amplification and sequence analysis of antibody H chain variable region

As primers for amplification of mouse antibody H chain variable region (VH) cDNAs, HB primer mixture and HF primer mixture described in the report by Krebber et al. (J. Immunol. Methods 1997; 201:35-55) were prepared. 25 µL of the reaction solution (2.5 µL of cDNA solution prepared in Example 3-1, KOD plus buffer (TOYOBO), 0.2 mM dNTPs, 1.5 mM MgCl₂, and 0.75 units of DNA polymerase KOD plus (TOYOBO)) was prepared using 0.5 µL each of 100 µM HB primer mixture and 100 µM HF primer mixture. PCR was carried out using Thermal Cycler GeneAmp PCR system 9700 (Perkin Elmer) either in the condition A (heating at 98°C for 3 minutes, followed by 32 cycles of reactions at 98°C for 20 seconds, 58°C for 20 seconds, and 72°C for 30 seconds) or in the condition B (heating at 94°C for 3 minutes, followed by 5 cycles of reactions at 94°C for 20 seconds, 46°C for 20 seconds, and 68°C for 30 seconds, and further 30 cycles of reactions at 94°C for 20 seconds, 58°C for 20 seconds, 72°C for 30 seconds) according to the amplification efficiency of cDNA fragments. After performing PCR, the reaction solution was subjected to 1% agarose gel electrophoresis. The amplified fragments of the desired size (about 400 bp) were purified using QIAquick Gel Extraction Kit (QIAGEN) by the method described in the attached instruction manual and eluted using 30 µL of sterilized water. The nucleotide sequence of each DNA fragment was determined using BigDye Terminator Cycle. Sequencing Kit (Applied Biosystems) with DNA sequencer ABI PRISM 3100 Genetic Analyzer (Applied Biosystems) according to the method described in the attached instruction manual. The sequences determined by the present method were compared and analyzed using GENETYX-SV/RC Version 6.1 (Genetyx) and those having a different sequence were selected.

### 3-3. Preparation of antibody variable region DNA fragments for cloning

In order to add restriction enzyme Sfi I cleavage site for cloning to both ends of antibody variable region amplification fragments, the following procedures were performed.

For amplifying the VH fragment including an added Sfi I cleavage site (Sfi I-VH), primer VH-5' end whose (Gly4Ser)2-linker sequence of primer HB had been changed into the sequence having Sfi I cleavage site (SEQ ID NO: 42) was prepared. Using 0.5 µL each of 10 µM sequence specific primer VH-5' end and 10 µM primer scfor (J. Immunol. Methods 1997; 201: 35-55), 20 µL of reaction solution (1 µL of purified VH cDNA amplification fragment solution prepared in Example 3-2, KOD plus buffer (TOYOBO), 0.2 mM dNTPs, 1.5 mM MgCl₂, 0.5 units of DNA polymerase KOD plus (TOYOBO)) was prepared. PCR was carried out using Thermal Cycler GeneAmp PCR system 9700 (Perkin Elmer) either in the condition A (heating at 98°C for 3 minutes, followed by 32 cycles of reactions at 98°C for 20 seconds, 58°C for 20 seconds, and 72°C for 30 seconds) or in the condition B (heating at 94°C for 3 minutes, followed by 5 cycles of reactions at 94°C for 20 seconds, 46°C for 20 seconds, and 68°C for 30 seconds, and further 30 cycles of reactions at 94°C for 20 seconds, 58°C for 20 seconds, and 72°C for 30 seconds) according to the amplification efficiency of cDNA fragments. After performing PCR, the reaction solution was subjected to 1% agarose gel electrophoresis. The amplified fragments of the desired size (about 400 bp) were purified using QIAquick Gel Extraction Kit (QIAGEN) by the method described in the attached instruction manual and eluted using 30 µL of sterilized water.

For amplifying a mouse antibody L chain variable region (VL) cDNA fragment, first, using 0.5 µL each of 100 µM LB primer mixture and 100 µM LF primer mixture described in the report by Krebber et al. (J. Immunol. Methods 1997; 201:35-55), 25 µL of the reaction solution (2.5 µL of cDNA solution prepared in Example 3-1, KOD plus buffer (TOYOBO), 0.2 mM dNTPs, 1.5 mM MgCl₂, 0.75 units of DNA polymerase KOD plus (TOYOBO)) was prepared. PCR was carried out using Thermal Cycler GeneAmp PCR system 9700 (Perkin Elmer) in the conditions of heating at 94°C for 3 minutes, followed by 5 cycles of reactions at 94°C for 20 seconds, 46°C for 20 seconds, and 68°C for 30 seconds, and further 30 cycles of reactions at 94°C for 20 seconds, 58°C for 20 seconds, and 72°C for 30 seconds, according to the amplification efficiency of the cDNA fragments. After performing PCR, the reaction solution was subjected to 1% agarose gel electrophoresis. The amplified fragments of the desired size (about 400 bp) were purified using QIAquick Gel Extraction Kit (QIAGEN) by the method described in the attached instruction manual and eluted using 30 µL of sterilized water. The fragments were in a state where (Gly4Ser) 3-linker sequence derived from primer LF was added to their C-terminus. To add an Sfi I cleavage site to their C-terminus, primer VL-3' end in which (Gly4Ser) 3-linker sequence of primer LF had been changed into the sequence having Sfi I cleavage site (SEQ ID NO: 43) was prepared. In order to amplify the VL fragment including the added Sfi I cleavage site (Sfi I-VL), 0.5 µL each of 10 µM VL-3' end primer mixture and 10 µM scback primer was used to prepare 20 µL of reaction solution (1 µL of purified VL cDNA amplification fragment solution, KOD plus buffer (TOYOBO), 0.2 mM dNTPs, 1.5 mM MgCl₂, 0.5 units of DNA polymerase KOD plus (TOYOBO)). PCR was carried out using Thermal Cycler GeneAmp PCR system 9700 (Perkin Elmer) under the conditions of heating at 94°C for 3 minutes, followed by 5 cycles of reactions at 94°C for 20 seconds, 46°C for 20 seconds and 68°C for 30 seconds, and further 30 cycles of reactions at 94°C for 20 seconds, 58°C for 20 seconds, and 72°C for 30 seconds . After performing PCR, the reaction solution was subjected to 1% agarose gel electrophoresis. The amplified fragments of the desired size (about 400 bp) were purified using QIAquick Gel Extraction Kit (QIAGEN) by the method described in the attached instruction manual and eluted using 30 µL of sterilized water.

The purified Sfi I-VH and Sfi I-VL fragments were digested with Sfi I (TAKARA) at 50°C overnight in the reaction solution prepared according to the method described in the attached instruction manual. Subsequently, the reaction solution was purified using QIAquick PCR Purification Kit (QIAGEN) by the method described in the attached instruction manual, and eluted using 30 µL of Buffer EB attached to the kit.

### 3-4. Bispecific IgG antibody expression plasmids

When the desired bispecific IgG antibodies were produced, amino acid substitution products in CH3 region of IgG4 were prepared with reference to the knobs-into-holes technique of IgG1 (Ridgway et al., Protein Eng. 1996; 9: 617-621) to form heterogeneous molecules of each H chain. Type a (IgG4ya) is a substitution product of Y349C or T366W, and type b (IgG4γb) is a substitution product of E356C, T366S, L368A, or Y407V. Furthermore, the substitutions (-ppcpScp- - and -ppcpPcp-) were introduced in the hinge region of both types of substitution products. According to the present technique, almost all of the H chains may become heterogeneous. However, this is not the case for L chains, and there is a fear that the unnecessary production of an antibody molecule can influence the subsequent activity measurement. Therefore, in the present strategy, expression vectors induced by different agents were used as expression vectors corresponding to each of antibody molecule one arm (referred to as HL molecule) in order to separately express each HL molecule having each specificity and efficiently produce the desired type of bispecific IgG antibody in the cells.

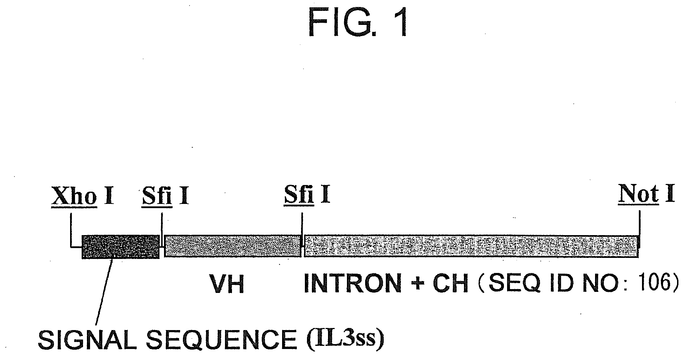

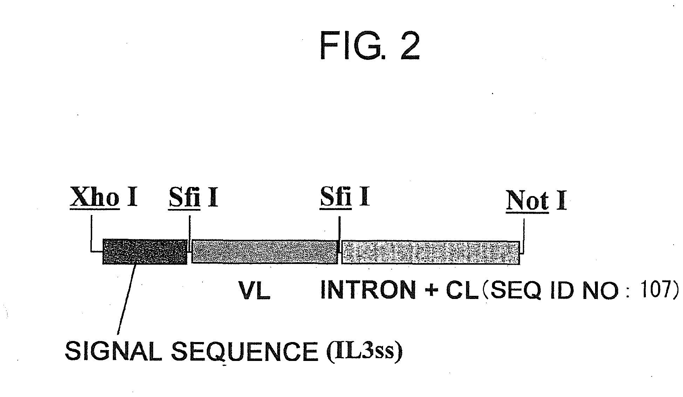

For expression of an antibody molecule one arm (for convenience, referred to as the right arm HL molecule), pcDNA4-g4H or pcDNA4-g4L was prepared, in which downstream of the corresponding region of H chain or L chain (Fig. 1 or 2), that is, the signal sequence for animal cells (IL3ss) (Proc. Natl. Acad. Sci. USA. 1984; 81: 1075), an appropriate mouse antibody variable region (VH or VL) and human IgG4γa constant region (SEQ ID NO: 44) or κ constant region (SEQ ID NO: 45) were incorporated to tetracycline-inducible vector pcDNA4 (Invitrogen). First, pcDNA4 was digested with restriction enzymes Eco RV and Not I (TAKARA) whose cleavage sites exist in the multicloning site. After the chimeric bispecific antibody right arm H chain or L chain-expressing unit (respectively, about 1.6 kb or about 1.0 kb) was digested with Xho I (TAKARA), it was purified using QIAquick PCR Purification Kit (QIAGEN) by the method described in the attached instruction manual, the ends were blunted with DNA polymerase KOD (TOYOBO) by reacting at 72°C for 10 minutes in the reaction solution described in the attached instruction manual. The blunt-ended fragments were purified using QIAquick PCR Purification Kit (QIAGEN) by the method described in the attached instruction manual and digested with Not I (TAKARA). The Not I-blunt fragments (about 1.6 kb and 1.0 kb, respectively) and pcDNA4 which had been digested with Eco RV-Not I were ligated using Ligation High (TOYOBO) according to the method described in the attached instruction manual. *E.coli* DH5 α strain (Competent high DH5 α (TOYOBO)) was transformed with the reaction solution. Respective plasmid DNAs were isolated from the obtained ampicillin resistant clones using QIAprep Spin Miniprep Kit (QIAGEN).

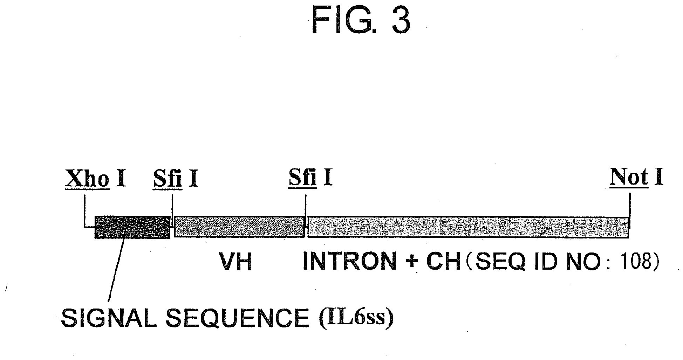

For the other one arm (for convenience, referred to as the left arm HL molecule), pIND-g4H or pIND-g4L was prepared, in which downstream of the corresponding regions of H chain or L chain (Fig. 2 or 3), that is, the signal sequence for animal cells (IL3ss) (EMBO. J. 1987; 6: 2939), an appropriate mouse antibody variable region (VH or VL) and human IgG4yb constant region (SEQ ID NO: 46) or κ constant region (SEQ ID NO: 45) were incorporated to ecdysone analogue-inducible vector pIND (Invitrogen) according to the above-described method. The respective plasmid DNAs were then isolated as described above.

### 3-5. Construction of bispecific antibody expression vectors

Tetracycline-inducible expression plasmid (pcDNA4-g4H or pcDNA4-g4L) prepared in Example 3-4 was digested with Sfi I, and the reaction solution was subjected to 1% agarose gel electrophoresis. The fragments (about 5 kb) in which the antibody variable region originally present (VH or VL (see Fig. 1 or 2)) had been removed were purified using QIAquick Gel Extraction Kit (QIAGEN) by the method described in the attached instruction manual, and eluted using 30 µL of sterilized water. These fragments and their corresponding Sfi I-VH or Sfi I-VL fragments, derived from the Sfi I-digested antibody F. IXa prepared in Example 3-3, were ligated using Quick Ligation Kit (New England Biolabs) according to the method described in the attached instruction manual. *E. coli* DH5 α strain (Competent high DH5α (TOYOBO)) was transformed with the reaction solution. Moreover, the fragment in which the antibody variable region (VH or VL, see Fig. 3 or 2) had been removed from Sfi I-digested ecdysone analogue-inducible expression plasmid (Example 3-4, pIND-g4H or pIND-g4L) by a method similar to that described above, and Sfi I-VH or Sfi I-VL fragment derived from Sfi I-digested anti-F. X antibody were incorporated by a method similar to that described above.

The resulting respective ampicillin resistant transformants were confirmed to have the insertion of the desired fragment using a primer that sandwiches the inserted fragment by the colony PCR method. First, for anti-F. IXa antibody chimeric H chain or L chain expression vector, primer CMVF (SEQ ID NO: 47), which is 21-mer and anneals to CMV Forward priming site existing upstream of the insertion site, and primer BGHR (SEQ ID NO: 48), which is 18-mer and anneals to BGH Reverse priming site existing downstream of the insertion site, were synthesized (Sigma Genosys). For anti-F. X antibody chimeric H chain or L chain expression vector, primer EcdF (SEQ ID NO: 49), which is 24-mer and anneals to the upstream of the insertion site, and primer BGHR (SEQ ID NO: 48), which is 18-mer and anneals to BGH Reverse priming site existing downstream of the insertion site, were synthesized (Sigma Genosys). For colony PCR, 20 µL of the reaction solution (0.2 µL each of 10 µM primer, KOD dash buffer (TOYOBO), 0.2 mM dNTPs, 0.75 units of DNA polymerase KOD dash (TOYOBO)) were prepared. The appropriate amount of the transformants was added to the reaction solution, and PCR was carried out. PCR was performed using Thermal Cycler GeneAmp PCR system 9700 (Perkin Elmer) under the conditions of heating at 96°C for 1 minute, followed by 30 cycles of reactions at 96°C for 10 seconds, 55°C for 10 seconds, and 72°C for 30 seconds. After performing PCR, the reaction solution was subjected to 1% agarose gel electrophoresis, and the clones whose amplified fragments had the desired size were selected. In the PCR products, excessive primers and dNTPs were inactivated using ExoSAP-IT (Amersham Biosciences) according to the attached instruction manual. The nucleotide sequence of each DNA fragment was determined using BigDye Terminator Cycle Sequencing Kit (Applied Biosystems) with DNA sequencer ABI PRISM 3100 Genetic Analyzer (Applied Biosystems) according to the attached instruction manual. The sequences determined by the present method were analyzed using an analyzing software GENETYX-SV/RC Version 6.1 (Genetyx), the desired clones in which for VH, insertions, deletions, mutations and the like were not introduced, and the desired clones in which for VL, insertions, deletions, mutations and the like were not introduced different from pseudo VL gene derived from P3U1 used in hybridomas, were selected.

The respective plasmid DNAs were isolated from the desired clones using QIAprep Spin Miniprep Kit (QIAGEN) and dissolved in 100 µL of sterilized water. Anti-F. IXa antibody chimeric H chain expression vector, anti-F. IXa antibody chimeric L chain expression vector, anti-F. X antibody chimeric H chain expression vector, and anti-F. X antibody chimeric L chain expression vector were dubbed as pcDNA4-g4IXaHn, pcDNA4-g4IXaLn, pIND-g4XHn, and pIND-g4XLn. The respective plasmid solutions were preserved at 4°C until use.

### [Example 4] Expression of chimeric bispecific antibodies

### 4-1. Preparation of DNA solutions

Expression vectors for the antibody right arm HL molecule (pcDNA4-g4IXaHn and pcDNA4-g4IXaLn) are induced by tetracycline. In order to completely suppress the expression in the absence of tetracycline, a plasmid pcDNA6/TR (Invitrogen) encoding Tet repressor is required. Moreover, expression vectors for the antibody left arm HL molecule (pIND-g4XHn and pIND-g4XLn) are induced by ecdysone analogue (Ponasterone A), which is a hormone of insects. Thus, a plasmid pVgRXR (Invitrogen) is required, which encodes an ecdysone receptor that reacts with Ponasterone A and induces expression and a retinoid X receptor. Therefore, a total of 6 kinds of plasmid DNA mixed solutions were prepared to transfect animal cells. For 1 mL of cell culture, 218.8 ng each of pcDNA4-g4IXaHn, pcDNA4-g4IXaLn, pIND-g4XHn, and pIND-g4XLn, and 1312.5 ng each of pcDNA6/TR and pVgRXR were used.

### 4-2. Transfection of animal cells

The HEK293H strain (Invitrogen) derived from human fetal renal carcinoma cell was suspended in DMEM medium containing 10% FCS (MOREGATE), 1 mL of it (5 x 10⁵ cells/mL) was plated in each well of a 12-well plate for adherent cell (CORNING) cultured in a CO₂ incubator (37°C, 5% CO₂). The plasmid DNA mixture prepared in Example 4-1 and 7 µL of transfection reagent, Lipofectamine 2000 (Invitrogen) was added to 250 µL of Opti-MEM I medium (Invitrogen) and left to stand at room temperature for 20 minutes, and the resulting mixture was added to the cells in each well, and then incubated for 4 to 5 hours in a CO₂ incubator (at 37°C, 5% CO₂).

### 4-3. Induced expression of bispecific IgG antibodies

As described above, the medium was removed by aspiration from the transfected cell culture, and then 1 mL of CHO-S-SFM-II medium (Invitrogen) containing 1 µg/mL of tetracycline (Wako Pure Chemical Industries, Ltd.) was added thereto and cultured for one day in a CO₂ incubator (at 37°C, 5% CO₂) to induce the primary expression of the antibody right arm HL molecule. Subsequently, the medium was removed by aspiration and the cells were washed once with 1 mL of CHO-S-SFM-II medium. 1 mL CHO-S-SFM-II medium containing 5 µM of Ponasterone A (Invitrogen) was and the cells were cultured for 2 or 3 days in a CO₂ incubator (at 37°C, 5% CO₂) to induce the secondary expression of antibody left arm HL molecule and secrete a bispecific IgG antibody into the medium. After the culture supernatant was collected, the cells were removed by centrifugation (at approximately 2,000x g for 5 minutes at room temperature) and, as necessary, the resulting solution was concentrated using Microcon® YM-50 (Millipore). This sample was then preserved at 4°C until use.

### [Example 5] Quantitative determination of human IgG concentration

1 µg/mL of Goat affinity purified antibody to human IgG Fc (Cappel) was prepared with the coating buffer and immobilized in a Nunc-Immuno plate. After the plate was blocked with the diluent buffer (DB), the culture supernatant sample, appropriately diluted using DB, was added. Moreover, as the standard for calculating antibody concentration, a two-fold dilution series of human IgG4 (humanized anti-TF antibody, see WO 99/51743) which was produced by an 11-step dilution from 1000 ng /mL using DB were similarly added. After the sample was washed three times, goat anti-human IgG and alkaline phosphatase (Biosource International) were reacted. After the mixture was washed five times, Sigma 104® phosphatase substrate (Sigma-Aldrich) was colored as a substrate, the absorbance at 405 nm was measured with a reference wavelength of 655 nm using an absorbance reader Model 3550 (Bio-Rad Laboratories). Human IgG concentration in the culture supernatant was calculated from the standard curve using Microplate Manager III (Bio-Rad Laboratories) software.

### [Example 6] Activation coagulation factor VIII (F. VIIIa)-like activity assay

F. VIIIa-like activity of the bispecific antibody was evaluated by the following enzyme assay. Moreover, all of the following reactions were carried out at room temperature. A mixture of 40 µL of Factor IX (3.75 µg/mL, Enzyme Research Laboratories) and 10 µL of the antibody solution were incubated for one hour in a 96-well plate. Furthermore, 10 µL of Factor XIa (10 ng/mL, Enzyme Research Laboratories), 20 µL of Factor X (50 µg/mL, Enzyme Research Laboratories), 5 µL of phospholipid (400 µg/mL, see Example 1-3), and 15 µL of TBSB containing 5 mM CaCl₂ and 1 mM MgCl₂ (hereinafter, referred to as TBSB-S) were added thereto, and the enzyme reaction was initiated. After the reaction was performed for 30 minutes, it was stopped by adding 10 µL of 0.5M EDTA.

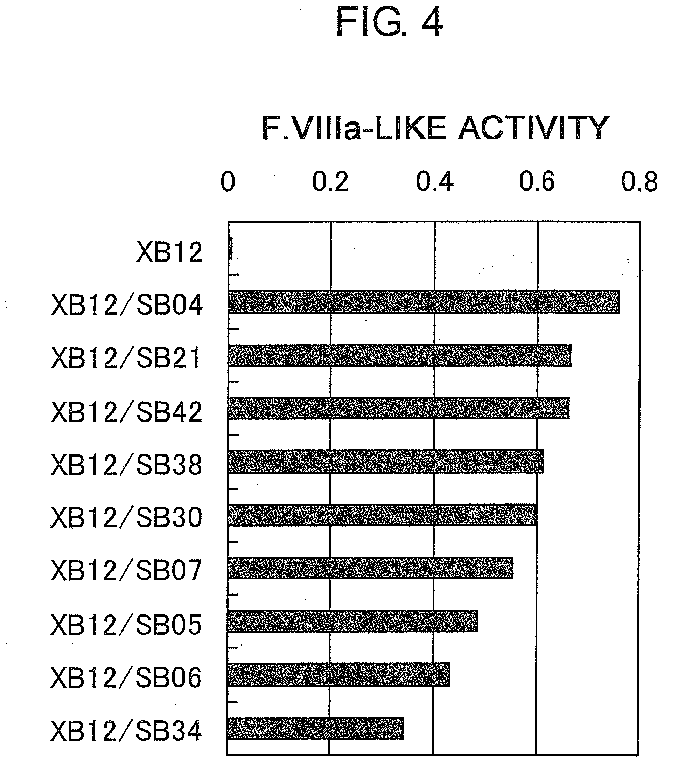

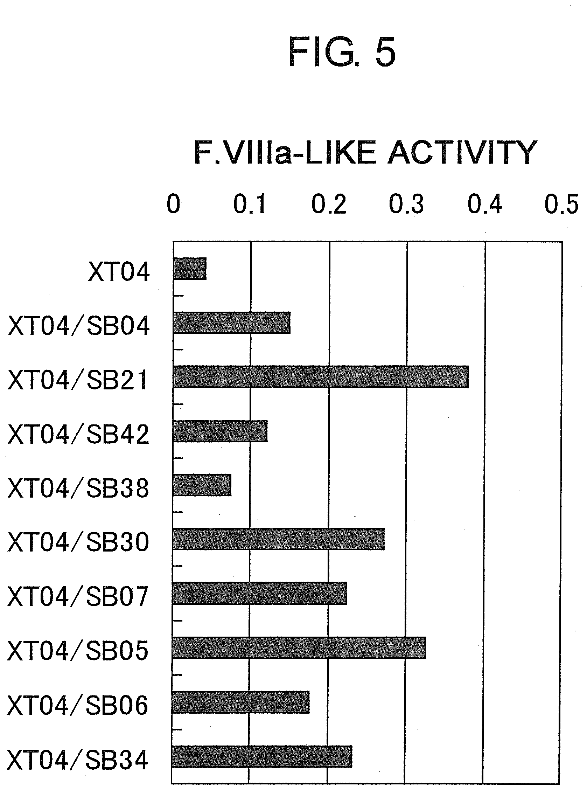

After 50 µL of colorimetric substrate solution was added to each well, the absorbance at 405 nm (reference wavelength, 655 nm) was measured at 0 minute and 30 minutes using a Model 3550 Microplate Reader (Bio-Rad Laboratories). F. VIIIa-like activity was represented by the value in which the absorbance change value in the absence of antibody for 30 minutes was subtracted from that in the presence of antibody for 30 minutes (see Figs. 4 and 5).

TBSB was used as a solvent of phospholipid, and TBSB-S was used as a solvent of Factor XIa, Factor IX, and Factor X. The colorimetric substrate solution was the mixture of "tesutochimu" colorimetric substrate S-2222 (Chromogenix) which has been dissolved according to the attached instruction manual and polybrene solution (0.6 mg/L hexadimethrine bromide (Sigma)) at the ratio of 1:1.

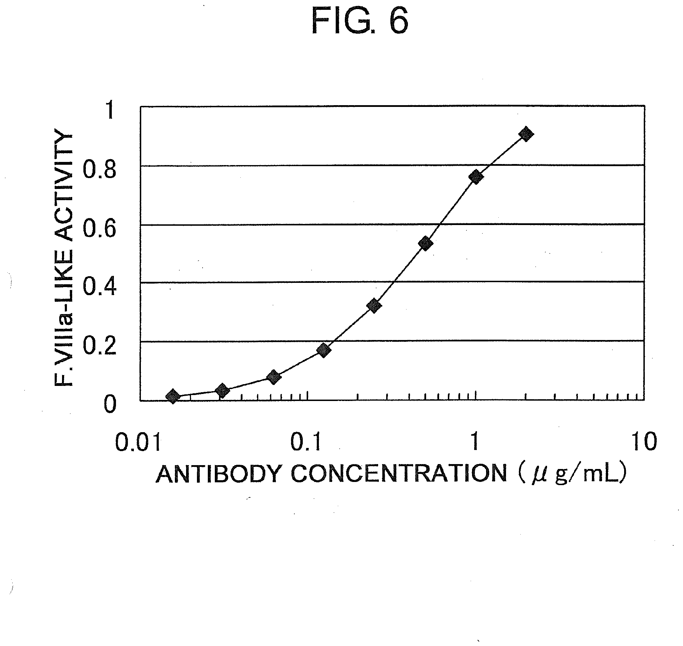

Furthermore, for XB12/SB04 which has the highest activity, the concentration dependency of F. VIIIa-like activity was measured (Fig. 6).

### [Example 7] Plasma coagulation assay

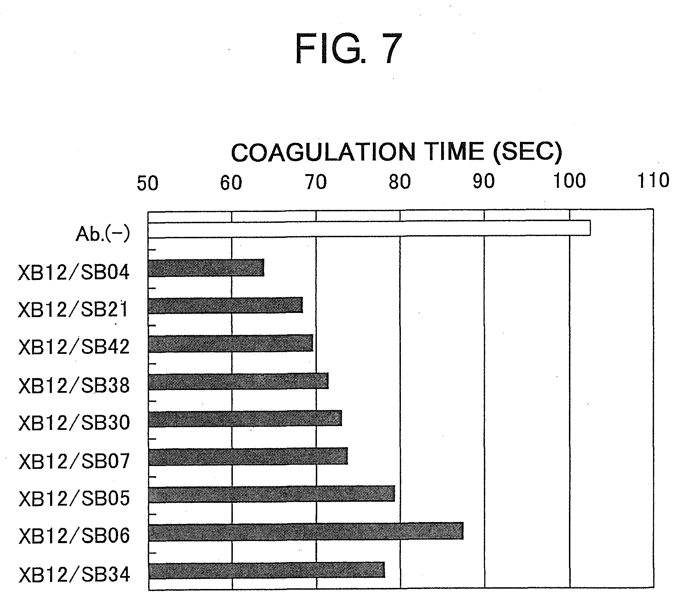

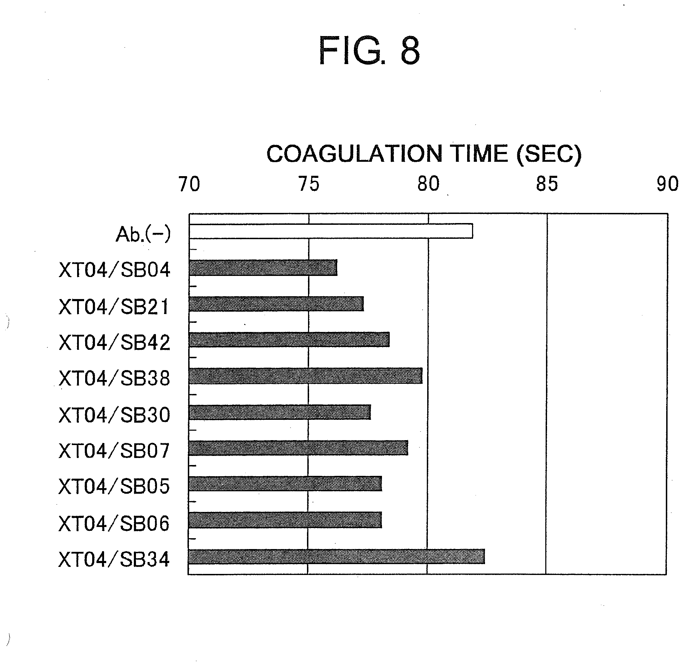

To determine whether the bispecific antibodies of the present invention were capable of correcting the coagulation ability of the blood of hemophilia A, the effect of these antibodies on the activated partial thromboplastin time (APTT) using F. VIII deficient plasma was examined. A mixture of 50 µL of an antibody solution having a variety of concentrations, 50 µL of F. VIII deficient plasma (Biomerieux), and 50 µL of the APTT reagent (Dade Behring) was warmed at 37°C for 3 minutes. The coagulation reaction was initiated by adding 50 µL of 20 mM CaCl₂(Dade Behring) to the mixture. The time period until coagulation was measured with KC10A (Amelung) connected to CR-A (Amelung) (Figs. 7 and 8).

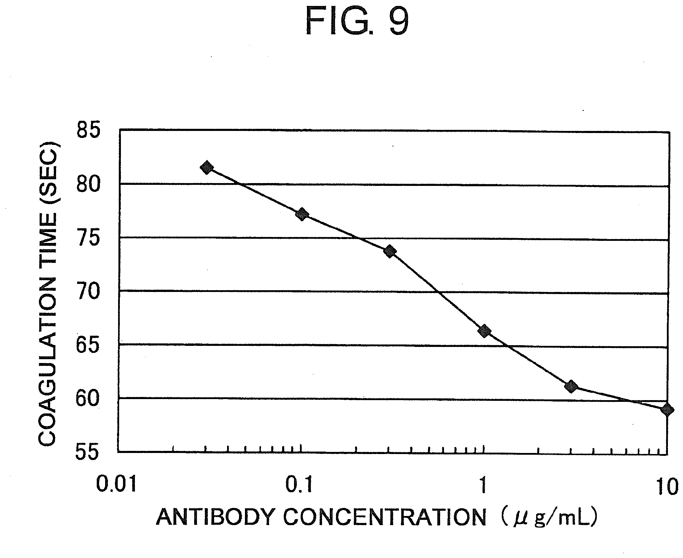

Furthermore, the concentration dependency of XB12/SB04, which exhibited the highest coagulation time-reducing effect, was measured (see Fig. 9).

### [Example 8] Antibody purification

10 mL of the culture supernatant obtained by the method described in Example 4 was concentrated to 1 mL using Centricon® YM-50 (Millipore). To this, 10 µL of 10% BSA, 10 µL of 1% Tween® 20, and 100 µL of rProtein A Sepharose™ Fast Flow (Amersham Biosciences) were added and mixed by inversion overnight at 4°C. The solution was transferred to a 0.22 µm filter cup, Ultrafree®-MC (Millipore) and washed three times with 500 µL of TBS containing 0.01% Tween® 20. Subsequently, rProtein A Sepharose™ resin was suspended in 10 mM HCl, pH 2.0 containing 100 µL of 0.01% Tween® 20 and left to stand for 3 minutes, and then the antibody was eluted. Immediately after this, 5 µL of 1 M Tris-HCl, pH 8.0 was added to it and neutralized. Human IgG concentration in the culture supernatant was calculated from the standard curve using Microplate Manager III (Bio-Rad Laboratories) software. The antibody concentration was quantitatively determined according to Example 5.

### [Example 9] GST-AP of anti-F.X antibody Western blotting

A recombinant *E. coli* for expressing fusion protein (GST-AP) between activated peptide of F. X (AP) and glutathione S transferase (GST) was constructed. After cDNA covering the full length translation region of human F. X was amplified from human liver Marathon-Ready cDNA (Clontech) by the PCR method, it was further used as a template to amplify the coding region of the AP region (Leytus et al., Biochemistry 1986; 25: 5098) by the PCR method, and then was subcloned into pGEM-T vector (Promega) to obtain pGEX-F10AP encoding GST-AP. *E. coli* which was transformed with this plasmid was cultured, and 1 mM IPTG was added when the OD 600 reached 0.8 to induce the expression of GST-AP. After the culture medium was centrifuged (at 3,000x g, for 30 minutes, at 4°C), the bacterial bodies were collected and stored at -20°C until use.

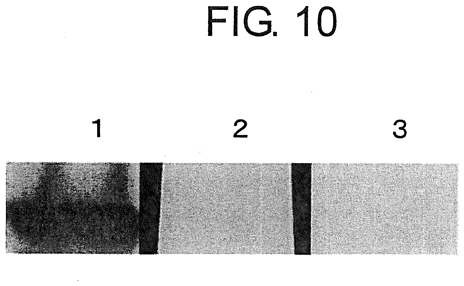

The bacterial body pellet was resuspended in 1/20 culture volume of PBS. SDS-PAGE sample buffer (IWAKI) was added at the ratio of 2.4 mL per 0.1 mL of the suspension, which was then boiled at 95°C for 5 minutes. 10 µL of the reaction solution was added to each well of the SDS-PAGE mini (14%) gel (Asahi Techno Glass Corporation), and the electrophoresis was carried out. The electrophoresed gel was transferred onto Immobilon-P™ Transfer Membrane (Millipore) using a semi-dry blotter (BIO-RAD), and the membrane was blocked with BT-PBS (PBS containing 2% BSA and 0.05% Tween® 20). After the blocking was completed, the membrane was reacted for one hour with anti-F. X mouse antibody SB04 or SB06, which were purified in Example 1-4 and diluted with BT-PBS to 2 µg/mL. After washing with PBS containing 0.05% Tween® 20, the membrane was reacted for one hour with alkaline phosphatase-labeled goat anti-mouse IgG (H+L) (Zymed Laboratories) which was diluted to 1/2000 with BT-PBS. After washing with PBS containing 0.05% Tween® 20, the membrane was reacted with the colorimetric substrate BCIP/NBT Phosphatase Substrate (Kirkegaard & Perry Laboratories) (see Fig. 10).

### [Example 10] Acquisition of bispecific antibodies from the scFv library derived from immunized mouse spleens

### 10-1. Antigen and immunization

Three BALB/c mice (male, aged 6 weeks at the initiation of immunization, Charles River Laboratories Japan), three MRL/1pr mice (male, aged 6 weeks at the initiation of immunization, Charles River Laboratories Japan), and three C57BL/6N mice (male, aged 6 weeks at the initiation of immunization, Charles River Laboratories Japan) were immunized against an antigen, Factor IXaβ (Enzyme Research Laboratories, Inc.) or Factor X (Enzyme Research Laboratories, Inc.) as described below. As the priming, 40 µg/head of an antigen emulsified by Freund's Complete Adjuvant (FCA) (H37 Ra, Difco Laboratories) was subcutaneously administered. After two weeks, 40 µg/head of an antigen emulsified by Freund's Incomplete Adjuvant (FIA) (Difco Laboratories) was subcutaneously administered. Thereafter, the booster immunizations were administered three times at weekly intervals. Eight days from the final immunization, the spleens were removed.

### 10-2. Construction of phage library

Portions of the removed spleens from the immunized mice which were prepared in Examples 1-1 and 2-1 and the removed spleens from the immunized mice prepared in Example 10-1 were added to Trizol Reagent (Invitrogen) (50 mg spleen/mL of the reagent) and homogenized using a glass homogenizer. Subsequently, according to the method described in the instruction manual attached to the reagent, total RNAs were extracted. Poly A (+) RNAs were extracted from the extraction using PolyATract System 1000 kit (Promega) according to the method described in the attached instruction manual. cDNAs were synthesized by RT-PCR (SuperScript III First-Strand Synthesis System for RT-PCR, Invitrogen), and stored at -20°C until use.

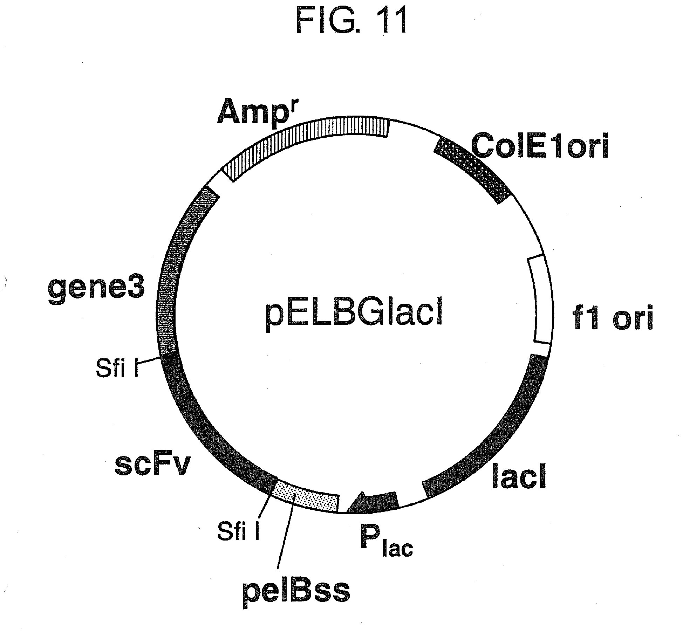

As primers for amplification of mouse antibody H chain variable region (VH) cDNA and mouse antibody L chain variable region (VL) cDNA, HB primer mixture, HF primer mixture, LB primer mixture, and LF primer mixture which were used in Examples 3-2 and 3-3 were prepared. As primers for VH amplification, 1 µL each of 100 µM HB primer mixture and 100 µM HF primer mixture was used to prepare 50 µL of the reaction solution (2.5 µL of cDNA solution, KOD plus buffer (TOYOBO), 0.2 mM dNTPs, 1.5 mM MgCl₂, 3.75 units of DNA polymerase KOD plus (TOYOBO)). As primers for VL amplification, 1 µL each of 100 µM LB primer mixture and 100 µM LF primer mixture was used to prepare 50 µL of the reaction solution having the similar components to the above-described solution. PCR was carried out using Thermal Cycler GeneAmp PCR system 9700 (Perkin Elmer) in the conditions of heating at 98°C for 3 minutes, followed by 32 cycles of reactions at 98°C for 20 seconds, 58°C for 20 seconds, and 72°C for 30 seconds. After PCR was carried out, the reaction solution was subjected to 2% agarose gel electrophoresis. The amplified fragments of the desired size (about 400 bp) were purified using QIAquick Gel Extraction Kit (QIAGEN) by the method described in the attached instruction manual and eluted using 50 µL of sterilized water. Next, for amplifying scFv fragments, 10 samples of 100 µL of the reaction solution (3 µL of VH fragment solution, 3 µL of VL fragment solution, KOD plus buffer (TOYOBO), 0.2 mM dNTPs, 1 mM MgCl₂, and 5 units of DNA polymerase KOD plus (TOYOBO)) were prepared and for the first PCR was performed in the conditions of heating at 94°C for 3 minutes, followed by 7 cycles of reactions at 94°C for 1 minutes and 63°C for 4 minutes. The reaction solution was maintained at 63°C and then 2.5 µL each of 10 µM scfor primer and 10 µM scback primer was added to each tube, and further the second PCR (heating at 94°C for 35 seconds, followed by 30 cycles of reactions at 94°C for 2 minutes and 63°C for 2 minutes) was carried out. After performing PCR, the reaction solution was purified by QIAquick PCR purification kit (QIAGEN), and the purified products were digested with restriction enzyme Sfi I (TAKARA) at 50°C overnight. The digestion products were subjected to 2% agarose gel electrophoresis, and the amplified fragments of the desired size (about 800 bp) were purified using QIAquick Gel Extraction Kit (QIAGEN) by the method described in the attached instruction manual and then eluted with an appropriate amount of sterilized water. For the presentation of scFv on phage gene III protein, pELBGlacI (see Fig. 11) was used as a phagemid vector. After 10 µg of the vector was digested with restriction enzyme Sfi I (TAKARA) at 50°C overnight, the digested fragments of the desired size (about 5 kb) were purified using QIAquick Gel Extraction Kit (QIAGEN) by the method described in the attached instruction manual and eluted with an appropriate amount of sterilized water. The purified PCR products and the purified vector fragments were ligated at 16°C overnight using Ligation High (TOYOBO) according to the method described in the attached instruction manual. The resultant solution was used to transform *E. coli* XL1blue electrocompetent cells (Stratagene) or electromax DH12s (Invitrogen) by an electroporation method according to the method described in the attached instruction manual. All of the obtained ampicillin resistant transformants were collected and stored at -20°C until use as a recombinant *E. coli* library.

The *E. coli* library (2 x 10⁹ cfu) was plated in 50 mL of 2x YTAG (2x TY containing 100 µg/mL ampicillin and 2% glucose) and cultured at 37°C until OD 600 value reached 0.4 to 0.5. Helper phage VCSM13 (Stratagene) (4 x 10¹¹) was added left to stand at 37°C for 15 minutes for infection. To this, 450 mL of 2x YTAK (2x TY containing 100 µg/mL ampicillin and 25 µg/mL kanamycin) and 25 µL of IPTG (1 mol/L) were added, and cultured at 30°C for 10 hours. The culture supernatant was collected by centrifugation and mixed with 100 mL of PEG-NaCl solution (10% polyethylene glycol 8000, 2.5 mol/L of NaCl), and then left to stand at 4°C for 60 minutes. The phages were precipitated by centrifugation at 10,800x g for 30 minutes and the precipitates were suspended in 40 mL of water. This was mixed with 8 mL of PEG-NaCl solution and then left to stand at 4°C for one hour. The phages were precipitated by centrifugation at 10,800x g for 30 minutes and then suspended in 5 mL of PBS to obtain a phage library. The phage library was then preserved at 4°C until use.

### 10-3. Binding phage concentration by panning method

Factor EXaβ or Factor X was biotinylated using No-Weigh Premeasured NHS-PEO₄-Biotin Microtubes (Pierce). 100 pmol of the biotinylated Factor IXaβ or Factor X was added to 600 µL of the phage library solution prepared in Example 10-2, and was contacted with the antigen for 60 minutes. 600 µL of Dynabeads M-280 Streptavidin (DYNAL) washed with 5% M-PBS (PBS containing 5% w/v skimmed milk) was added and the binding reaction was performed for 15 minutes. After bead-binding phages were washed several times with 1 mL of PBST (PBS containing 0.1% Tween-20), they were washed with PBS. The beads were suspended in 0.8 mL of glycine/HCl (0.1 mol/L, pH 2.2) for 5 minutes and the phages were eluted.

Alternatively, phage library (80 µL/well x 5) incubated for 15 minutes with 2.5% w/v skimmed milk was added to Factor IXaβ or Factor X (10 µg/well x 5) immobilized on Immunoplate (MaxiSorp, Nunc), and contacted with the antigen for 60 minutes. Antigen-binding phages were washed several times with 1 mL of PBST, and then washed with PBS. They were suspended in 0.8 mL of glycine/HCl (0.1 mol/L, pH 2.2) for 5 minutes and the phages were eluted.

The collected phage solution was neutralized by adding 45 µL of 2 mol/L Tris. It was then added to 10 mL of XL1-Blue in logarithmic growth phase (OD 600 = 0.4 to 0.5) and left to stand at 37°C for 30 minutes to infect the cells. This was plated on 2x YTAG plates, and cultured at 30°C. The colonies were collected, inoculated into 2x YTAG, and cultured at 37°C until OD 600 reached 0.4 to 0.5. 5 µL of IPTG (1 mol/L) and 1 x 10¹¹ pfu of helper phage (VCSM13) were added to 10 mL of the culture, and left to stand at 37°C for 30 minutes. After the cells were collected by centrifugation, they were resuspended in 100 mL of 2x YTAK, and cultured at 30°C for 10 hours. The culture supernatant was collected by centrifugation, mixed with 20 mL of 10% PEG-5 mol/L NaCl solution, and left to stand at 4°C for 20 minutes. The phages were precipitated by centrifugation at 10,800x g for 30 minutes. The precipitate was suspended in 2 mL of PBS and this was used for the subsequent panning.

### 10-4. Phage ELISA

The above-described single colonies were inoculated in 100 µL of 2x YTAG and cultured at 30°C overnight. After 5 µL of the culture was inoculated in 500 µL of 2x YTAG and cultured at 37°C for 5 hours, 2 x 10⁸ pfu of helper phage was added and left to stand at 37°C for 30 minutes. Furthermore, this was cultured with shaking at 37°C for 30 minutes, and then 120 µL of 2x YTAK containing 0.5 mM IPTG was added to it. This was cultured at 30°C overnight and the supernatant after centrifugation was subjected to ELISA. In order to perform ELISA for the clones obtained by panning of the biotinylated antigen, StreptaWell 96 microtiter plate (Roche) coated using 1.0 µg/mL of biotinylated antigen was used. Moreover, to carry out ELISA for the clones obtained by panning of a native antigen, Immunoplate (MaxiSorp, Nunc) to which 1.0 µg/mL of the native antigen was immobilized was used. After the plates were washed with PBST to remove the antigens, blocking was carried out at room temperature for one hour using 200 µL of 2% M-PBS or 2% BSA-PBS (PBS containing 2% w/v BSA) as a blocking buffer. The buffer was removed, and the culture supernatant was added thereto and left to stand for 60 minutes to bind the phages. After the plates were washed, the binding phages were detected by HRP-conjugated anti-M13 antibody (Amersham Pharmacia Biotech) and TMB substrate (Zymed). The reaction was stopped by adding 1 mol/L of H₂SO₄. The A450 was then measured using a plate reader.

### 10-5. Sequencing and clone selection

Recombinant *E. coli* 2x YTAG cultures of positive clones in ELISA were used to amplify scFv region by PCR using primers of PBG3-F1 (5'-CAGCTATGAAATACCTATTGCC-3'/SEQ ID NO: 38) and PBG3-R1 (5'-CTTTTCATAATCAAAATCACCGG-3'/ SEQ ID NO: 39) and its nucleotide sequence was determined. 1 µL of the culture, 1.5 µL of 10x KOD Dash buffer, 0.2 µL each of 10 µmol/L primers, and 15 µL of PCR reaction solution containing 0.3 µL of KOD Dash polymerase (2.5 U/ L, TOYOBO) were used for amplification by 30 cycles of reactions at 96°C for 10 seconds, 55°C for 10 seconds, and 72°C for 30 seconds using Thermal Cycler GeneAmp PCR system 9700 (Perkin Elmer). After performing PCR, 3 µL of ExoSAP-IT (Amersham) was added to 5 µL of the reaction solution, and maintained at 37°C for 15 minutes and subsequently at 80°C for 15 minutes. This sample was used for PCR utilizing PBG3-F2 (5'-ATTGCCTACGGCAGCCGCT-3'/SEQ ID NO: 40) or PBG3-R2 (5'-AAATCACCGGAACCAGAGCC-3'/SEQ ID NO: 41) as a primer, with BigDye Terminator Cycle Sequencing kit (Applied Biosystems), and the products were subjected to electrophoresis with Applied Biosystems PRISM 3700 DNA Sequencer. 52 clones, each having a different amino acid sequence of CDR3 deduced from the nucleotide sequence, were selected for anti-Factor IXa, and 33 clones were selected for anti-Factor X.

### 10-6. Construction of bispecific IgG antibody expression vectors

For expressing scFv antibody as IgG type, antibody variable regions (VH, VL) were cloned into inducible expression vectors by a method similar to Examples 3-3, 3-4, and 3-5. Anti-F. IXa antibody variable regions (VH and VL) were incorporated into tetracycline-inducible vectors (pcDNA4-g4H and pcDNA4-g4L, respectively). Anti-F. X antibody variable regions (VH and VL) were incorporated into ecdysone analogue-inducible vectors (pIND-g4H and pcDNA4-g4L, respectively). The respective plasmid DNAs were isolated from the desired clones using QIAprep Spin Miniprep Kit (QIAGEN) and dissolved into 100 µL of sterilized water.

### 10-7. Expression of chimeric bispecific antibodies in animal cells

Using DNA solutions prepared by a method similar to that described in Example 4-1, the antibodies were expressed in animal cells by a method similar to that described in Examples 4-2 and 4-3, and the culture supernatants were collected. The samples were then stored at 4°C until use.

### [Example 11] Antibody purification

100 µL of rProtein A Sepharose™ Fast Flow (Amersham Biosciences) was added to 10 mL of the culture supernatants obtained in Example 10-7, and they were mixed by inversion overnight at 4°C. The solutions were transferred to 0.22 µm filter cup Ultrafree® -MC (Millipore) and washed three times with 500 µL of TBS containing 0.01% Tween® 20. rProtein A Sepharose™ resin was suspended in 10 mM HCl (pH 2.0) containing 100 µL of 0.01% Tween® 20 and left to stand for 3 minutes, after which the antibodies were eluted. Immediately after this, 5 µL of 1M Tris-HCl, pH 8.0 was added for neutralization. Human IgG concentration in the culture supernatants were calculated from the standard curve of human IgG4 (humanized anti-TF antibody, see WO 99/51743) using Microplate Manager III (Bio-Rad Laboratories) software. The antibody concentrations were determined according to Example 5.

### [Example 12] F. VIIIa -like activity assay

F. VIIIa-like activities of the bispecific antibodies were evaluated by the following enzyme assay. Moreover, all of the following reactions were carried out at room temperature. The mixture of 10 µL of 15 µg/mL Factor IX (Enzyme Research Laboratories), 5 µL of TBSB containing 100 mM CaCl₂ and 20 mM MgCl₂, and 50 µL of a culture supernatant obtained by the method described in Example 10-7 was incubated for one hour in a 96-well plate. Furthermore, 10 µL of 10 ng/mL Factor XIa (Enzyme Research Laboratories), 20 µL of 50 µg/mL Factor X (Enzyme Research Laboratories), and 5 µL of 400 µg/mL phospholipid were added to the mixture for initiating the enzyme reaction. After performing the reaction for 30 minutes, it was stopped by adding 10 µL of 0.5M EDTA.

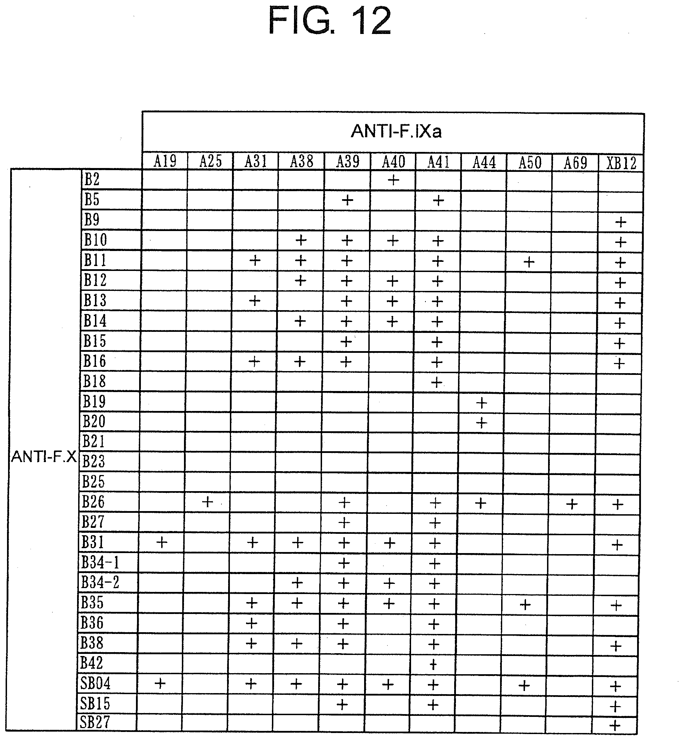

After 50 µL of colorimetric substrate solution was added to each well, the absorbance at 405 nm (reference wavelength, 655 nm) at 0 minute and 60 minutes was measured using Model 3550 Microplate Reader (Bio-Rad Laboratories). F. VIIIa-like activities were represented by the values in which the absorbance change value for 60 minutes of a culture supernatant expressing no antibody was subtracted from that of an antibody-expressing culture supernatant (see Fig. 12).

For a solvent of phospholipid, Factor XIa, Factor IX and Factor X, TBSB was used. The colorimetric substrate solution is the mixture of "tesutochimu" colorimetric substrate S-2222 (Chromogenix) which was dissolved according to the attached instruction manual and polybrene solution (0.6 mg/L hexadimethrine bromide (Sigma)) at the ratio of 1:1.

### [Example 13] Plasma coagulation assay

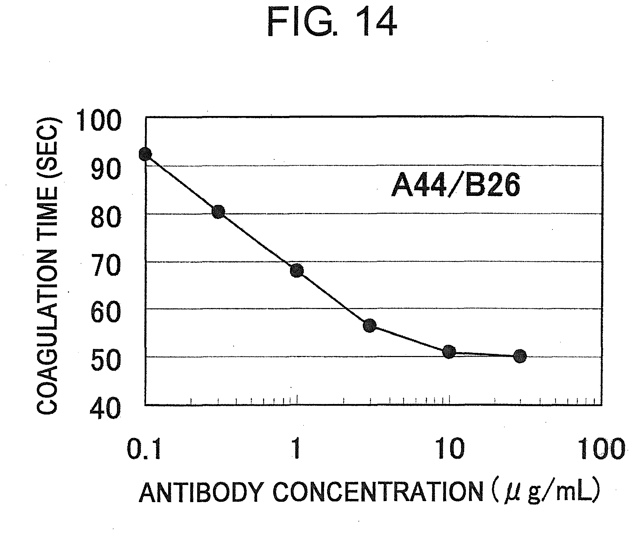

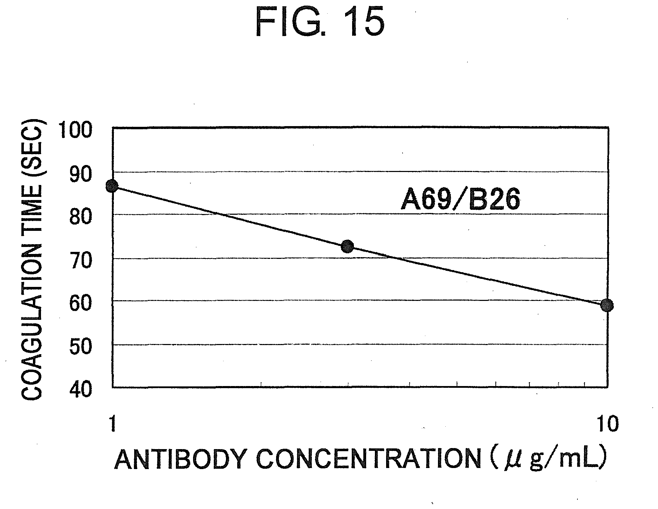

To determine whether or not the bispecific antibodies purified in Example 11 recovered the coagulation ability of the blood of hemophilia A, the effects of these antibodies on the activated partial thromboplastin time (APTT) using F. VIII deficient plasma were evaluated by the method similar to that described in Example 7 (see Fig. 13). Furthermore, the concentration dependency was measured for A44/B26 and A69/B26, which exhibited great coagulation time-reducing effect (see Figs. 14 and 15).

### [Example 14] Consideration of combined use of a bispecific IgG antibody and F. VIII

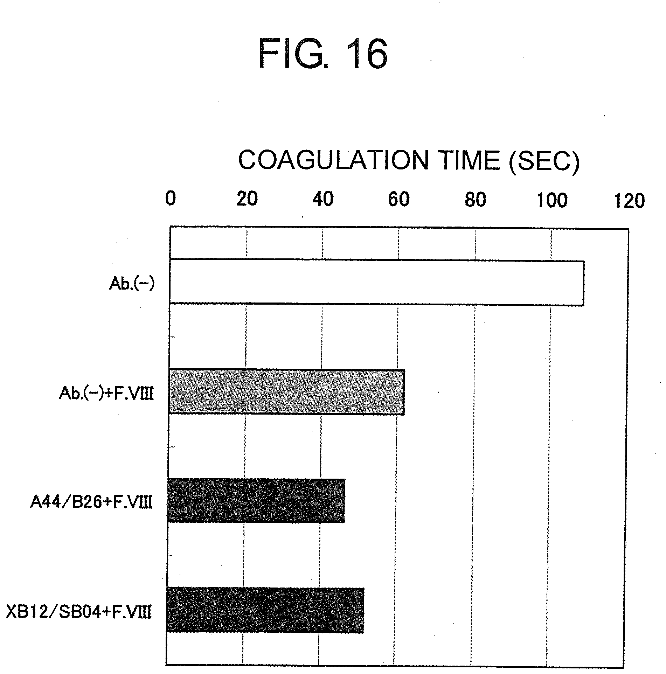

Combined use of a bispecific IgG antibody and F. VIII was considered using the following plasma coagulation assay. The mixture of 40 µL of an antibody solution (25 µg/mL) and 50 µL of F.VIII deficient plasma (Biomerieux) was incubated at room temperature for 30 minutes. Furthermore, to the mixture, 10 µL of recombinant coagulation factor VIII preparation Kogenate® FS (1 U/mL, BAYER) and 50 µL of APTT reagent (Dade Behring) were added, and it was warmed at 37°C for 3 minutes. The coagulation reaction was initiated by adding 50 µL of 20 mM CaCl₂ (Dade Behring) to the mixture described above. The time period until coagulation was occurred was measured using KC10A(Amelung) connected to CR-A (Amelung) (see Fig. 16).

### [Example 15] The effect of bispecific IgG antibodies on inhibitor plasma

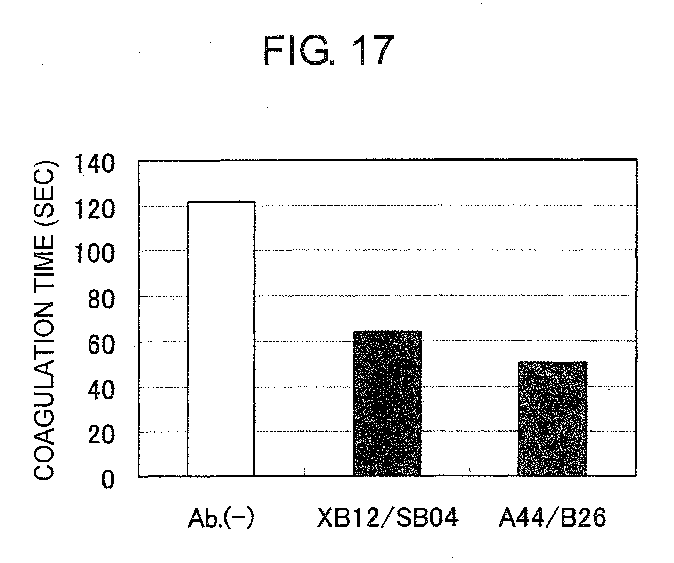

The effect of bispecific IgG antibodies on inhibitor plasma was evaluated by the following plasma coagulation assay. The mixture of 50 µL of F. VIII deficient plasma (Biomerieux) and 10 µL of anti-human F. VIII neutralizing antibody (100 µg/mL, Catalog Number: MAB3440, CHEMICON) was incubated at room temperature for 30 minutes. This was used as inhibitor plasma. 40 µL of antibody solution (25 µg/mL) and 50 µL of APTT reagent (Dade Behring) was added thereto, and the mixture was warmed at 37°C for 3 minutes. The coagulation reaction was initiated by adding 50 µL of 20 mM CaCl₂ (Dade Behring) to the mixture described above. The time period until coagulation occurred was measured using KC10A (Amelung) to which CR-A (Amelung) was connected (see Fig. 17).

### [Example 16] Humanization of bispecific antibodies

Among the bispecific antibodies obtained in Examples 1-7, XB12 (mouse anti-Factor IXa antibody)/SB04 (mouse anti-Factor X antibody) which exhibited the highest blood coagulation time-reducing effect, was humanized as follows.

### 16-1. Homology search of human antibody

Human antibody amino acid sequence data was obtained from Kabat Database (ftp://ftp.ebi.ac.uk/pub/databases/kabat/) and IMGT Database (http://imgt.cines.fr/), both of which are publicly available, and the constructed database was used to search homology in mouse XB12-H chain variable region, mouse XB12-L chain variable region, mouse SB04-H chain variable region, and mouse SB04-L chain variable region, separately. As a result, since the high homologies to the following human antibody sequences were confirmed, they were used as framework regions (hereinafter, abbreviated as FRs) of a humanized antibody.
(1) XB12-H chain variable region: KABATID-020619 (Kabat Database) (Mariette et al., Arthritis Rheum. 1993; 36:1315-1324)
(2) XB12-L chain variable region: EMBL Accession No. X61642 (IMGT Database) (Mark et al., J Mol Biol. 1991; 222: 581-597)
(3) SB04-H chain variable region: KABATID-025255 (Kabat Database) (Demaison et al., Immunogenetics 1995; 42: 342-352).
(4) SB04-L chain variable region: EMBL Accession No. AB064111 (IMGT Database) (Unpublished data)

A humanized antibody in which complementarity-determining regions of the respective mouse antibodies were implanted into human antibody FRs of (1)-(4) was then prepared.

Moreover, using the homology search Web site (http://www.ncbi.nlm.nih.gov/BLAST/), which is also publicly available through NCBI, secretory signal sequences of human antibody highly homologous to human antibodies of (1)-(4) were searched. The following secretory signal sequences were obtained and used for the subsequent procedures.
(1) XB12-H chain variable region: GenBank Accession No. AF062120
(2) XB12-L chain variable region: GenBank Accession No. M74019
(3) SB04-H chain variable region: GenBank Accession No. BC019337
(4) SB04-L chain variable region: GenBank Accession No. AY204756

### 16-2. Construction of humanized antibody gene expression vectors

For the nucleotide sequence encoding an amino acid sequence from secretory signal sequence to antibody variable region, 12 oligo DNAs of about 50 bases were alternately prepared such that about 20 bases at the 3' side hybridized thereto. Furthermore, a primer which hybridizes to the 5' side of the antibody variable region gene and comprises Xho I cleavage sequence, and a primer which hybridizes to the 3' side of the antibody variable region gene and comprises Sfi I cleavage sequence were prepared.

The respective 1 µL of synthesized oligo DNAs (2.5 µM) were mixed, 1x TaKaRa Ex Taq Buffer, 0.4 mM dNTPs, and 0.5 units of TaKaRa Ex Taq (all of these, obtained from TAKARA) were added, and prepared so that the reaction solution became 48 µL. After heating at 94°C for 5 minutes, two cycles of reactions at 94°C for 2 minutes, 55°C for 2 minutes, and 72°C for 2 minutes were carried out, assembly and elongation reaction of the respective synthesized oligo DNAs were carried out. Next, 1 µL of primers (each 10 µM) which was hybridized to 5' side or 3' side of the antibody gene were added, 35 cycles of reactions at 94°C for 30 seconds, 55°C for 30 seconds, and 72°C for one minute were carried out, and reacted at 72°C for 5 minutes to amplify the antibody variable region gene. After PCR was carried out, the reaction solution was subjected to 1% agarose gel electrophoresis. The amplified fragments of the desired size (about 400 bp) were purified using QIAquick Gel Extraction Kit (QIAGEN) by the method described in the attached instruction manual and eluted using 30 µL of sterilized water. The fragments were cloned using pGEM-T Easy Vector Systems (Promega) by the method described in the attached instruction manual. The nucleotide sequences of the respective DNA fragments were sequenced using BigDye Terminator Cycle Sequencing Kit (Applied Biosystems) with DNA sequencer ABI PRISM 3700 DNA Sequencer (Applied Biosystems) according to the method described in the attached instruction manual.

After the plasmids, confirmed to comprise proper humanized antibody variable region gene sequences, were digested with Xho I and Sfi I, the reaction solutions were subjected to 1% agarose gel electrophoresis. DNA fragments having the desired size (about 400 bp) were purified using QIAquick Gel Extraction Kit (QIAGEN) by the method described in the attached instruction manual and eluted using 30 µL of sterilized water. Moreover, tetracycline-inducible expression plasmids (pcDNA4-g4H, pcDNA4-g4L) and ecdysone analogue-inducible expression plasmids (pIND-g4H, pIND-g4L), which were prepared in Example 3-4 and digested with Xho I and Sfi I, fragments (about 5 kb) comprising an antibody constant region, were purified using QIAquick Gel Extraction Kit (QIAGEN) by the method described in the attached instruction manual and eluted using 30 µL of the sterilized water. The humanized XB12 antibody gene fragments, which had been digested with Xho I and Sfi I (H chain variable region (hereinafter, abbreviated as VH)) or L chain variable region (hereinafter, abbreviated as VL), and the tetracycline-inducible expression plasmids, which had been digested with Xho I and Sif I (pcDNA4-g4H and pcDNA4-g4L), were ligated using Rapid DNA Ligation Kit (Roche Diagnostics) by the method described in the attached instruction manual. Furthermore, the humanized SB04 antibody gene fragments, which had been digested with Xho I and Sfi I (VH or VL), and ecdysone analogue -inducible expression plasmids, which had been digested with Xho I and Sif I (pIND-g4H and pIND-g4L), were ligated using Rapid DNA Ligation Kit (Roche Diagnostics) by the method described in the attached instruction manual. A portions of each reaction solution was used to transform *E. coli* DH5 α strain (TOYOBO).

### 16-3. Preparation of humanized bispecific antibody

Using 4 kinds of humanized antibody expression vector, pcDNA6/TR, and pVgRXR, the gene introduction and induced expression in HEK293H were performed by the method described in Examples 4-2 and 4-3. Furthermore, the antibody was purified and its concentration was determined by the method described in Examples 8 and 5.

### 16-4. Activity evaluation of humanized bispecific antibody and modification of antibody sequence

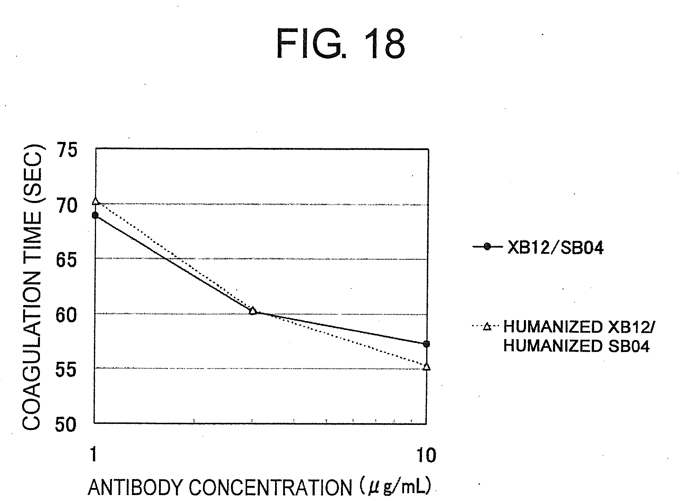

To evaluate the plasma coagulation ability of the prepared humanized bispecific antibody and chimeric bispecific antibody (XB12/SB04), the effects of the antibodies on APTT were examined using F. VIII deficient plasma according to the method of Example 7. For the humanized bispecific antibody whose blood coagulation ability was decreased, the amino acids of human antibody FRs were modified aiming at increasing the activity. Moreover, cysteine residues of CDR3 of XB12 antibody VH which may cause the decrease in thermostability and such were modified to alanine residues. Specifically, the mutations were introduced into the humanized antibody expression vectors using QuikChange Site-Directed Mutagenesis Kit (Stratagene) by the method described in the attached instruction manual. A humanized bispecific antibody (humanized XB12 antibody (VH:hXB12f-A, VL: hXBVL))/humanized SB04 antibody (VH: hSBo4e, VL: hSBVL-F3f) having a blood coagulation activity equivalent to that of XB12/SB04 was obtained by repeating the amino acid modifications in FR sequences and the activity evaluation (see Fig. 18).

### [Example 17] Construction of bispecific IgG4 antibody H chain expression vectors

Furthermore, the bispecific antibodies of A44 and B26 using an L chain which had been CDR shuffled were expressed.

pCAGGss-g4CH vector was constructed in which downstream of CAGG promoter, an animal cell signal sequence and intron immediately before human IgG1CH1 sandwiched two Sfi I sites, and further downstream of them, human IgG4 constant region cDNA existed. An expression vector for animal cells to secrete as IgG4H chain can be constructed by inserting between the Sfi I sites VH gene which was sandwiched by signal sequence processing site and splicing donor sequence. Furthermore, in order to preferentially express IgG4 which has H chains of heterozygous combination, amino acid substitution products for CH3 of IgG4 were used with reference to the knobs-into-holes technique in IgG1 (Protein Engineering Vol.9, 617-621, 1996). Type a is a substitution product of Y349C or T366W, and type b is a substitution product of E356C, T366S, L368A, or Y407V. Furthermore, an amino acid substitution (-ppcpScp- → -ppcpPcp-) was also introduced into the hinge region to promote the dimmer formation of H chains. Regarding signal sequence, mouse IL-3 and human IL-6 was used for type a and type b, respectively (pCAGG-IL3ss-g4CHPa, pCAGG-IL6ss-g4CHPb). The VH fragment of antibody A44 obtained in the above-described Example was inserted into Sfi I site of pCAGG-IL3ss-g4CHPa to obtain pCAGG-chiA44-g4a and the VH fragment of antibody A69 was inserted into Sfi I site of pCAGG-IL3 ss-g4CHPa to obtain pCAGG-chiA69-g4a. In addition, pCAGG-chiB26-g4b was obtained by similarly inserting the VH fragment of antibody B26 into Sfi I site of pCAGG-IL6ss-g4CHPb.

### [Example 18] Construction of CDR exchange L chain expression vector

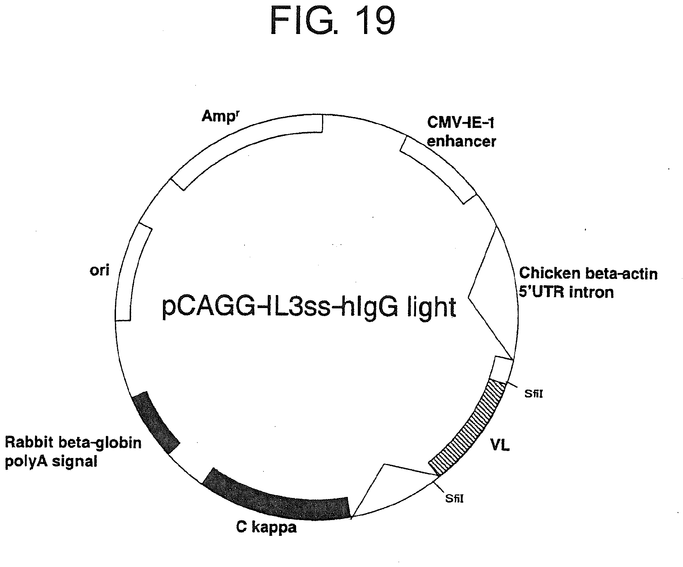

pCAGG-κ (pCAGG-IL3ss-hIgG light) vector was constructed in which downstream of CAGG promoter, mouse IL-3 signal sequence and intron immediately before human κ constant region sandwiched two Sfi I sites, and further downstream of them, human κ chain constant region (CL) exon existed (see Fig. 19). An expression vector for animal cells to secrete as κ chain can be constructed by inserting between the Sfi I sites VL gene sandwiched by signal sequence processing site and splicing donor sequence.

In order to synthesize DNA encoding L chain variable region in which frameworks and CDRs of A44 antibody L chain and CDRs of A50, A69, and B26 antibody L chain were combined, synthetic oligo DNAs having about 60 bases were alternately prepared so that about 20 bases at their termini hybridized thereto. Furthermore, a primer scback which comprises a signal sequence processing site and Sfi I site and hybridizes to the 5' side of VL gene was prepared, and a primer scfor which comprises a splicing donor sequence and Sfi I site and hybridizes to the 3' side of VL gene.
A44LR2 (SEQ ID NO: 53)
   GAAGCGATCAGGGACTCCAGTGTGCCGGGTGGATGCCCAGTAAATCAGTAGTTTAGG
A44LF3 (SEQ ID NO: 54)
   GGAGTCCCTGATCGCTTCACAGGCAGTAGATATGGGACAGATTTCACTCTCACCATT
A44LR3 (SEQ ID NO: 55)
   ACAGAGATAATCTGCCAGGTCTTCAGACTGCACATTGCTAATGGTGAGAGTGAAATC
A44LF4 (SEQ ID NO: 56)
   CTGGCAGATTATCTCTGTCAGCAATATAGCAACTATATCACGTTCGGTGGTGGGACC
B26LR2_A44fr (SEQ ID NO: 60)
   GAAGCGATCAGGGACTCCACTGTACCGGTAGGATGCCGAGTAAATCAGTAGTTTAGG
A50LF4_A44fr (SEQ ID NO: 63)
   CTGGCAGATTATCTCTGTCAGCAATATAGCAGCTATTTAACGTTCGGTGGTGGGACC
scback (SEQ ID NO: 64)
   TTACTCGCGGCCCAGCCGGCCATGGCGGACTACAAAG
scfor (SEQ ID NO: 65)
   GGAATTCGGCCCCCGAG

1 µL each of the synthesized oligo DNAs prepared at 10 µM was mixed in the combination shown in Table 1, and 45 µL of the reaction solutions comprising 1x enzyme added buffer, 0.33 mM dNTPs, 2.5 unit of Proof Start Polymerase (Qiagen) or LATaq (TAKARA) were prepared. After heating at 94°C for 5 minutes, 7 cycles of reactions at 94°C for one minute and 63 °C for 4 minutes were carried out, subsequently 5 µL each of 5 µM scback and 5 µM scfor solutions was added, and 30 cycles of reactions at 94°C for 30 seconds, 55°C for 30 seconds, and 72°C for 30 seconds were performed to amplify the VL gene.

**Table 1**

| | | | | | | | | |
|---|---|---|---|---|---|---|---|---|
| BBA : | B26LR1_A44fr | B26LR2_A44fr | A44LF4 | A44LF1 | A44LF2 | A44LF3 | A44LR3 | A44LR4 |
| BAA : | B26LR1_A44fr | A44LR2 | A44LF4 | A44LF1 | A44LF2 | A44LF3 | A44LR3 | A44LR4 |
| ABA : | A44LR1 | B26LR2_A44fr | A44LF4 | A44LF1 | A44LF2 | A44LF3 | A44LR3 | A44LR4 |
| AAA : | A44LR1 | A44LR2 | A44LF4 | A44LF1 | A44LF2 | A44LF3 | A44LR3 | A44LR4 |
| AAa : | A44LR1 | A44LR2 | A50LF4_A44fr | A44LF1 | A44LF2 | A44LF3 | A44LR3 | A44LR4 |
| BAa : | B26LR1_A44fr | A44LR2 | A50LF4_A44fr | A44LF1 | A44LF2 | A44LF3 | A44LR3 | A44LR4 |
| ABa : | A44LR1 | B26LR2_A44fr | A50LF4_A44fr | A44LF1 | A44LF2 | A44LF3 | A44LR3 | A44LR4 |
| BBa : | B26LR1_A44fr | B26LR2_A44fr | A50LF4_A44fr | A44LF1 | A44LF2 | A44LF3 | A44LR3 | A44LR4 |
| aAA : | A69LR1_A44fr | A44LR2 | A44LF4 | A44LF1 | A44LF2 | A44LF3 | A44LR3 | A44LR4 |
| aBA : | A69LR1_A44fr | B26LR2_A44fr | A44LF4 | A44LF1 | A44LF2 | A44LF3 | A44LR3 | A44LR4 |
| BBA(G) : | B26LR1_A44fr | B26LR2_A44fr | A44LF4 | A44LF1 | A44LF2 | A44LF3 | A44LR3 | A44LR4Gly |
| BAA(G) : | B26LR1_A44fr | A44LR2 | A44LF4 | A44LF1 | A44LF2 | A44LF3 | A44LR3 | A44LR4Gly |
| ABA (G) : | A44LR1 | B26LR2_A44fr | A44LF4 | A44LF1 | A44LF2 | A44LF3 | A44LR3 | A44LR4Gly |
| AAA (G) : | A44LR1 | A44LR2 | A44LF4 | A44LF1 | A44LF2 | A44LF3 | A44LR3 | A44LR4Gly |
| AAa (G) : | A44LR1 | A44LR2 | A50LF4_A44fr | A44LF1 | A44LF2 | A44LF3 | A44LR3 | A44LR4Gly |
| Baa (G) : | B26LR1_A44fr | A44LR2 | A50LF4_A44fr | A44LF1 | A44LF2 | A44LF3 | A44LR3 | A44LR4Gly |
| ABa (G) : | A44LR1 | B26LR2_A44fr | A50LF4_A44fr | A44LF1 | A44LF2 | A44LF3 | A44LR3 | A44LR4Gly |
| BBa (G) : | B26LR1_A44fr | B26LR2_A44fr | A50LF4_A44fr | A44LF1 | A44LF2 | A44LF3 | A44LR3 | A44LR4Gly |
| aAA (G) : | A69LR1_A44fr | A44LR2 | A44LF4 | A44LF1 | A44LF2 | A44LF3 | A44LR3 | A44LR4Gly |
| aBA (G) : | A69LR1_A44fr | B26LR2_A44fr | A44LF4 | A44LF1 | A44LF2 | A44LF3 | A44LR3 | A44LR4Gly |

After performing PCR, the products were purified from the total reaction solutions using QIAquick PCR Purification Kit (Qiagen) by the method described in the attached instruction manual, and eluted using sterilized water. After the fragments were digested with restriction enzyme Sfi I (TOYOBO), they were subjected to 2% agarose gel electrophoresis. The amplification fragments having about 0.4 kb were purified using QIAquick Gel Extraction Kit (Qiagen) by the method described in the attached instruction manual, and eluted using sterilized water. The obtained fragments were ligated using Ligation High (TOYOBO) with the above-described L chain expression vector pCAGG-κ which had been digested with Sfi I. Portion of each reaction solution was used to transform *E. coli* DH5 α strain (TOYOBO). The nucleotide sequences were determined and confirmed using BigDye Terminator Cycle Sequencing Kit (Applied Biosystems) with DNA sequencer ABI PRISM 3700 DNA Sequencer (Applied Biosystems) according to the method described in the attached instruction manual. pCAGG-A44BBA was obtained by inserting BBA fragment and other expression vectors were obtained similarly by inserting other VL fragments. The respective antibody variable region sequences are described in the following SEQ ID NOs.

**Table 2**

| | | Nucleotide SEQ ID NO: | Amino acid SEQ ID NO: |
|---|---|---|---|
| **(1) AAA** | **(pCAGG-A44L)** | **66** | **67** |
| **(2) BBA** | **(pCAGG-A44BBA)** | **68** | **69** |
| **(3) BAA** | **(pCAGG-A44BAA** | **70** | **71** |
| **(4) ABA** | **(pCAGG-A44ABA)** | **72** | **73** |
| **(5) AAa** | **(pCAGG-A44AAa)** | **74** | **75** |
| **(6) BAa** | **(pCAGG-A44BAa)** | **76** | **77** |
| **(7) ABa** | **(pCAGG-A44ABa)** | **78** | **79** |
| **(8) BBa** | **(pCAGG-A44BBa)** | **80** | **81** |
| **(9) aAA** | **(pCAGG-A44aAA)** | **82** | **83** |
| **(10) aBA** | **(pCAGG-A44aBA)** | **84** | **85** |
| **(11) AAA (G)** | **(pCAGG-A44LG)** | **86** | **87** |
| **(12) BBA (G)** | **(pCAGG-A44BBAG)** | **88** | **89** |
| **(13) BAA (G)** | **(pCAGG-A44BAAG)** | **90** | **91** |
| **(14) ABA (G)** | **(pCAGG-A44ABAG)** | **92** | **93** |
| **(15) AAa (G)** | **(pCAGG-A44AAaG** | **94** | **95** |
| **(16) BAa (G)** | **(pCAGG-A44BAaG)** | **96** | **97** |
| **(17) ABa (G)** | **(pCAGG-A44ABaG)** | **98** | **99** |
| **(18) BBa (G)** | **(pCAGG-A44BBaG)** | **100** | **101** |
| **(19) aAA (G)** | **(pCAGG-A44aAAG)** | **102** | **103** |
| **(20) aBA (G)** | **(pCAGG-A44aBAG)** | **104** | **105** |

### [Example 19] Preparation of antibodies

HEK293 strain cells derived from human fetal renal carcinoma cells were suspended in DMEM medium (Invitrogen) containing 10% FCS (Moregate), 6 x 10⁶ cells were plated in 10 cm diameter dishes for adherent cells (Coming) and cultured in a CO₂ incubator (37°C, 5% CO₂) overnight. Any of the L chain expression vectors of Example 18, two kinds of H chain expression vector (30 µg) of pCAGG-chiB26-g4b and pCAGG-chiA44-g4a or pCAGG-chiA69-g4a of Example 17, and 1.5 mL of OPTI-MEMI medium were added to the mixture of 60 µL of transfection reagent Lipofectamine 2000 (Invitrogen) and 1.5 mL of Opti-MEM I medium (Invitrogen) and left to stand at room temperature for 20 minutes, and the resulting mixture was added to the dishes and cultured for 3 days in a CO₂ incubator (37°C, 5% CO₂), To the obtained culture supernatant, 100 µL of rProtein A Sepharose™ Fast Flow (Amersham Biosciences) was added and mixed by inversion at 4°C overnight. The resin was precipitated by centrifugation and washed with TBS containing 0.01% Tween® 20 three times. Subsequently, the resin was suspended in 10 mM HCl, 150 mM NaCl, pH 2.0 containing 100 µL of 0.01% Tween® 20 and left to stand for 3 minutes, and then the antibody was eluted. Immediately after the elution, 5 µL of 1M Tris-HCl, 150 mM NaCl, pH 8.0 was added and neutralized.

### [Example 20] Quantitative determination of IgG concentration

Goat affinity purified antibody to human IgG Fc (Cappel) was prepared to the concentration of 1 µg/mL with PBS, and immobilized to a Nunc-Immuno plate. After the plate was blocked with PBS containing 2% BSA, the culture supernatant sample, appropriately diluted using this buffer, was added. Moreover, as the standard for calculating antibody concentration, a two-fold dilution series of human IgG4 (humanized anti-TF antibody, see WO 99/51743) which was produced by a 11-step dilution from the concentration of 1 µg/mL with DB was similarly added. After washing three times, goat anti-human IgG. alkaline phosphatase (Biosource International) was reacted. After washing five times, Sigma 104® phosphatase substrate (Sigma-Aldrich) was used as a substrate, and the absorbance at 405 nm with reference wavelength of 655 nm was measured using absorbance reader SUNRISE RAINBOW (TECAN). Human IgG concentration in the culture supernatant was calculated from the standard curve using LS-PLATE manager 2001 (TECAN) software.

### [Example 21] Plasma coagulation assay

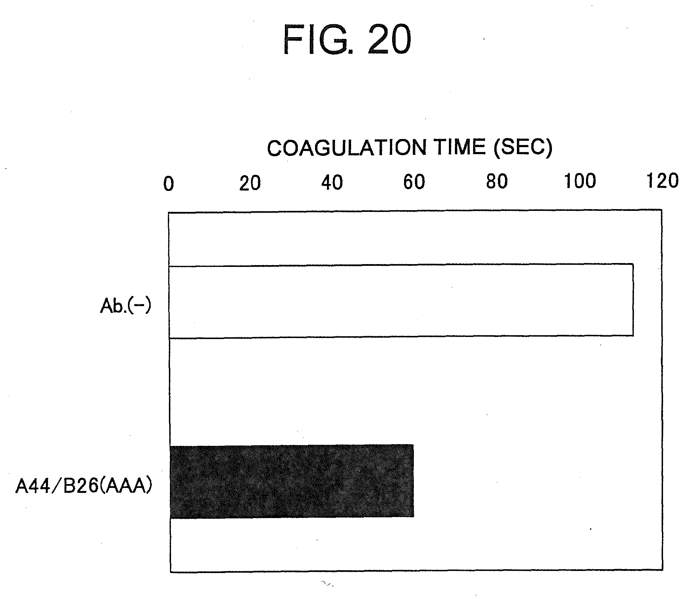

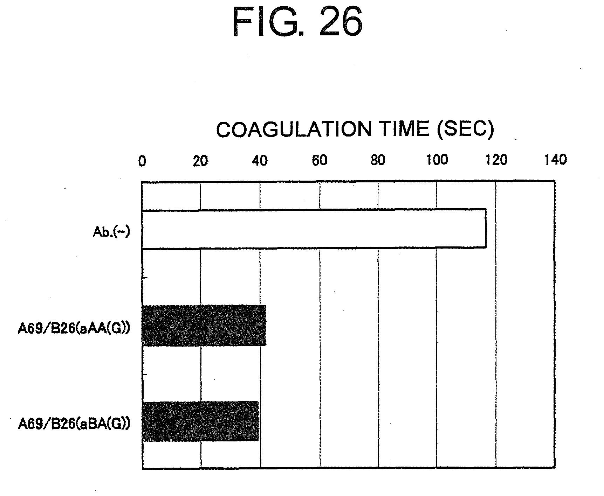

To determine whether or not a bispecific antibody of the present invention was capable of correcting the coagulation ability of the blood of hemophilia A, the influence of the same antibody with respect to the activated partial thromboplastin time (APTT) using F. VIII deficient plasma was considered. The mixture of 50 µL of an antibody solution having a variety of concentrations, 50 µL of F. VIII deficient plasma (Biomerieux) and 50 µL of APTT reagent (Dade Behring) were warmed at 37°C for 3 minutes. The coagulation reaction was initiated by adding 50 µL of 20 mM CaCl₂ (Dade Behring) to the same mixture as described above. The time period until coagulation was measured by KC10A (Amelung) to which CR-A (Amelung) has been connected (Figs. 20 and 26). The results demonstrated that the bispecific antibody shortened the coagulation time as compared to the case where antibody was not added.

### [Example 22] Humanization of a bispecific antibody comprising hybrid L chains

Humanization was carried out as follows on the bispecific antibody comprising a combination of anti-factor IXa antibody A69-VH, anti-factor X antibody B26-VH, and hybrid L chains (BBA), which was the most effective for reducing the blood coagulation time.

### 22-1. Homology search of human antibodies

Human antibody amino acid sequence data was obtained from Kabat Database (ftp://ftp.ebi.ac.uk/pub/databases/kabat/) and IMGT Database (http://imgt.cines.fr/), both of which are publicly accessible, and homology search was conducted separately for mouse A69-H chain variable region (amino acid sequence: SEQ ID NO: 20), mouse B26-H chain variable region (amino acid sequence: SEQ ID NO: 24), and mouse BBA-L chain variable region (amino acid sequence: SEQ ID NO: 69) using the constructed database. As a result, the following human antibody sequences were found to be highly homologous; therefore, they were used as the framework regions (hereinafter, FRs) for the humanized antibodies.
(1) A69-H chain variable region: KABATID-000064 (Kabat Database) (Kipps et al., J. Clin. Invest. 1991; 87:2087-2096)
(2) B26-H chain variable region: EMBL Accession No. AB063872 (IMGT Database) (Unpublished data)
(3) BBA-L chain variable region: KABATID-024300 (Kabat Database) (Welschof et al., J. Immunol. Method. 1995; 179:203-214)
Humanized antibodies, in which each of the mouse antibody complementarity determining regions (CDRs) were grafted into the human antibody FRs of (1) to (3), were produced.

The inventors searched for human antibody secretory signal sequences highly homologous to the sequences of the human antibodies of (1) to (3) using the publicly available NCBI Web site for homology searches (http://www.ncbi.nlm.nih.gov/BLAST/). The following secretory signal sequences obtained from the search were used:
(1) for A69-H chain variable region: GenBank Accession No. AF062257
   SEQ ID NO: 123 (nucleotide sequence), SEQ ID NO: 124 (amino acid sequence);
(2) for B26-H chain variable region: GenBank Accession No. AAC18248
   SEQ ID NO: 125 (nucleotide sequence), SEQ ID NO: 126 (amino acid sequence); and
(3) for BBA-L chain variable region: GenBank Accession No. AAA59100
   SEQ ID NO: 127 (nucleotide sequence), SEQ ID NO: 128 (amino acid sequence)

### 22-2. Construction of humanized antibody gene expression vectors

Regarding the nucleotide sequence encoding the amino acid sequence ranging from the secretory signal sequence to an antibody variable region, twelve synthetic oligo DNAs of about 50 bases were produced alternately, such that approximately 20 bases at the 3' end hybridize thereto. The oligo DNAs were designed so that the sequence ranging from the 5' end to the 3' end is human antibody sequence or so that the 5' end is human antibody sequence and the 3' end is mouse antibody sequence. A primer that anneals to the 5' end of the antibody variable region gene and comprises an XhoI restriction sequence, and a primer that anneals to the 3' end of the antibody variable region gene, comprises an SfiI restriction sequence, and encodes the 5' end of the intron sequence were produced.

1 µL each of the synthetic oligo DNAs prepared at 2.5 µM were mixed, 1x TaKaRa Ex Taq Buffer, 0.4 mM dNTPs, and 0.5 units of TaKaRa Ex Taq (all from TaKaRa) were added, and the reaction solution was adjusted to 48 µL. After incubating at 94°C for 5 minutes, two cycles of reactions at 94°C for 2 minutes, 55°C for 2 minutes, and 72°C for 2 minutes were performed to form an assembly of each of the synthetic oligo DNAs and perform elongation reactions. Next, 1 µL of primers (each at 10 µM) that anneal to the 5' end and 3' end of the antibody gene were added, 35 cycles of reactions at 94°C for 30 seconds, 55°C for 30 seconds, and 72°C for 1 minute were carried out, and then this was reacted at 72°C for 5 minutes to amplify the antibody variable region gene. After PCR, the whole reaction solution was subjected to 1% agarose gel electrophoresis. The amplified fragments having the desired size (approximately 400 bp) were purified using QIAquick Gel Extraction Kit (QIAGEN) according to the method described in the attached instructions, and eluted with 30 µL of sterilized water. The fragments were cloned using pGEM-T Easy Vector Systems (Promega) according to the method described in the attached instructions. The nucleotide sequence of each DNA fragment was determined on DNA sequencer ABI PRISM 3700 DNA Sequencer or ABI PRISM 3730xL DNA Sequencer (Applied Biosystems) using BigDye Terminator Cycle Sequencing Kit (Applied Biosystems) according to the method described in the attached instructions.

After digesting the H chain variable region fragment-inserted plasmid and the L chain variable region fragment-inserted plasmid, which were confirmed to have the correct humanized antibody variable region gene sequence, with XhoI and SfiI, and with EcoRI, respectively, the reaction solutions were subjected to 1% agarose gel electrophoresis. DNA fragments having the desired size (approximately 400 bp) were purified using QIAquick Gel Extraction Kit (QIAGEN) according to the method described in the attached instructions, and eluted with 30 µL of sterilized water. Subsequently, the prepared variable region genes were inserted into animal cell expression vectors (pCAGG-IL3ss-g4CHPa, and pCAGG-IL6ss-g4CHPb) by the method described below in order to preferentially express IgG4 produced in Example 17, in which the two H chains form a heterodimer. After digesting pCAGG-IL3ss-g4CHPa with Xhol and SfiI (both from TaKaRa), fragments comprising the mouse IL-3 signal sequence were removed by subjecting the reaction solution to 1% agarose gel electrophoresis and collecting the vector region fragments, and this fragment was used to produce the humanized A69-H chain expression vector (the constant region comprises Y349C and T366W substitution) by inserting the humanized A69-H chain variable region gene fragment obtained above. Similarly, after digesting pCAGG-IL6ss-g4CHPb with XhoI and Sfil (TaKaRa), fragments comprising the mouse IL-6 signal sequence were removed by subjecting the reaction solution to 1% agarose gel electrophoresis and collecting the vector region fragments, and this fragment was used to produce the humanized B26-H chain expression vector (the constant region comprises E356C, T366S, L368A, and Y407V substitutions) by inserting the humanized B26-H chain variable region gene fragment obtained above. Similarly, the prepared H chain variable region gene was inserted into the animal cell expression vector (pCAGGss-g4CH) carrying the wildtype constant region gene produced in Example 17. After digesting pCAGGss-g4CH with XhoI and SfiI, fragments comprising the signal sequence were removed by subjecting the reaction solution to 1% agarose gel electrophoresis and collecting the vector region fragments, and this fragment was used to produce the humanized H chain expression vector (the constant region is wildtype) by inserting the humanized H chain variable region gene fragment obtained above. In addition, after digesting the L chain expression vector (pCAGG-IL3ss-hIgG light) produced in Example 18 with EcoRI, fragments comprising the mouse IL-3 signal sequence were removed by subjecting the reaction solution to 1% agarose gel electrophoresis and collecting the vector region fragments, and this fragment was used to produce the humanized BBA-L chain expression vector was produced by inserting the humanized BBA-L chain variable region gene fragment obtained above. The ligation reactions were performed using Rapid DNA Ligation Kit (Roche Diagnostics), and the resulting vectors were used to transform *E. coli* strain DH5α (TOYOBO).

### 22-3. Preparation of humanized bispecific antibodies

Humanized bispecific antibodies were expressed by the method described in Example 4-2 or by the following method. HEK293H cell line (Invitrogen) derived from human embryonic kidney cancer cells was suspended in DMEM medium (Invitrogen) containing 10% Fetal Bovine Serum, 10 mL of this suspension was plated in each dish (for adherent cells, 10 cm diameter, CORNING) at a cell density of 5 x 10⁵ to 6 x 10⁵ cells/mL, and after culturing for about 24 hours in a CO₂ incubator (37°C, 5% CO₂), the culture medium was removed by aspiration, and 6.9 mL of CHO-S-SFM-II medium containing 1% Fetal Bovine Serum was added. The plasmid DNA solution prepared in 22-2 (a total of 13.8 µg) was mixed with 20.7 µL of 1 µg/mL Polyethylenimine (Polysciences Inc.) and 690 µL of CHO-S-SFMII medium, left to stand for 10 minutes at room temperature, and this mixture was added to the cells in each of the dishes, and then incubated in a CO₂ incubator (37°C, 5% CO₂) for 4 to 5 hours. Subsequently, 6.9 mL of CHO-S-SFM-II medium (Invitrogen) containing 1% Fetal Bovine Serum (Invitrogen) was added, and this was cultured in a CO₂ incubator for three days. After collecting the culture supernatant, the cells were removed by centrifugation (at approximately 2,000x g, for 5 minutes, at room temperature). The supernatant was then sterilized through a 0.22 µm filter, MILLEX®-GV (Millipore). This sample was then stored at 4°C until use.

Next, the antibodies were purified by the method of Example 11, and its concentration was determined by the method of Example 5 or by the method described below. Biacore 1000 (BIACORE) was used and Protein A was immobilized onto Sensor Chip CM5 (BIACORE). More specifically, according to the manufacturer's protocol, the activated sensor chip was reacted with Protein A (SIGMA) solution diluted to 50 µg/mL using 10 mM sodium acetate solution (pH 4.0, BIACORE) at 5 µL/minute for 30 minutes, and then a blocking procedure was carried out to produce a Protein A-immobilized sensor chip. This sensor chip was used to measure the concentrations in culture supernatants and purified products with Biacore 1000 (BIACORE). HBS-EP Buffer (BIACORE) was used for immobilization of the sensor chip and for concentration measurements. A two-fold dilution series of human IgG4 (humanized anti-TF antibody, see WO99/51743) in HBS-EP Buffer produced by a six-step dilution from 4000 ng/mL was used as the standard for the concentration measurements.

### 22-4. Activity evaluation and antibody sequence modification of the humanized bispecific antibodies

To evaluate the plasma coagulation ability of the prepared humanized bispecific antibodies and the chimeric bispecific antibody (A69/B26/BBA), the effects of the antibodies on APTT was examined using F. VIII deficient plasma according to the method of Example 21. Human antibody FR amino acids were modified to increase the activity of the humanized bispecific antibody whose blood coagulation ability had decreased. During expression and secretion, three types of antibodies, humanized A69/humanized BBA antibody, humanized B26/humanized BBA antibody, and humanized A69/humanized B26/humanized BBA bispecific antibody, are expressed. These antibodies were separated, and for purifying only the bispecific antibody, amino acid modifications were carried out to lower the isoelectric point of the humanized A69H chain variable region, and to increase the isoelectric point of the humanized B26H chain variable region. At the same time, amino acid modifications were performed to prevent pyroglutamylation of the H chain amino termini, inhibit deamidation of the CDR sequences, and increase thermostability. More specifically, mutations were introduced into the humanized antibody variable regions using QuikChange® Site-Directed Mutagenesis Kit (Stratagene) according to the method described in the attached instructions. The H chain variable region gene fragment-inserted plasmid and the L chain variable region gene fragment-inserted plasmid, which were confirmed to have the desired humanized antibody variable region gene sequences, were digested with XhoI and SfiI, and with EcoRI, respectively, and then the reaction solutions were subjected to 1% agarose gel electrophoresis. DNA fragments having the desired size (approximately 400 bp) were purified using QIAquick Gel Extraction Kit (QIAGEN) according to the method described in the attached instructions, and then eluted with 30 µL of sterilized water. Subsequently, these fragments were ligated to the antibody constant region gene by the method indicated in Example 22-2 to produce antibody expression plasmids. Humanized bispecific antibodies were prepared by the method of Example 22-3, and the blood coagulation activity was evaluated by the method of Example 21.

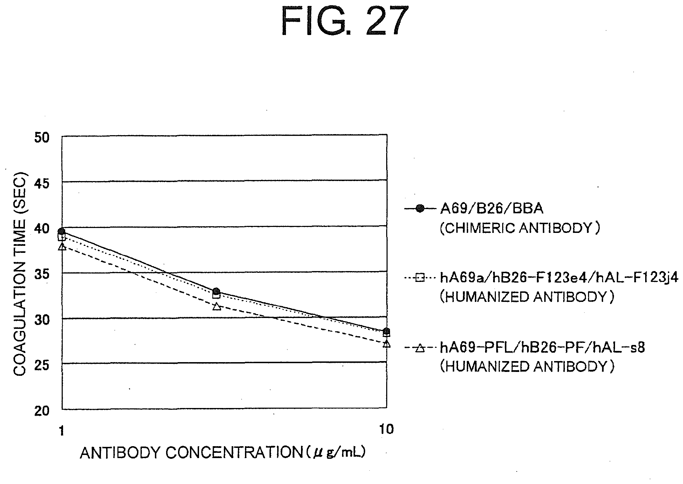

By repeating amino acid modifications of the FR sequence and evaluation of coagulation activity, humanized bispecific antibodies (humanized A69 (hA69a)/humanized B26 (hB26-F123e4)/humanized BBA (hAL-F123j4) and humanized A69 (hA69-PFL)/humanized B26 (hB26-PF)/humanized BBA (hAL-s8)) having activity equivalent to that of the chimeric bispecific antibody (A69/B26/BBA) were obtained. Fig. 27 shows the blood coagulation activity of humanized bispecific antibodies in which a heterodimer was formed using the knobs-into-holes technique (Protein Engineering vol.9, 617-621, 1996) on the constant region sequence. The variable region sequences of each of the humanized antibodies are described in the following SEQ ID NOs:
(1) humanized A69 antibody VH (hA69a)
   SEQ ID NO: 129 (nucleotide sequence), SEQ ID NO: 130(amino acid sequence);
(2) humanized B26 antibody VH (hB26-F123e4)
   SEQ ID NO: 131 (nucleotide sequence), SEQ ID NO: 132 (amino acid sequence);
(3) humanized BBA antibody VL (hAL-F123j4)
   SEQ ID NO: 133 (nucleotide sequence), SEQ ID NO: 134 (amino acid sequence);
(4) humanized A69 antibody VH (hA69-PFL)
   SEQ ID NO: 135 (nucleotide sequence), SEQ ID NO: 136 (amino acid sequence);
(5) humanized B26 antibody VH (hB26-PF)
   SEQ ID NO: 137 (nucleotide sequence), SEQ ID NO: 138 (amino acid sequence); and
(6) humanized BBA antibody VL (hAL-s8)
   SEQ ID NO: 139 (nucleotide sequence), SEQ ID NO: 140 (amino acid sequence).

### 22-5. Activity evaluation of humanized bispecific antibodies comprising a wildtype constant region, and modification of the antibody sequences.

When producing a commonly shared L chain bispecific antibody, three types of antibodies may be expressed during expression and secretion from animal cells. Expression of three types of antibodies, humanized A69/humanized BBA antibody, humanized B26/humanized BBA antibody, and humanized A69/humanized B26/humanized BBA bispecific antibody were expected for the antibodies of this example as well. These antibodies were separated, and for purifying only the bispecific antibodies, amino acid modifications were carried out to lower the isoelectric point of the humanized A69H chain variable region, and to increase the isoelectric point of the humanized B26H chain variable region. As a result, these procedures allowed the desired bispecific antibodies to be separated, and thus humanized bispecific antibodies carrying a wildtype constant region were prepared and the coagulation activity was evaluated. To increase the thermostability, the humanized A69 and humanized BBA variable region amino acid sequences of the humanized bispecific antibody described in Example 22-4 (humanized A69 (hA69-PFL)/humanized B26 (hB26-PF)/humanized BBA (hAL-s8)) were modified. Each of the humanized antibody variable region sequences are described in the following SEQ ID NOs:
(7) humanized A69 antibody VH (hA69-KQ)
   SEQ ID NO: 141 (nucleotide sequence), SEQ ID NO: 142 (amino acid sequence); and
(8) humanized BBA antibody VL (hAL-AQ)
   SEQ ID NO: 143 (nucleotide sequence), SEQ ID NO: 144 (amino acid sequence).

The antibody expression plasmids were produced by ligating the above-mentioned variable region sequences to a wildtype constant region gene (human IgG4 constant region or κ constant region) by the method indicated in Example 22-2.

The humanized bispecific antibodies were prepared by the method of Example 22-3, and then purified using cation exchange chromatography. The conditions for the cation exchange chromatography are indicated below. Since three types of peaks corresponding to the homogeneous combination of humanized A69, the desired bispecific antibody, which is the heterogeneous combination of humanized A69 and humanized B26, and the homogeneous combination of humanized B26 were obtained, the bispecific antibody was purified by collecting the peak fractions corresponding to the bispecific antibody. The fractions containing the bispecific antibody were concentrated using Amicon Ultra, MWCO 10000 (Millipore), and dialyzed overnight at a cold place against 20 mM sodium acetate, 150 mM NaCl, pH 6.0 solution, and then its concentration was determined.

| | |
|---|---|
| Column: | ProPac WCX-10, 4 x 250 mm, (Dionex) |
| Mobile phase; | A: 10 mmol/L NaH₂PO₄/Na₂HPO₄, pH 6.25 |
| | B: 10 mmol/LNaH₂PO₄/Na₂HPO₄, 500 mmol/L NaCl, pH 6.25 |
| Flow rate: | 1.0 mL/min |
| Gradient: | 10% B (5 min)→(40 min)→60% B→(5 min)→100% B (5 min) |
| Detection: | 220 nm |

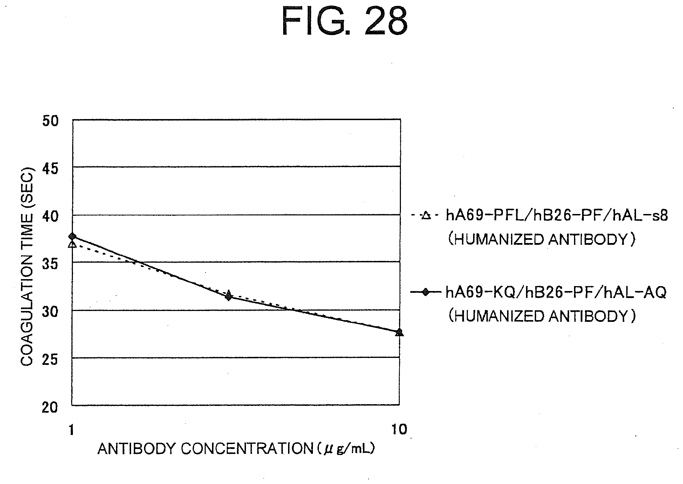

Using the purified bispecific antibodies, blood coagulation activity was evaluated by the method of Example 21. As described in Fig. 28, the humanized antibody (humanized A69 (hA69-PFL)/humanized B26 (hB26-PF)/humanized BBA (hAL-s8)) that showed activity equivalent to the chimeric antibody in Example 22-4, and the newly prepared humanized antibody (humanized A69 (hA69-KQ)/humanized B26 (hB26-PF)/humanized BBA (hAL-AQ)) were confirmed to have the similar level of blood coagulation activity.

### [Example 23] Combined use of two or more types of antibodies

The effect of using a bispecific antibody in combination with one or more other antibodies was confirmed by a plasma coagulation assay. 50 µL of the antibody solution, 100 µL of F. VIII deficient plasma (Biomerieux), and 50 µL of 0.3% kaolin solution (Biomerieux) were mixed and warmed at 37°C for 3 minutes. The coagulation reaction was initiated by adding 100 µL of 20 mM CaCl₂ (Dade Behring) to this mixed solution. The time taken until coagulation was measured using KC10A (Amelung) connected to CR-A (Amelung). The results of measuring the plasma coagulation time when bispecific antibody A69/B26/BBA was mixed with the anti-F. IXa antibody (XB12), anti-F. X antibody (SB04), XB12 and SB04, and bispecific antibody SB12/SB04µg/mL.

### Industrial Applicability

The present disclosure provides highly active multispecific antibodies that functionally substitute for a coagulation factor VIII and recognize both an enzyme and its substrate.

Since the multispecific antibodies disclosed herein are likely to be highly stable in blood and have low antigenicity, they are highly expected to become pharmaceuticals.

## Claims

1. A method for producing a bispecific antibody comprising a first H chain, a second H chain, and commonly shared L chains, wherein the method comprises the steps of:
(1) preparing a first antibody against a first antigen, and a second antibody against a second antigen;
(2) producing a bispecific antibody against the first antigen and the second antigen, which comprises variable regions of the first antibody and the second antibody;
(3) measuring the antigen binding activity or the biological activity of the bispecific antibody produced in step (2);
(4) producing a commonly shared L chain antibody by linking the H chain of the first antibody and the H chain of the second antibody with the L chain of the first antibody or the second antibody;
(5) measuring the antigen binding activity or biological activity of the commonly shared L chain antibody produced in step (4);
(6) substituting one, two, or three CDRs of the commonly shared L chains produced in step (4) with the L chain CDRs of the first antibody, or the second antibody;
(7) selecting the antibody having a desired activity by comparing the antigen binding activity or the biological activity of the antibody obtained in step (6) with that of the original bispecific antibody produced in step (2) or the antibody produced in step (4); and
(8) obtaining the antibody which has an activity equivalent to or higher than that of the original bispecific antibody produced in step (2), by repeating steps (6) and (7) as necessary for the antibody selected in step (7).

2. The method of claim 1, wherein the steps (6) and (7) are repeated two or more times.

## Patentansprüche

1. Verfahren zum Herstellen eines bispezifischen Antikörpers, der eine erste H-Kette, eine zweite H-Kette und allen gemeinsame L-Ketten umfasst, wobei das Verfahren die Schritte umfasst:
(1) Herstellen eines ersten Antikörpers gegen ein erstes Antigen und eines zweiten Antikörpers gegen ein zweites Antigen;
(2) Herstellen eines bispezifischen Antikörpers gegen das erste Antigen und das zweite Antigen, das variable Regionen des ersten Antikörpers und des zweiten Antikörpers umfasst;
(3) Messen der Antigenbindungsaktivität oder der biologischen Aktivität des in Schritt (2) hergestellten bispezifischen Antikörpers;
(4) Herstellen eines Antikörpers mit einer allen gemeinsamen L-Kette durch Verknüpfen der H-Kette des ersten Antikörpers und der H-Kette des zweiten Antikörpers mit der L-Kette des ersten Antikörpers oder des zweiten Antikörpers;
(5) Messen der Antigenbindungsaktivität oder der biologischen Aktivität des in Schritt (4) hergestellten Antikörpers mit einer allen gemeinsamen L-Kette;
(6) Substituieren von einer, zwei oder drei CDRs der in Schritt (4) hergestellten allen gemeinsamen L-Ketten mit den CDRs der L-Ketten des ersten Antikörpers oder des zweiten Antikörpers;
(7) Auswählen des Antikörpers, der eine gewünschte Aktivität aufweist, durch Vergleichen der Antigenbindungsaktivität oder der biologischen Aktivität des in Schritt (6) erhaltenen Antikörpers mit der des ursprünglichen bispezifischen in Schritt (2) hergestellten Antikörpers oder des in Schritt (4) hergestellten Antikörpers; und
(8) Erhalten des Antikörpers, der eine Aktivität aufweist, die gleich oder höher als die des ursprünglichen bispezifischen in Schritt (2) hergestellten Antikörpers ist, durch Wiederholen der Schritte (6) und (7) wie es für den in Schritt (7) ausgewählten Antikörper notwendig ist.

2. Verfahren nach Anspruch 1, wobei die Schritte (6) und (7) zwei Mal oder mehrere Male wiederholt werden.

## Revendications

1. Méthode pour produire un anticorps bispécifique comprenant une première chaîne H, une deuxième chaîne H, et des chaînes L partagées en commun, laquelle méthode comprend les étapes suivantes :
(1) préparation d'un premier anticorps dirigé contre un premier antigène, et d'un deuxième anticorps dirigé contre un deuxième antigène ;
(2) production d'un anticorps bispécifique dirigé contre le premier antigène et le deuxième antigène, qui comprend des régions variables du premier anticorps et du deuxième anticorps ;
(3) mesure de l'activité de liaison à un antigène ou de l'activité biologique de l'anticorps bispécifique produit dans l'étape (2) ;
(4) production d'un anticorps à chaîne L partagée en commun par liaison de la chaîne H du premier anticorps et de la chaîne H du deuxième anticorps avec la chaîne L du premier anticorps ou du deuxième anticorps ;
(5) mesure de l'activité de liaison à un antigène ou de l'activité biologique de l'anticorps à chaîne L partagée en commun produite dans l'étape (4) ;
(6) remplacement d'une, deux ou trois CDR des chaînes L partagées en commun produites dans l'étape (4) par les CDR de chaîne L du premier anticorps, ou du deuxième anticorps ;
(7) sélection de l'anticorps ayant une activité souhaitée par comparaison de l'activité de liaison à un antigène ou de l'activité biologique de l'anticorps obtenu dans l'étape (6) avec celle de l'anticorps bispécifique original produit dans l'étape (2) ou de l'anticorps produit dans l'étape (4) ; et
(8) obtention de l'anticorps qui a une activité équivalente ou supérieure à celle de l'anticorps bispécifique original produit dans l'étape (2), par répétition des étapes (6) et (7) selon les besoins pour l'anticorps choisi dans l'étape (7).

2. Méthode selon la revendication 1, dans laquelle les étapes (6) et (7) sont répétées deux fois ou plus.
